(19) Europäisches Patentamt
European Patent Office
Office européen des brevets

(11) Veröffentlichungsnummer: **0 303 172**
**A2**

(12) # EUROPÄISCHE PATENTANMELDUNG

(21) Anmeldenummer: **88112640.3**

(22) Anmeldetag: **03.08.88**

(51) Int. Cl.⁴: **C07D 501/20 , C07D 277/20 , C07C 143/82 , C07C 147/10 , A61K 31/545 , C07D 317/46**

(30) Priorität: **14.08.87 CH 3133/87**
**03.12.87 CH 4726/87**

(43) Veröffentlichungstag der Anmeldung:
**15.02.89 Patentblatt 89/07**

(84) Benannte Vertragsstaaten:
**AT BE CH DE ES FR GB GR IT LI LU NL SE**

(71) Anmelder: **F. HOFFMANN-LA ROCHE & CO. Aktiengesellschaft**

**CH-4002 Basel(CH)**

(72) Erfinder: **Götschi, Erwin, Dr.**
**Landhofweg 23**
**CH-4153 Reinach(CH)**

(74) Vertreter: **Lederer, Franz, Dr. et al**
**Patentanwält Dr. Franz Lederer Lucile Grahnstrasse 22**
**D-8000 München 80(DE)**

(54) **Oxyimino-Cephalosporine.**

(57) 1. Acylderivate der allgemeinen Formel

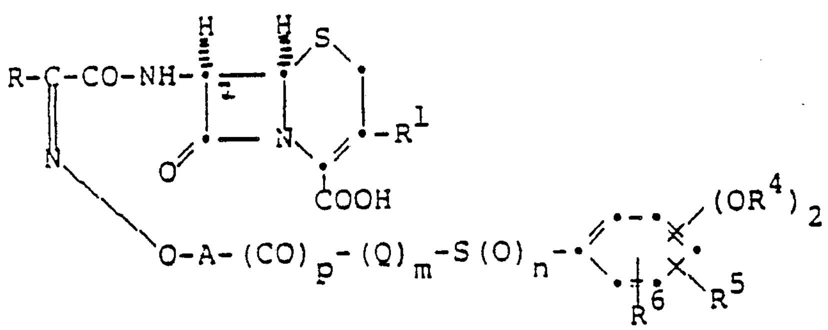

I

worin R eine einkernige, carbocyclische aromatische Gruppe, eine 5-gliedrige, aromatische, heterocylische Gruppe, welche als Heteroringglieder ein Sauerstoff-oder Schwefelatom oder eine Imino- der niedere Alkyliminogruppe und gegebenenfalls noch ein oder zwei Stickstoffatome enthält, oder eine 6-gliedrige, aromatische, heterocyclische Gruppe, welche ein bis drei Stickstoffatome als Heteroringglieder enthält, $R^1$ Wasserstoff oder ein in der Cephalosporinchemie verwendbarer 3-Substituent, A gegebenenfalls durch Carboxy, Carbamoyl, niederes Alkylcarbamoyl oder Di(niederes Alkyl)carbamoyl substituiertes niederes Alkylen oder $C_{3-7}$-Cycloalkylen, Q gegebenenfalls durch Carboxy, Carbamoyl, niederes Alkylcarbamoyl oder Di(niederes Alkyl)carbamoyl substituiertes niederes Alkylen oder $C_{3-7}$-Cycloalkylen oder die Gruppe $-NR^2$- oder $-NR^2NR^3$-, $R^2$ und $R^3$ je Wasserstoff oder niederes Alkyl, p und m die Zahl 0 oder 1, n die Zahl 0, 1 oder 2, $R^4$ (Wasserstoff, niederes Alkanoyl oder
Tri(niederes Alkyl)-silyl), 2 Reste $R^4$ zusammen Diphenylmethylen, $R^5$ Wasserstoff, niederes Alkyl, Hydroxy,

EP 0 303 172 A2

niederes Alkoxy, Halogen, Nitro oder die Gruppe $-OCOR^7$, $-OCOOR^{71}$, $-N(R^7)_2$, $-NHCOR^7$, $-NCOOR^{71}$, $-COR^7$, $-SR^7$, $-SOR^7$, $-SO_2R^7$, $-SO_3H$, $-COOR^7$ oder
$-CON(R^7)_2$, $R^6$ Wasserstoff, niederes Alkyl oder Halogen, $R^7$ Wasserstoff oder niederes Alkyl und $R^{71}$ niederes Alkyl bedeuten, und die beiden Gruppen $-OR^4$ über benachbarte Kohlenstoffatome des Phenylringes gebunden sind,
sowie leicht hydrolysierbare Ester und pharmazeutisch annehmbare Salze dieser Verbindungen und Hydrate von Verbindungen der Formel I bzw. von deren Estern und Salzen. Die Produkte sind antibiotisch, insbesondere antibakteriell wirksam.

## Oxyimino-Cephalosporine

Die vorliegende Erfindung betrifft neue Acylderivate, und zwar Cephalosporinderivate der allgemeinen Formel

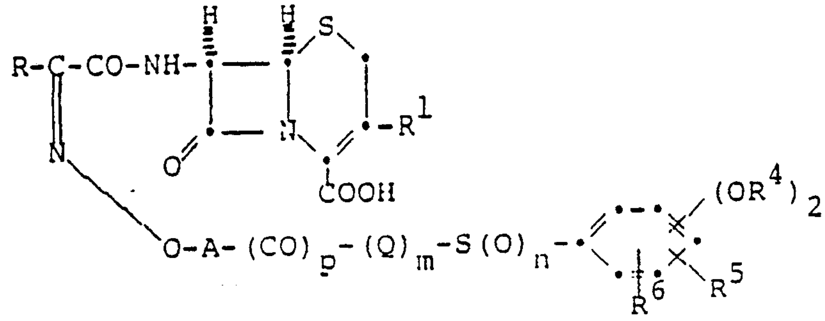

I

worin R eine einkernige, carbocyclische aromatische Gruppe, eine 5-gliedrige, aromatische, heterocyclische Gruppe, welche als Heteroringglieder ein Sauerstoff-oder Schwefelatom oder eine Imino- oder niedere Alkyliminogruppe und gegebenenfalls noch ein oder zwei Stickstoffatome enthält, oder eine 6-gliedrige, aromatische, heterocyclische Gruppe, welche ein bis drei Stickstoffatome als Heteroringglieder enthält, $R^1$ Wasserstoff oder einen in der Cephalosporinchemie verwendbaren 3-Substituenten, A gegebenenfalls durch Carboxy, Carbamoyl, niederes Alkylcarbamoyl oder Di(niederes Alkyl)carbamoyl substituiertes niederes Alkylen oder $C_{3-7}$-Cycloalkylen, Q gegebenenfalls durch Carboxy, Carbamoyl, niederes Alkylcarbamoyl oder Di(niederes Alkyl)carbamoyl substituiertes niederes Alkylen oder $C_{3-7}$-Cycloalkylen oder die Gruppe $-NR^2-$ oder $-NR^2NR^3-$, $R^2$ und $R^3$ je Wasserstoff oder niederes Alkyl, p und m die Zahl 0 oder 1, n die Zahl 0, 1 oder 2, $R^4$ Wasserstoff, niederes Alkanoyl oder Tri(niederes Alkyl)silyl, zwei Reste $R^4$ zusammen Diphenylmethylen, $R^5$ Wasserstoff, niederes Alkyl, Hydroxy, niederes Alkoxy, Halogen, Nitro oder die Gruppe $-OCOR^7$, $-OCOOR^{71}$, $-N(R^7)_2$, $-NHCOR^7$, $-NHCOOR^{71}$, $-COR^7$, $-SR^7$, $-SOR^7$, $-SO_2R^7$, $-SO_3H$, $-COOR^7$ oder $-CON(R^7)_2$, $R^6$ Wasserstoff, niederes Alkyl oder Halogen, $R^7$ Wasserstoff oder niederes Alkyl und $R^{71}$ niederes Alkyl bedeuten, und die beiden Gruppen $-OR^4$ über benachbarte Kohlenstoff- atome des Phenylringes gebunden sind,
sowie leicht hydrolysierbare Ester und pharmazeutisch annehmbare Salze dieser Verbindungen und Hydrate von Verbindungen der Formel I bzw. von deren Estern und Salzen.

Diese erfindungsgemässen Produkte besitzen wertvolle antibiotische Eigenschaften.

Die Verbindungen der Formel I liegen vorzugsweise in der in der obigen Formel dargestellten syn-isomeren Form vor. Sie können jedoch auch als Gemische mit der entsprechenden anti-isomeren Form vorliegen, in welchen die syn-isomere Form überwiegt.

Gegenstand der vorliegenden Erfindung sind: Die obigen Verbindungen der Formel I, deren leicht hydrolysierbare Ester und pharmazeutisch annehmbare Salze sowie die Hydrate dieser Stoffe als solche und zur Anwendung als therapeutische Wirkstoffe; ein Verfahren zur Herstellung dieser Produkte; Arzneimittel auf der Basis dieser Produkte und deren Herstellung; die Verwendung der erfindungsgemässen Produkte bei der Bekämpfung oder Verhütung von Krankheiten und die Verwendung der erfindungsgemässen Produkte zur Herstellung von antibiotisch wirksamen Arzneimitteln.

Der Ausdruck "nieder" bezeichnet Reste und Verbindungen mit höchstens 7, vorzugsweise höchstens 4 Kohlenstoffatomen. Der Ausdruck "Alkyl" bezeichnet geradkettige oder verzweigte, gesättigte Kohlenwasserstoffreste, wie Methyl, Aethyl, Propyl, Isopropyl und t-Butyl. Der Ausdruck "Alkylen" bezeichnet entsprechende Kohlenwasserstoffreste, welche jedoch zwei freie Valenzen besitzen, wie Methylen, Dimethylen, Aethyliden, Propyliden, Butyliden, Isopropyliden und 1,2-Isobutylen. Der Ausdruck "Alkoxy" bezeichnet über ein Sauerstoffatom gebundene Alkylgruppen, wie Methoxy und Aethoxy. Der Ausdruck "Alkanoyl" bezeichnet von geradkettigen oder verzweigten, gesättigten Fettsäuren abgeleitete Reste, wie Formyl und Acetyl. Der Ausdruck "$C_{3-7}$-Cycloalkyl" bezeichnet gesättigte, cyclische Kohlenwasserstoffreste, d.h. gegebenenfalls durch Alkylgruppen substituierte Cycloalkylreste, wie Cyclopropyl, Cyclobutyl, Cyclohexyl und Methylcyclopropyl. Der Ausdruck "$C_{3-7}$-Cycloalkylen" bezeichnet cyclische Kohlenwassertoffreste, welche jedoch zwei freie Valenzen besitzen, wie Cyclopropyliden, Cyclobutyliden, Cyclohexyliden und 2-Methylcyclopropyliden.

Der Ausdruck "Halogen" bezeichnet die vier Formen Chlor, Fluor, Brom und Jod, wobei die Bedeutungsmöglichkeit Chlor bevorzugt ist.

Der Ausdruck "einkernige, carbocyclische aromatische Gruppe" bezeichnet unsubstituierte oder substituierte Phenylgruppen, vorzugsweise gegebenenfalls durch Halogen, Hydroxy, Nitro, Amino, Cyano, Trifluormethyl, niederes Alkyl oder niederes Alkoxy mono-, di- oder trisubstituierte Phenylgruppen.

Die 5-gliedrigen, aromatischen, heterocyclischen Gruppen sind vorzugsweise unsubstituiert oder durch Halogen, Hydroxy, Nitro, Amino, Cyano, Trifluormethyl, niederes Alkyl oder niederes Alkoxy substituiert. Sie sind vorzugsweise durch eine Aminogruppe substituiert. Beispiele für derartige Heterocyclen, welche wie erwähnt unsubstituiert oder substituiert sein können, sind: Furyl, Thienyl, Oxazolyl, Thiazolyl, Thiadiazolyl, Imidazolyl, Pyrazolyl und Triazolyl. Bevorzugte heterocyclische Gruppen sind: 2-Amino-4-thiazolyl, 5-Amino-1,2,4-thiadiazol-3-yl, 2-Amino-4-oxazolyl und 2-Amino-4-imidazolyl.

Die 6-gliedrigen, aromatischen, heterocyclischen Gruppen sind vorzugsweise unsubstituiert oder durch Halogen, Hydroxy, Nitro, Amino, Cyano, Trifluormethyl, niederes Alkyl oder niederes Alkoxy substituiert. Beispiele für derartige Heterocyclen, welche wie erwähnt unsubstituiert oder substituiert sein können, sind: Pyridyl, Pyrimidinyl und Triazinyl. Eine bevorzugte 6-gliedrige heterocyclische Gruppe ist die 2-Amino-6-pyridylgruppe.

Der Ausdruck "ein in der Cephalosporinchemie verwendbarer 3-Substitutent" bezeichnet die üblichen, in der 3-Stellung des Cephalosporingerüstes von therapeutisch aktiven Cephalosporinen vorhandenen Substituenten. Dieser Ausdruck bezeichnet vorzugsweise niederes Alkyl, wie Methyl, niederes Alkoxy, wie Methoxy, Halogen, wie Chlor, oder die Gruppe $-CH_2R'$ oder $-CH_2S-R''$, wobei $R'$ Azido, niederes Alkanoyloxy, Carbamoyloxy, N-(niederes Alkyl)carbamoyloxy, N,N-Di(niederes Alkyl)carbamoyloxy oder eine über ein Stickstoffatom gebundene, N-haltige heterocyclische Gruppe und $R''$ eine über ein Kohlenstoffatom gebundene heterocyclische Gruppe bedeuten.

Der Ausdruck "N-haltige heterocyclische Gruppe" bezeichnet vorzugsweise gesättigte, partiell ungesättigte und aromatische heterocyclische Gruppen, welche bis zu 4 Stickstoffatome als Heteroatome enthalten. Sie sind vorzugsweise 5- oder 6-gliedrig und können noch durch einen 5- oder 6-gliedrigen Cycloalkanring oder durch einen Benzolring aneliert sein. Sie sind vorzugsweise unsubstituiert oder durch niederes Alkyl oder Carbamoyl substituiert. Das Stickstoffatom, über welches die N-haltige heterocyclische Gruppe gebunden ist, kann auch quaternär substituiert sein. 5-Methyl-2-tetrazolyl und Pyridinium-1-yl seind Beispiele für N-haltige heterocyclische Gruppen.

Der Ausdruck "heterocyclische Gruppe" bezeichnet vorzugsweise monocyclische, insbesondere 5- und 6-gliedrige, partiell ungesättigte oder aromatische heterocyclische Gruppen, welche als Heteroatome vorzugsweise ein Sauerstoff- oder Schwefelatom und/oder 1-4 Stickstoffatome enthalten, und bicyclische, insbesondere 8-10-gliedrige, partiell ungesättigte oder aromatische heterocyclische Gruppen, welche als Heteroatome vorzugsweise ein Sauerstoff- oder Schwefelatom und/oder 1-5 Stickstoffatome enthalten. Diese Gruppen sind vorzugsweise unsubstituiert oder durch niederes Alkyl, niederes Alkoxy, niederes Alkandiyl, Halogen, Trifluormethyl, Hydroxy, Oxo, Carboxy, niederes Alkoxycarbonyl, Carbamoyl, N-(niederes Alkyl)-carbamoyl, N,N-Di(niederes Alkyl)carbamoyl, Amino, niederes Alkylamino, Di(niederes Alkyl)amino, Mercapto, niederes Alkylthio, niederes Hydroxyalkyl, niederes Aminoalkyl, niederes Alkylamino-niederes Alkyl, Di-(niederes Alkyl)amino-niederes Alkyl, niederes Carboxyalkyl, Carbamoyl-niederes Alkyl, N-(niederes Alkyl)-carbamoyl-niederes Alkyl, N,N-Di(niederes Alkyl)-carbamoyl-niederes Alkyl und/oder Sulfo-niederes Alkyl mono-, di- oder trisubstituiert.

Die monocyclischen, heterocyclischen Gruppen sind vorzugsweise aromatisch und vorzugsweise durch niederes Alkyl, Halogen, Hydroxy, Oxo, Carboxy, Carbamoyl, Amino, niederes Alkylamino, Di(niederes Alkyl)amino, Mercapto, niederes Alkylthio, niederes Hydroxyalkyl, niederes Aminoalkyl, niederes Alkylamino-niederes Alkyl, Di(niederes Alkyl)amino-niederes Alkyl, niederes Carboxyalkyl, Carbamoyl-niederes Alkyl, N-(niederes Alkyl)carbamoyl-niederes Alkyl, N,N-Di(niederes Alkyl)-carbamoyl-niederes Alkyl und/oder Sulfo-niederes Alkyl substituiert. Als Beispiele seien erwähnt Tetrazolyl, Triazolyl, Thiadiazolyl und Triazinyl. Bevorzugte Gruppen sind: 1-Methyl-tetrazol-5-yl, 1-(2-Hydroxyäthyl)-5-tetrazolyl, 5-Methyl-1,3,4-thiadiazol-2-yl, 1,2,3-Thiadiazol-5-yl, 1,4,5,6-Tetrahydro-4-methyl-5,6-dioxo-as-triazin-3-yl, 2,5-Dihydro-6-hydroxy-2-methyl-5-oxo-as-triazin-3-yl und 1,2,5,6-Tetrahydro-2-methyl-5,6-dioxo-as-triazin-3-yl.

Die bicyclischen Gruppen sind vorzugsweise aromatisch. Sie sind vorzugsweise unsubstituiert oder durch Amino, niederes Alkylamino Di(niederes Alkyl)amino, Carboxy, niederes Alkoxycarbonyl, Carbamoyl, N-(niederes Alkyl)carbamoyl, N,N-Di(niederes Alkyl)carbamoyl, niederes Alkyl, Trifluormethyl und/oder $C_{3-4}$-Alkandiyl substituiert. Sie sind vorzugsweise 9-gliedrig und besitzen 2 bis 4 Stickstoffatome als Heteroringglieder.

In einer bevorzugten Ausführungsform bedeutet A niederes Alkyliden und n die Zahl 2 und entweder m und p die Zahl 0 oder m und p die Zahl 1, Q die Gruppe $-NR^2NR^3-$ und $R^2$ und $R^3$ je Wasserstoff. $R^4$, $R^5$ und $R^6$ bedeuten vorzugsweise je Wasserstoff. Vorzugsweise befinden sich die beiden Gruppen $-OR^4$ in

den Stellungen 3 und 4 des Phenylringes.

In einer besonders bevorzugten Ausführungsform bedeutet A Methylen oder Isopropyliden.

Bevorzugte Substituenten in 7-Stellung der Cephalosporine der Formel I sind:

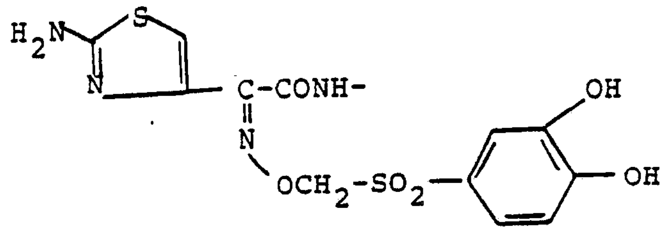

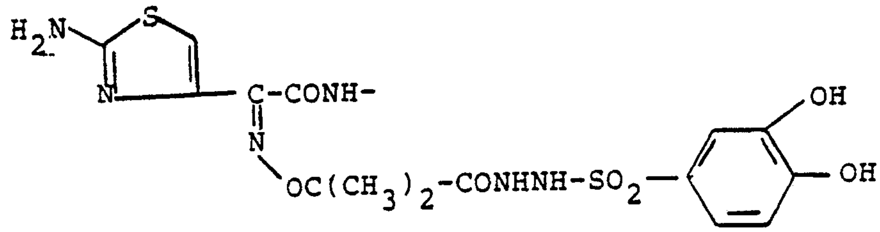

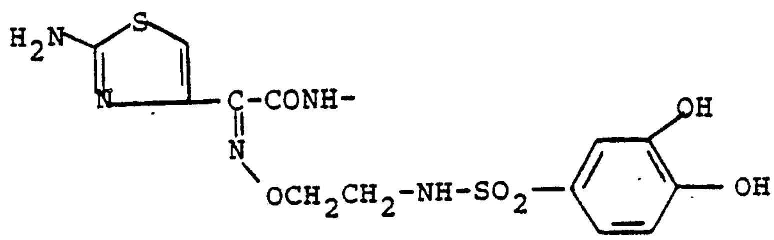

$R^1$ bedeutet vorzugsweise Wasserstoff, niederes Alkyl, niederes Alkoxy, Halogen oder die Gruppe $-CH_2-R'$ oder $-CH_2-S-R''$, wobei $R'$ Azido, niederes Alkanoyloxy, Carbamoyloxy, N-(niederes Alkyl)carbamoyloxy, N,N-di(niederes Alkyl)carbamoyloxy oder eine über ein Stickstoffatom gebundene, N-haltige heterocyclische Gruppe und $R''$ eine über ein Kohlenstoffatom gebundene heterocyclische Gruppe bedeuten.

In einer besonders bevorzugten Ausführungsform bedeutet $R^1$ die Gruppe $-CH_2-S-R''$. $R''$ bedeutet vorzugsweise eine bicyclische, 8- bis 10-gliedrige partiell ungesättigte oder aromatische heterocyclische Gruppe, welche ein Sauerstoff- oder Schwefelatom und/oder 1-5 Stickstoffatome als Heteroringglieder enthält. Die heterocyclische Gruppe $R''$ ist dabei vorzugsweise unsubstituiert oder durch Amino, niederes Alkyl, niederes Alkoxy, niederes Alkandiyl, Halogen, Trifluormethyl, Hydroxy, Oxo, Carboxy, niederes Alkoxycarbonyl, Carbamoyl, N-(niederes Alkyl)carbamoyl und/oder N,N-di(niederes Alkyl)carbamoyl mono-, di- oder trisubstituiert.

In einer ganz besonders bevorzugten Ausführungsform entspricht die heterocyclische Gruppe $R''$ der allgemeinen Formel

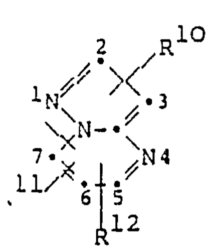 (a), 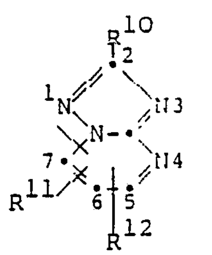 (b), 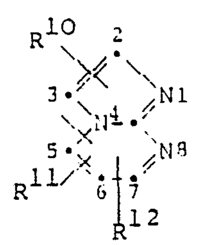 (c) oder 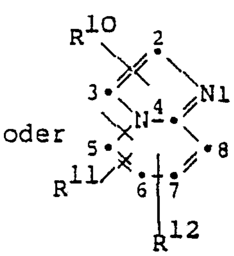 (d)

worin $R^{10}$ Wasserstoff, Amino, Carboxy, niederes Alkoxycarbonyl, Carbamoyl, Hydroxymethyl, N-(niederes

Alkyl)carbamoyl oder N,N-Di(niederes Alkyl)carbamoyl und $R^{11}$ und $R^{12}$ je Wasserstoff, niederes Alkyl oder Trifluormethyl oder zusammen eine $C_{3-4}$-Alkandiylgruppe bedeuten.

Die heterocyclischen Gruppen der Formeln (a) und (b) sind dabei vorzugsweise über die 5- oder die 7-Stellung, insbesondere über die 7-Stellung verknüpft. Die heterocyclische Gruppe der Formel (c) ist vorzugsweise über die 5- oder 7-Stellung, insbesondere über die 5-Stellung verknüpft, und diejenige der Formel (d) ist vorzugsweise über die 5- oder 8-Stellung, insbesondere über die 5-Stellung verknüpft. Der Substituent $R^{10}$ befindet sich vorzugsweise in der 3-Stellung, wenn die heterocyclische Gruppe der Formel (a), (c) oder (d) entspricht. $R^{10}$ bedeutet vorzugsweise Carbamoyl oder Hydroxymethyl. $R^{11}$ bedeutet vorzugsweise Wasserstoff, und $R^{12}$ bedeutet vorzugsweise niederes Alkyl oder Trifluormethyl, insbesondere Methyl.

In weiteren ganz besonders bevorzugten Ausführungsformen bedeutet $R''$ 2-Carbamoyl-5-methyl-s-triazolo[1,5-a]pyrimidin-7-yl oder 2-(Hydroxymethyl)-5-methyl-s-triazolo[1,5-a]pyridin-7-yl.

R bedeutet vorzugsweise 2-Amino-4-thiazolyl.

Im Rahmen der vorliegenden Erfindung ganz besonders bevorzugte Produkte sind:

(6R,7R)-7-[(Z)-2-(2-Amino-4-thiazolyl)-2-[[[(3,4-dihydroxyphenyl)sulfonyl]methoxy]imino]acetamido]-3-[[(2-carbamoyl-5-methyl-s-triazolo[1,5-a]pyrimidin-7-yl)thio]-methyl]-8-oxo-5-thia-1-azabicyclo[4.2.0]oct-2-en-2-carbonsäure,

(6R,7R)-7-[(Z)-2-(2-Amino-4-thiazolyl)-2-[[[(3,4-dihydroxyphenyl)sulfonyl]methoxy]imino]acetamido]-3-[[[2-(hydroxymethyl)-5-methyl-s-triazolo[1,5-a]pyrimidin-7-yl]thio]methyl]-8-oxo-5-thia-1-azabicyclo[4.2.0]oct-2-en-2-carbonsäure

und deren pharmazeutisch annehmbare Salze.

Weitere erfindungsgemässe Produkte sind die nachfolgend aufgeführten Verbindungen der Formel I und deren pharmazeutisch annehmbare Salze:

(6R,7R)-7-[(Z)-2-(2-Amino-4-thiazolyl)-2-[[[(3,4-dihydroxyphenyl)sulfonyl]methoxy]imino]acetamido]-3-[[(2,5-dihydro-6-hydroxy-2-methyl-5-oxo-as-triazin-3-yl)thio]methyl]-8-oxo-5-thia-1-azabicyclo[4.2.0]oct-2-en-2-carbonsäure,

(6R,7R)-3-(Acetoxymethyl)-7-[(Z)-2-(2-amino-4-thiazolyl)-2-[[[(3,4-dihydroxyphenyl)sulfonyl]methoxy]imino]-acetamido]-8-oxo-5-thia-1-azabicyclo[4.2.0]oct-2-en-2-carbonsäure,

(6R,7R)-7-[(Z)-2-(2-Amino-4-thiazolyl)-2-[[[(3,4-dihydroxyphenyl)sulfonyl]methoxy]imino]acetamido]-3-[[(2-carboxy-5-methyl-s-triazolo[1,5-a]pyrimidin-7-yl)thio]methyl]-8-oxo-5-thia-1-azabicyclo[4.2.0]oct-2-en-2-carbon säure,

(6R,7R)-7-[(Z)-2-(2-Amino-4-thiazolyl)-2-[[[(3,4-dihydroxyphenyl)sulfonyl]methoxy]imino]acetamido]-3-(azidomethyl)-8-oxo-5-thia-1-azabicyclo[4.2.0]oct-2-en-2-carbonsäure,

1-[(6R,7R)-7-[(Z)-2-(2-Amino-4-thiazolyl)-2-[[[(3,4-dihydroxyphenyl)sulfonyl]methoxy]imino]acetamido]-2-carboxy-8-oxo-5-thia-1-azabicyclo[4.2.0]oct-2-en-3-yl]pyridiniumbetain,

(6R,7R)-7-[(Z)-2-(2-Amino-4-thiazolyl)-2-[[[(3,4-dihydroxyphenyl)sulfonyl]methoxy]imino]acetamido]-3-[[(5-methyl-1,3,4-thiadiazol-2-yl)thio]methyl]-8-oxo-5-thia-1-azabicyclo[4.2.0]oct-2-en-2-carbonsäure,

(6R,7R)-7-[(Z)-2-(2-Amino-4-thiazolyl)-2-[[[(3,4-dihydroxyphenyl)sulfonyl]methoxy]imino]acetamido]-3-[[(3-carbamoyl-5-methylpyrazolo[1,5-a]pyrimidin-7-yl)thio]methyl]-8-oxo-5-thia-1-azabicyclo[4.20]oct-2-en-2-carbonsäure,

(6R,7R)-7-[(Z)-2-(2-Amino-4-thiazolyl)-2-[[[(3,4-dihydroxyphenyl)sulfonyl]methoxy]imino]acetamido]-3-[[(3-carbamoyl-7-(trifluormethyl)pyrazolo[1,5-a]pyrimidin-5-yl)thio]methyl]-8-oxo-5-thia-1-azabicyclo[4.2.0]oct-2-en-2-carbonsäure,

(6R,7R)-7-[(Z)-2-(2-Amino-4-thiazolyl)-2-[[1-[3-[(3,4-dihydroxyphenyl) sulfonyl]carbazolyl]-1-methyläthoxy]-imino]acetamido]-3-[[(2-carbamoyl)-5-methyl-s-triazolo[1,5-a]pyrimidin-7-yl)thio]methyl]-8-oxo-5-thia-1-azabicyclo[4.2.0]oct-2-en-2-carbonsäure,

(6R,7R)-7-[(Z)-2-(2-Amino-4-thiazolyl)-2-[[[(3,4-dihydroxyphenyl)sulfonyl]methoxy]imino]acetamido]-3-[[[2-(methoxycarbonyl)-5-methyl-s-triazolo[1,5-a]pyrimidin-7-yl]thio]methyl]-8-oxo-5-thia-1-azabicyclo[4.2.0]oct-2-en-2-carbonsäure,

(6R,7R)-7-[(Z)-2-(2-Amino-4-thiazolyl)-2-[[[(3,4-dihydroxyphenyl)sulfonyl]methoxy]imino]acetamido]-3-[[(7-methylpyrazolo[1,5-a]pyrimidin-5-yl)thio]methyl-8-oxo-5-thia-1-azabicyclo[4.2.0]oct-2-en-2-carbonsäure,

(6R,7R)-7-[(Z)-2-(2-Amino-4-thiazolyl)-2-[[[(3,4-dihydroxyphenyl)sulfonyl]methoxy]imino]acetamido]-3-[[(5-methylpyrazolo[1,5-a]pyrimidin-7-yl)thio]methyl]-8-oxo-5-thia-1-azabicyclo[4.2.0]oct-2-en-2-carbonsäure,

(6R,7R)-7-[(Z)-2-(2-Amino-4-thiazolyl)-2-[[[(3,4-dihydroxyphenyl)sulfonyl]methoxy]imino]acetamido]-3-[[(3-carboxy-7-methylpyrazolo[1,5-a]pyrimidin-5-yl)thio]methyl]-8-oxo-5-thia-1-azabicyclo[4.2.0]oct-2-en-2-carbonsäure,

(6R,7R)-7-[(Z)-2-(2-Amino-4-thiazolyl)-2-[[[(3,4-dihydroxyphenyl)sulfonyl]methoxy]imino]acetamido]-3-[[(2,5-dimethylpyrazolo[1,5-a]pyrimidin-7-yl)thio]methyl]-8-oxo-5-thia-1-azabicyclo[4.2.0]oct-2-en-2-carbonsäure,

(6R,7R)-7-[(Z)-2-(2-Amino-4-thiazolyl)-2-[[[(3,4-dihydroxyphenyl)sulfonyl]methoxy]imino]acetamido]-3-[[(2,8-bis(trifluormethyl)-4-chinolinyl]thio]methyl-8-oxo-5-thia-1-azabicyclo[4.2.0]oct-2-en-2-carbonsäure,
3-[[[(6R,7R)-7-[(Z)-2-(2-Amino-4-thiazolyl)-2-[[[(3,4-dihydroxyphenyl)sulfonyl]methoxy]imino]acetamido]-2-carboxy-8-oxo-5-thia-1-azabicyclo[4.2.0]oct-2-en-3-yl]methyl]thio]-1-(carbamoylmethyl)pyridiniumbetain,
(6R,7R)-7-[(Z)-2-(2-Amino-4-thiazolyl)-2-[[[(3,4-dihydroxyphenyl)sulfonyl]methoxy]imino]acetamido]-3-[[[2-(hydroxymethyl)-5-methyl-s-triazolo[1,5-a]pyrimidin-7-yl]-thio]methyl]-8-oxo-5-thia-1-azabicyclo[4.2.0]oct-2-en-2-carbonsäure,
(6R,7R)-7-[(Z)-2-(2-Amino-4-thiazolyl)-2-[[2-[3,4-dihydroxyphenyl)sulfonamido]äthoxy]imino]acetamido]-3-[[-(2-carbamoyl-5-methyl-s-triazolo[1,5-a]pyrimidin-7-yl)thio]methyl]-8-oxo-5-thia-1-azabicyclo[4.2.0]oct-2-en-2-carbonsäure,
(6R,7R)-7-[(Z)-2-(2-Amino-4-thiazolyl)-2-[[(3,4-dihydroxyphenyl)sulfonyl]äthoxy]imino]acetamido]-3-[[[2-(hydroxymethyl)-5-methyl-s-triazolo[1,5-a]pyrimidin-7-yl]thio]methyl]-8-oxo-5-thia-1-azabicyclo[4.2.0]oct-2-en-2-carbonsäure,
sowie ihre pharmazeutisch annehmbaren Salze und Hydrate dieser Säuren und Salze.

Die Verbindungen der Formel I bilden pharmazeutisch annehmbare Salze mit Basen. Beispiele von Salzen von Verbindungen der Formel I sind die Alkalimetallsalze, beispielsweise die Natrium- und Kaliumsalze, die Ammoniumsalze, die Erdalkalimetallsalze, beispielsweise Calciumsalze, die Salze mit organischen Basen, beispielsweise mit Aminen, wie Diisopropylamin, Benzylamin, Dibenzylamin, Triäthanolamin, Triäthylamin, N,N-Dibenzyläthylendiamin, N-Methylmorpholin, Pyridin, Piperazin, N-Aethylpiperidin, N-Methyl-D-glucamin und Procain, oder mit Aminosäuren, wie Arginin und Lysin. Je nach Zahl der sauren Gruppen der Verbindung der Formel I können Mono-, Di-, Trisalze etc. entstehen.

Die Verbindungen der Formel I, welche einen basischen Substituenten aufweisen, bilden auch innere Salze und Säureadditionssalze mit organischen und anorganischen Säuren. Beispiele von Säureadditionssalzen von Verbindungen der Formel I sind Salze mit Mineralsäuren, beispielsweise Halogenwasserstoffsäuren, wie Salzsäure, Bromwasserstoff und Jodwasserstoff, Schwefelsäure, Salpetersäure, Phosphorsäure und dergleichen, Salze mit organischen Sulfonsäuren, beispielsweise mit Alkyl- und Arylsulfonsäuren, wie Aethansulfonsäure, p-Toluolsulfonsäure, Benzolsulfonsäure und dergleichen, sowie Salze mit organischen Carbonsäuren, beispielsweise mit Essigsäure, Weinsäure, Maleinsäure, Zitronensäure, Benzoesäure, Salicyclsäure, Ascorbinsäure und dergleichen.

Die erwähnten, pharmazeutisch annehmbaren Salze können nach an sich bekannten und jedem Fachmann geläufigen Methoden hergestellt werden.

Bei den leicht hydrolysierbaren Estern von Verbindungen der Formel I handelt es sich vorzugsweise um Ester, welche unter milden Bedingungen hydrolysiert werden können, insbesondere um solche, welche unter physiologischen Bedingungen, beispielsweise enzymatisch hydrolysiert werden können. Beispiele solcher Ester, die herkömmlicher Art sein können, sind die 1-(niederen Alkanoyloxy)-niederen Alkylester, z.B. der Acetoxymethyl-, Pivaloyloxymethyl-, 1-Acetoxyäthyl- und der 1-Pivaloyloxyäthylester, die 1-(niederen Alkoxycarbonyloxy)niederen Alkylester, z.B. der (Methoxycarbonyloxy)methyl-, 1-(Aethoxycarbonyloxy)äthyl- und der 1-(Isopropoxycarbonyloxy)äthylester, die Laktonylester, z.B. der Phthalidyl- und der Thiophthalidylester, die 1-(niederen Alkoxy)-niederen Alkylester, z.B. der Methoxymethylester, die 1-(niederen Alkanoylamino)-niederen Alkylester, z.B. der Acetamidomethylester, die Benzylester, die Cyanomethylester und die (2-Oxo-1,3-dioxol-4-yl)methylester.

Allfällig in einer Verbindung der Formel I zusätzlich vorhandene Carboxygruppen können ebenfalls in Form von leicht hydrolysierbaren Estergruppen vorliegen.

Die leicht hydrolysierbaren Ester von Verbindungen der Formel I können nach an sich bekannten und jedem Fachmann geläufigen Verfahren hergestellt werden.

Die erfindungsgemässen Produkte können hydratisiert sein. Derartige Hydrate werden zumeist automatisch im Zuge des Herstellungsverfahrens oder als Folge hygroskopischer Eigenschaften eines zunächst wasserfreien Produktes erhalten. Zur gezielten Herstellung eines Hydrates kann ein ganz oder teilweise wasserfreies Produkt einer feuchten Atmosphäre ausgesetzt werden.

Die erfindungsgemässen Produkte, d.h. die Verbindungen der obigen Formel I, ihre leicht hydrolysierbaren Ester und pharmazeutisch annehmbaren Salze, sowie die Hydrate dieser Verbindungen, Ester und Salze, können erfindungsgemäss dadurch hergestellt werden, dass man

a) eine Verbindung der allgemeinen Formel

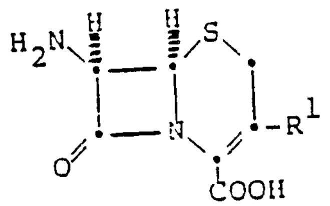

II

worin $R^1$ obige Bedeutung besitzt, oder einen leicht hydrolysierbaren Ester davon oder ein Säureadditionssalz einer dieser Verbindungen mit einer Verbindung der allgemeinen Formel

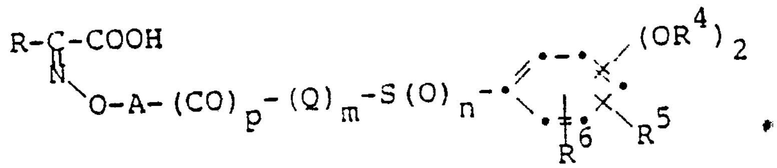

III

worin R, $R^4$, $R^5$, $R^6$, A, Q, m, n und p obige Bedeutung besitzen, acyliert, oder

b) eine Verbindung der allgemeinen Formel

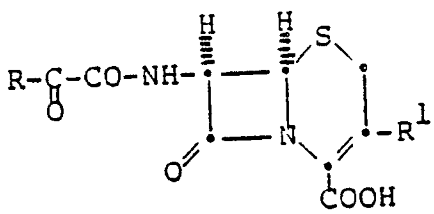

IV

worin R und $R^1$ obige Bedeutung besitzen, oder einen leicht hydrolysierbaren Ester davon gegebenenfalls in Gegenwart eines Kupfersalzes mit einer Verbindung der allgemeinen Formel

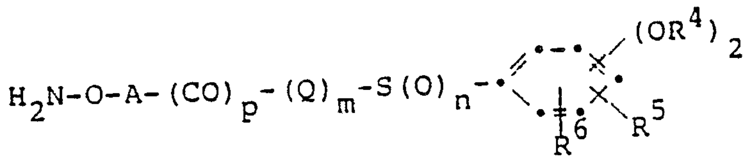

V

worin $R^4$, $R^5$, $R^6$, A, Q, m, n und p obige Bedeutung besitzen, oder einem Säureadditionssalz davon umsetzt, oder

c) eine Verbindung der allgemeinen Formel

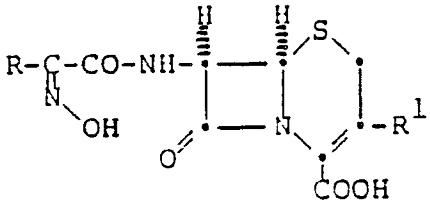

VI

worin R und $R^1$ obige Bedeutung besitzen, oder einen leicht hydrolysierbaren Ester davon mit einer Verbindung der allgemeinen Formel

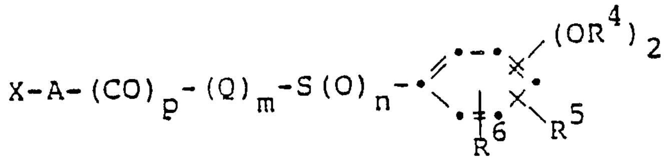

VII

worin X eine Abgangsgruppe bedeutet, und $R^4$, $R^5$, $R^6$, A, Q, m, n und p obige Bedeutung besitzen, alkyliert, oder

d) eine Verbindung der allgemeinen Formel

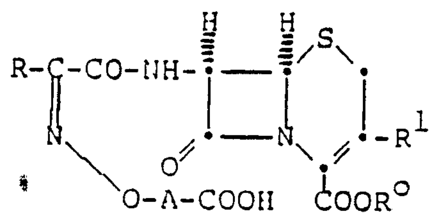

VIII

worin $R^o$ eine durch Hydrolyse entfernbare Gruppe bedeutet, und R, $R^1$ und A obige Bedeutung besitzen, mit einer Verbindung der allgemeinen Formel

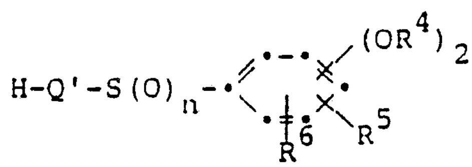

IX

worin Q′ die Gruppe $-NR^2-$ oder $-NR^2NR^3-$ bedeutet, und $R^2$, $R^3$, $R^4$, $R^5$, $R^6$ und n obige Bedeutung besitzen,
umsetzt, oder

e) eine Verbindung der allgemeinen Formel

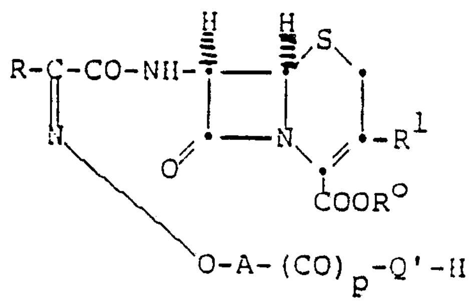

X

worin $R^o$, R, $R^1$, A, Q′ und p obige Bedeutung besitzen,
mit einem reaktiven Derivat einer Sulfonsäure der allgemeinen Formel

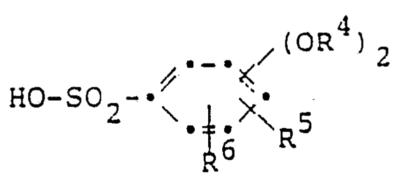

XI

worin $R^4$, $R^5$ und $R^6$ obige Bedeutung besitzen,
umsetzt, oder

f) eine Verbindung der allgemeinen Formel

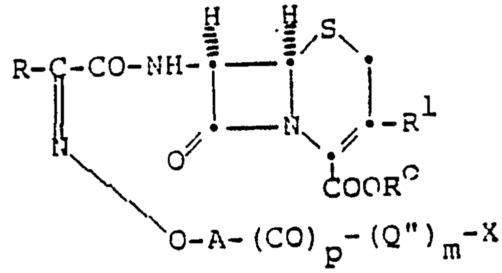

XII

worin Q″ gegebenenfalls durch Carboxy, Carbamoyl, niederes Alkylcarbamoyl oder Di(niederes Alkyl)-carbamoyl substituiertes niederes Alkylen oder $C_{3-7}$-Cycloalkylen bedeutet, und R°, R, R', A, p, m und X obige Bedeutung besitzen,
entweder in Gegenwart einer Base mit einer Sulfinsäure oder einem Mercaptan der allgemeinen Formel

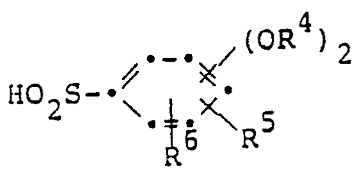

XIII bzw.

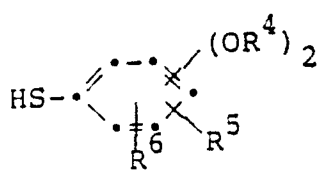

XIV

worin $R^4$, $R^5$ und $R^6$ obige Bedeutung besitzen,
oder mit einem Salz davon umsetzt, oder

g) eine Verbindung der allgemeinen Formel

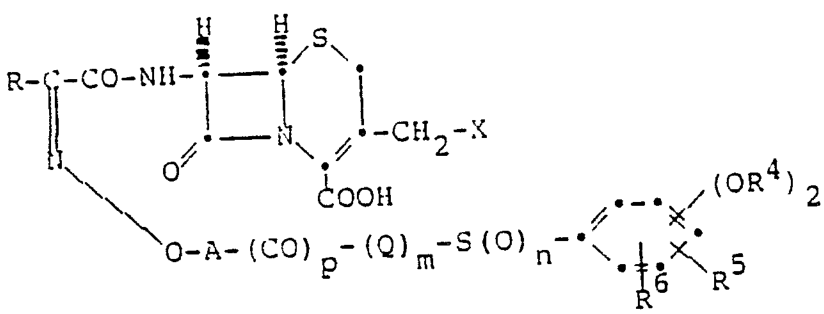

XV

worin R, $R^4$, $R^5$, $R^6$, A, Q, X, m, n und p obige Bedeutung besitzen,
oder einen leicht hydrolysierbaren Ester davon mit einer Verbindung der allgemeinen Formel
HS-R″ XVI
worin R″ eine über ein Kohlenstoffatom gebundene heterocyclische Gruppe bedeutet,
umsetzt,

h) erwünschtenfalls einen erhaltenen, leicht hydrolysierbaren Ester einer Verbindung der Formel I hydrolysiert und

i) erwünschtenfalls ein erhaltenes Produkt in ein pharmazeutisch annehmbares Salz und/oder ein Hydrat überführt.

Bei verschiedenen der obigen erfindungsgemässen Verfahren müssen allfällig vorhandene reaktionsfähige Amino-, Hydroxyl- und/oder Carboxygruppen durch Schutzgruppen blockiert sein. Diese Fälle sind für den Fachmann ohne weiteres erkennbar, und auch die Auswahl der jeweils geeigneten Schutzgruppen bereitet ihm keine Schwierigkeiten.

Es ist ferner möglich, dass die erfindungsgemässen Produkte als Mischungen mit den den erfindungsgemässen Produkten entsprechenden isomeren Produkten anfallen. Dabei kann es sich beispielsweise um [E]-konfigurierte Oxime oder um $\Delta^2$ -Isomere der erfindungsgemässen Cephalosporan-Derivate handeln. Die Abtrennung dieser Nebenprodukte, sowie deren allfällige Rückführung in erfindungsgemässe Produkte kann nach allgemein bekannten und jedem Fachmann geläufigen Methoden erfolgen. Es kommen dabei insbesondere chromatographische Methoden und Kristallisationsmethoden in Frage.

Die Acylierung gemäss Verfahrensvariante (a) des erfindungsgemässen Verfahrens kann nach an sich bekannten und jedem Fachmann geläufigen Methoden durchgeführt werden. Man kann beispielsweise die Verbindung der Formel II mit der freien Carbonsäure der Formel III oder einem Salz davon mit einer Base acylieren, wobei man in diesem Fall in Gegenwart eines geeigneten Kondensationsmittels arbeitet und die Verbindung der Formel II vorgängig in einen leicht hydrolysierbaren Ester überführt. Geeignete Kondensationsmittel sind beispielsweise N,N′-disubstituierte Carbodiimide, wie N,N′-Dicyclohexylcarbodiimid, welche vorzugsweise zusammen mit N-Hydroxybenzotriazol oder N-Hydroxysuccinimid verwendet werden, 2-Halogenpyridiniumsalze, wie 2-Chlor-1-methylpyridiniumchlorid, Phosphoroxychlorid, Thionylchlorid und Oxalylchlorid.

Es ist aber auch möglich, die Carbonsäure der Formel III in Form eines reaktiven Derivates einzusetzen. Als reaktionsfähige Derivate kommen insbesondere Säurechloride, Säureanhydride, gemischte Anhydride (beispielsweise Anhydride mit Trifluoressigsäure, Benzolsulfonsäure, Mesitylensulfonsäure, p-Toluolsulfonsäure und p-Chlorsulfonsäure) und aktive Thiolester, beispielsweise S-(2-Benzothiazolyl)thioester in Frage.

Die Acylierung kann gegebenenfalls in Gegenwart eines säurebindenden Mittels durchgeführt werden, wobei als säurebindende Mittel insbesondere Natriumhydrogencarbonat, Kaliumcarbonat, Triäthylamin, Pyridin oder N-Methylmorpholin in Frage kommen. Geeignete Lösungsmittel sind beispielsweise cyclische Aether, wie Tetrahydrofuran und Dioxan, halogenierte niedere Kohlenwasserstoffe, wie Chloroform und Methylenchlorid, Dimethylformamid, Dimethylacetamid, Acetonitril, Aceton und Wasser, sowie Mischungen davon. Die Reaktionstemperatur kann in einem weiten Bereich variieren und liegt in der Regel zwischen -50 °C und 50 °C, vorzugsweise zwischen etwa -10 °C und 30 °C.

Die Oximbildung gemäss Verfahrensvariante b) des erfindungsgemässen Verfahrens kann ebenfalls nach an sich bekannten und jedem Fachmann geläufigen Methoden durchgeführt werden. Die Verbindung der Formel V wird dabei vorzugsweise in Form eines Säureadditionssalzes eingesetzt, beispielsweise als Hydrochlorid oder als p-Toluolsulfonat. Geeignete Lösungsmittel sind beispielsweise Wasser, niedere Alkohole, wie Methanol, cyclische Aether, wie Tetrahydrofuran und Dioxan, Acetonitril, Dimethylformamid und Dimethylacetamid, sowie Mischungen davon. In einer bevorzugten Ausführungsform verwendet man Dimethylacetamid als Lösungsmittel. In einer weiteren bevorzugten Ausführungsform arbeitet man in Gegenwart eines Kupfersalzes, wobei sowohl Kupfer(I)- als auch Kupfer(II)salze in Frage kommen. Geeignete Salze sind beispielsweise die entsprechenden Halogenide, z.B. Chloride und Bromide, Sulfate, Acetate, Nitrate, Oxide, Carbonate, Perchlorate und die Tetrafluoroborate. Die Reaktionstemperatur liegt in einem Bereich von -20 °C bis 40 °C, vorzugsweise von 0 °C bis 30 °C.

Die Alkylierung gemäss Verfahrensvariante c) des erfindungsgemässen Verfahrens kann ebenfalls nach an sich bekannten und jedem Fachmann geläufigen Methoden durchgeführt werden. Man kann beispielsweise die Verbindungen der Formeln VI und VII in Gegenwart einer anorganischen oder organischen Base und in einem inerten organischen Lösungsmittel, vorzugsweise in einem aprotischen Lösungsmittel, miteinander zur Umsetzung bringen. Geeignete Basen sind z.B. Kaliumcarbonat, Natriumhydrid oder tertiäre Amine, wie Triäthylamin. Geeignete Lösungsmittel sind Dimethylformamid, Dimethylacetamid, Dimethylsulfoxid und Aceton, sowie Mischungen davon.

Es ist aber auch möglich, das Oxim der Formel VI mit einer starken Base, wie Natrium- oder Lithiumhydrid, in das entsprechende Natrium- bzw. Lithiumsalz zu überführen, und dieses dann mit der Verbindung der Formel VII umzusetzen. Die oben erwähnten Lösungsmittel eignen sich auch für diese Verfahrensführung.

Die mit X bezeichnete Abgangsgruppe ist vorzugsweise ein Halogenatom, beispielsweise ein Chlor-, Brom- oder Jodatom, oder eine Arylsulfonyloxygruppe, beispielsweise die p-Toluolsulfonyloxygruppe. Die Reaktion wird gewöhnlich bei Temperaturen zwischen -70 °C und 60 °C, vorzugsweise zwischen -20 °C und 20 °C durchgeführt.

Gemäss Verfahrensvariante d) des erfindungsgemässen Verfahrens können leicht hydrolysierbare Ester von Verbindungen der Formel I hergestellt werden, worin p und m die Zahl 1 und Q die Gruppe $-NR^2-$ oder $-NR^2NR^3-$ bedeuten, und $R^2$ und $R^3$ und die übrigen Substituenten die obige Bedeutung besitzen. Auch dieses Verfahren kann nach an sich bekannten und jedem Fachmann geläufigen Methoden durchgeführt werden. Man kann beispielsweise die weiter oben für die Verfahrensvariante a) angegebenen Reaktionsbedingungen anwenden.

Gemäss Variante e) des erfindungsgemässen Verfahrens können leicht hydrolysierbare Ester von Verbindungen der Formel I hergestellt werden, worin m die Zahl 1, n die Zahl 2 und Q′ die Gruppe $-NR^2-$ oder $-NR^2NR^3-$ bedeuten, und $R^2$, $R^3$ und die übrigen Symbole die obige Bedeutung besitzen. Auch dieses Verfahren kann nach an sich bekannten und jedem Fachmann geläufigen Methoden durchgeführt werden.

Als reaktives Sulfonsäurederivat verwendet man vorzugsweise ein Sulfonsäurehalogenid, insbesondere ein Sulfonsäurechlorid. Die Reaktion wird vorzugsweise in Gegenwart einer Base, z.B. in Gegenwart eines tertiären Amins, wie Triäthylamin, durchgeführt. Geeignete Lösungsmittel sind beispielsweise cyclische Aether, wie Tetrahydrofuran und Dioxan, offenkettige Aether, wie Diäthyläther, halogenierte niedere Kohlenwasserstoffe, wie Chloroform und Methylenchlorid, Dimethylformamid, Dimethylacetamid, Acetonitril und Aceton. Die Reaktion kann in einem Temperaturbereich von -50° C bis 50° C, vorzugsweise zwischen -10° C und 30° C durchgeführt werden.

Gemäss Variante f) des erfindungsgemässen Verfahrens können leicht hydrolysierbare Ester von Verbindungen der Formel I hergestellt werden, worin Q gegebenenfalls durch Carboxy, Carbamoyl, niederes Alkylcarbamoyl oder Di(niederes Alkyl)carbamoyl substituiertes niederes Alkylen oder $C_{3-7}$-Cycloalkylen und n die Zahl 0 oder 2 bedeuten, und die übrigen Symbole die obige Bedeutung besitzen. Auch dieses Verfahren kann nach an sich bekannten und jedem Fachmann geläufigen Methoden durchgeführt werden. Die Reaktion kann beispielsweise in einem inerten Lösungsmittel, beispielsweise in einem halogenierten niederen Kohlenwasserstoff, wie Methylenchlorid und Chloroform, in einem offenkettigen Aether, wie Diäthyläther, in Dimethylformamid, Dimethylacetamid, Dimethylsulfoxid oder in Aceton, in Gegenwart einer Base, wie Kaliumcarbonat oder einem tertiären Amin, wie Triäthylamin, durchgeführt werden. Es ist aber auch möglich, die Sulfinsäure der Formel XIII bzw. das Mercaptan der Formel XIV in Form eines Salzes einzusetzen, wobei insbesondere die Lithium-, Natrium- und Kaliumsalze für diesen Zweck in Frage kommen. Die Reaktion kann in einem weiten Temperaturbereich durchgeführt werden, wobei man jedoch vorzugsweise bei Raumtemperatur arbeitet.

Gemäss Variante g) des erfindungsgemässen Verfahrens können Verbindungen der allgemeinen Formel I hergestellt werden, worin $R^1$ die Gruppe $-CH_2-S-R''$ und $R''$ eine über ein Kohlenstoffatom gebundene heterocyclische Gruppe bedeuten, und die übrigen Symbole die obige Bedeutung besitzen. Auch dieses Verfahren kann nach an sich bekannten und jedem Fachmann geläufigen Methoden durchgeführt werden. Die mit X bezeichnete Abgangsgruppe ist vorzugsweise ein Halogenatom, z.B. Chlor, Brom oder Jod, eine niedere Alkyl-oder Arylsulfonyloxygruppe, wie Methansulfonyloxy oder p-Toluolsulfonyloxy, oder eine niedere Alkanoyloxygruppe, wie Acetoxy. Das Symbol X bedeutet vorzugsweise Acetoxy. Die Reaktion kann beispielsweise unter den gleichen Bedingungen durchgeführt werden, wie sie weiter oben für die Verfahrensvariante f) beschrieben sind.

Zur Herstellung der leicht hydrolysierbaren Ester der Carbonsäuren der Formel I gemäss Variante h) wird die Carbonsäure vorzugsweise mit dem entspechenden, die Estergruppe enthaltenden Halogenid, bevorzugt mit dem Jodid, umgesetzt. Die Reaktion kann mit Hilfe einer Base, z.B. einem Alkalimetallhydroxid oder Carbonat, oder einem organischen Amin, wie Triäthylamin, beschleunigt werden. Diese Reaktion wird vorzugsweise in einem inerten organischen Lösungsmittel durchgeführt, wie Dimethylacetamid, Hexamethylphosphorsäuretriamid, Dimethylsulfoxid oder, vorzugsweise, Dimethylformamid. Die Temperatur liegt vorzugsweise im Bereich von etwa 0 bis 40° C.

Die Herstellung der Salze und Hydrate der Verbindungen der Formel I bzw. der Hydrate dieser Salze gemäss Variante i) kann in an sich bekannter Weise erfolgen, z.B. durch Umsetzung der Carbonsäure der Formel I oder einem Salz davon mit einer äquivalenten Menge der gewünschten Base, zweckmässig in einem Lösungsmittel wie Wasser, oder in einem organischen Lösungsmittel, wie Aethanol, Methanol, Aceton oder anderen mehr. Entsprechend wird Salzbildung durch Addition einer organischen oder anorganischen Säure herbeigeführt. Die Temperatur der Salzbildung ist nicht kritisch. Sie liegt im allgemeinen bei Raumtemperatur, kann aber auch leicht darüber oder darunter, etwa im Bereiche von 0° C bis +50° C, sein.

Die Herstellung der Hydrate erfolgt zumeist automatisch im Zuge des Herstellungsverfahrens oder als Folge hygroskopischer Eigenschaften eines zunächst wasserfreien Produktes. Zur gezielten Herstellung eines Hydrats kann ein ganz oder teilweise wasserfreies Produkt (Carbonsäure der Formel I bzw. Ester oder Salze davon) einer feuchten Atmosphäre, z.B. bei etwa +10° bis +40° C, ausgesetzt werden.

Die verschiedenen als Ausgangsstoffe verwendeten Verbindungen sind bekannt oder können nach an sich bekannten Methoden und ausgehend von bekannten Ausgangsstoffen hergestellt werden. Die nachfolgenden Beispiele enthalten detaillierte Angaben betreffend die Herstellung dieser Ausgangsstoffe.

Wie bereits erwähnt besitzen die erfindungsgemässen Produkt wertvolle pharmakologische, insbesondere antibiotische Eigenschaften. Sie besitzen eine weites Wirkungsspektrum gegen gram-positive und gram-negative Mikroorganismen.

Zum Nachweis der antimikrobiellen Wirksamkeit der erfindungsgemässen Produkte wurden verschiedene der gemäss den nachstehenden Ausführungsbeispielen hergestellten Verbindungen auf ihre Aktivität in vitro getestet. Die ermittelten Aktivitäten (Mindesthemmkonzentration in µg/ml sind in den nachfolgenden Tabellen zusammengestellt:

Tabelle I

| Erreger | Endprodukt aus Beispiel | | | | | | | | |
|---|---|---|---|---|---|---|---|---|---|
| | 1 | 2 | 3 | 5 | 6 | 7 | 8 | 9 | 10 |
| E. coli 25922 | <0,06 | <0,06 | <0,06 | <0,06 | <0,06 | <0,06 | <0,06 | <0,06 | 0,12 |
| E. coli TEM 1 | <0,06 | <0,06 | <0,06 | <0,06 | <0,06 | <0,06 | <0,06 | <0,06 | <0,06 |
| K. pneumoniae 418 | <0,06 | <0,06 | <0,06 | <0,06 | <0,06 | <0,06 | <0,06 | <0,06 | <0,06 |
| K. oxytoca 1082 E | 0,25 | 0,12 | <0,06 | <0,06 | 1 | <0,06 | 0,12 | 0,12 | 0,12 |
| E. cloacae 15 M | 0,12 | 0,12 | 0,25 | <0,06 | <0,06 | <0,06 | - | 0,5 | 1 |
| S. marcescens 80315 | 0,12 | <0,06 | <0,06 | <0,06 | 0,12 | <0,06 | <0,06 | 0,12 | 0,25 |
| P. mirabilis 2117 | 0,12 | 0,12 | 0,12 | <0,06 | <0,06 | 0,12 | 0,12 | 0,25 | 0,12 |
| P. vulgaris 1028 | 0,12 | 0,12 | 0,12 | <0,06 | 0,25 | 0,12 | <0,06 | 0,12 | 0,25 |
| P. aeruginosa BA | 0,25 | 0,25 | 0,25 | 0,12 | 1 | 0,25 | 0,25 | 1 | 0,5 |
| S. pyogenes B15 | 0,25 | 0,25 | 0,25 | 1 | 2 | 0,5 | 0,25 | 0,5 | 2 |
| M. morganii 6H-137l | 0,25 | 0,25 | 1 | 0,25 | <0,06 | 0,5 | <0,06 | 0,25 | 0,5 |
| C. freundii 902 | 0,25 | 0,25 | 0,25 | 0,12 | 0,12 | 0,12 | <0,06 | 0,25 | 0,25 |

Tabelle II

| Erreger | Endprodukt aus Beispiel | |
|---|---|---|
| | 7 | 15 |
| E. coli 25922 | 0,12 | <0,06 |
| E. coli TEM 1 | <0,06 | <0,06 |
| K. pneumoniae 418 | <0,06 | <0,06 |
| K. oxytoca 1082 E | 0,25 | 0,5 |
| E. cloacae 15 M | 0,5 | 0,5 |
| S. marcescens 80315 | <0,06 | <0,06 |
| P. mirabilis 2117 | 0,25 | 0,12 |
| P. vulgaris 1028 | 0,25 | 0,12 |
| P. aeruginosa BA | 0,25 | 0,12 |
| S. pyogenes B15 | 0,5 | 0,5 |
| M. morganii 6H-137l | 0,5 | 0,25 |
| C. freundii 902 | 0,25 | 0,12 |

Die erfindungsgemässen Produkte können als Heilmittel, z.B. in Form pharmazeutischer Präparate zur enteralen oder parenteralen Applikation, Verwendung finden. Die erfindungsgemässen Produkte können beispielsweise peroral, z.B. in Form von Tabletten, Lacktabletten, Dragées, Hart- und Weichgelatinekapseln, Lösungen, Emulsionen oder Suspensionen, rektal, z.B. in Form von Suppositorien, oder parenteral, z.B. in Form von Injektionslösungen, verabreicht werden.

Die Herstellung der pharmazeutischen Präparate kann in jedem Fachmann geläufiger Weise dadurch erfolgen, dass man die erfindungsgemässen Stoffe, gegebenenfalls in Kombination mit anderen therapeutisch wertvollen Stoffen, zusammen mit geeigneten, nicht-toxischen, inerten, therapeutisch verträglichen festen oder flüssigen Trägermaterialien und gegebenenfalls den üblichen pharmazeutischen Hilfsstoffen in eine galenische Darreichungsform bringt.

Als solche Trägermaterialien eignen sich sowohl anorganische als auch organische Trägermaterialien. So kann man für Tabletten, Lacktabletten, Dragées und Hartgelatinekapseln beispielsweise Lactose, Maisstärke oder Derivate davon, Talk, Stearinsäure oder deren Salze als Trägermaterialien verwenden. Für Weichgelatinekapseln eignen sich als Träger beispielsweise pflanzliche Oele, Wachse, Fette und halbfeste und flüssige Polyole (je nach Beschaffenheit des Wirkstoffes sind jedoch bei Weichgelatinekapseln keine Träger erforderlich). Zur Herstellung von Lösungen und Sirupen eignen sich als Trägermaterialien beispielsweise Wasser, Polyole, Saccharose, Invertzucker und Glucose. Für Injektionslösungen eignen sich als Trägermaterialien beispielsweise Wasser, Alkohole, Polyole, Glycerin und pflanzliche Oele. Für Suppositorien eignen sich als Trägermaterialien beispielsweise natürliche oder gehärtete Oele, Wachse, Fette und halbflüssige oder flüssige Polyole.

Als pharmazeutische Hilfsstoffe kommen die üblichen Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel und Antioxydantien in Frage.

Die Verbindungen der Formel I und ihre Salze bzw. Hydrate kommen insbesondere für die parenterale Applikation in Betracht und werden zu diesem Zweck vorzugsweise als Lyophilisat oder Trockenpulver zur Verdünnung mit üblichen Trägern, wie Wasser oder isotonischer Kochsalzlösung, zur Verfügung gestellt. Die leicht hydrolysierbaren Ester von Verbindungen der Formel I und ihre Salze und Hydrate kommen insbesondere für die enterale Applikation in Betracht.

Die pharmazeutischen Präparate können die erfindungsgemässen Stoffe in Mengen von etwa 25-2000 mg, vorzugsweise 100-1000 mg, pro Einzeldosierungsform enthalten. Für die Prophylaxe und Therapie von Infektionskrankheiten kommt für den Erwachsenen eine tägliche Dosis von etwa 0,05 g bis etwa 4 g, insbesondere etwa 0,1 g bis etwa 2 g in Betracht.

Die nachfolgenden Beispiele sollen die vorliegende Erfindung näher erläutern, ihren Umfang jedoch in keiner Weise beschränken. Sämtliche Temperaturen sind in Celsiusgraden angegeben.

## Beispiel 1

In einem ersten Kolben wird eine Lösung von 74 mg 2-Amino-4-thiazolglyoxylsäure-(Z) -O-[[(3,4-dihydroxyphenyl)sulfonyl]methyl]oxim in 1 ml N,N-Dimethylformamid mit 83 mg N,O-Bis-(trimethylsilyl)-acetamid versetzt und während 15 Minuten bei 20°C gerührt. Die Lösung wird auf 0°C gekühlt und nacheinander mit 39 mg 1-Hydroxy-benzotriazol und 51 mg N,N′-Dicyclohexylcarbodiimid versetzt. Das Reaktionsgemisch wird 1 Stunde bei 0°C gerührt, wobei sich ein Niederschlag bildet.

In einem zweiten Kolben werden 64 mg (7R)-7-Aminocephalosporansäure und 124 mg N,O-Bis-(trimethylsilyl)acetamid in 1 ml N,N-Dimethylformamid während 20 Minuten bei 20°C gerührt. Es entsteht eine klare Lösung, welche bei 0°C zu der Lösung im ersten Kolben zugegeben wird. Das Reaktionsgemisch wird 1 Stunde bei 0°C und 2 Stunden bei 20°C gerührt. Der Niederschlag wird abgenutscht, und das Filtrat wird zwischen 2-%iger wässriger Natriumhydrogencarbonlösung und Aethylacetat verteilt. Die Wasserphase wird mit 3N Salzsäure auf pH 7 gestellt, im Vakuum bis auf ein Volumen von ca. 5 ml eingeengt, und die konzentrierte Lösung wird an MCl-Gel CHP20P (Mitsubishi Chemical Industries, Ltd.) chromatographiert. Man eluiert zunächst mit 1-%iger wässriger Essigsäure und anschliessend mit Gemischen von 1-%iger wässriger Essigsäure/Methanol bei steigenden Anteilen von Methanol. Durch Lyophilisieren der im Vakuum eingeengten Produktfraktionen erhält man (6R,7R)-3-(Acetoxymethyl)-7-[(Z)-2-(2-amino -4-thiazolyl)-2-[[[(3,4-dihydroxyphenyl)sulfonyl] methoxy]imino]acetamido]-8-oxo-5-thia -1-azabicyclo[4.2.0]oct-2-en-2-carbonsäure als weisses Pulver.

$^1$H NMR (DMSO-$d_6$): δ 2,03 (s, 3H); 3,56 (d, J=22 Hz, 1H, Signal des Kopplungspartners überlagert durch $H_2O$-Signal bei 3,45); 4,70 (d, J=12,5 Hz, 1H); 4,99 (d, J=12,5 Hz, 1H); 5,08 (d, J=5Hz, 1H); 5,17 (d, J=12,5 Hz, 1H); 5,25 (d, J=12,5 Hz, 1H); 5,69 (dd, J=8 und 5 Hz, 1H); 6,72 (s, 1H); 6,90 (d, J=8 Hz, 1H); 7,19 (d, J=1 Hz, 1H); 7,22 (dd, J=8 und 1 Hz, 1H); 7,32 (breites s, 2H); 9,66 (breites d, J=8 Hz, 1H) ppm.

Das als Ausgangsmaterial eingesetzte 2-Amino-4-thiazolglyoxylsäure-(Z) -O-[[(3,4-dihydroxyphenyl)-sulfonyl]methyl]oxim kann wie folgt hergestellt werden:

a) Zu 220 ml auf -40°C vorgekühltem Diäthyläther werden innerhalb 3 Minuten 135 ml einer, 1,7M Butyllithium/n-Hexan-Lösung zugetropft. Die Lösung wird auf -50°C gekühlt und dann innerhalb 5 Minuten mit einer Lösung von 50,4 g 5-Brom-2,2-dimethyl-1,3-benzodioxol in 100 ml Diäthyläther versetzt, wobei die Temperatur bis auf -35°C ansteigt. Die Reaktionslösung wird auf -10°C erwärmt und 30 Minuten bei dieser Temperatur gerührt. In die anschliessend auf -60°C abgekühlte Reaktionslösung werden portionenweise 6,6 g Schwefel eingetragen, wobei die Temperatur auf -25°C ansteigt. Das Reaktionsgemisch wird noch 15 Minuten bei 0°C gerührt. Dann werden 34 g Methyljodid zugegeben, und das Reaktionsgemisch wird 1 Stunde bei 0°C gerührt. Die Reaktionslösung wird mit Aethylacetat verdünnt und nacheinander mit 200 ml 2N wässriger Natronlauge und 200 ml Wasser gewaschen. Die organische Phase wird über Natriumsulfat getrocknet, und das Lösungsmittel wird im Vakuum entfernt. Das verbleibende Oel wird im Vakuum über eine 60 cm lange Vigreux-Kolonne fraktioniert. Man erhält 2,2-Dimethyl-5-(methylthio) -1,3-benzodioxol als farbloses Oel vom Siedepunkt 80-83°C (20 Pa).

b) Eine Lösung von 19,6 g 2,2-Dimethyl-5-(methylthio)-1,3-benzodioxol in 500 ml Methylenchlorid wird bei 0° C mit 20,3 g 85-%iger m-Chlorperbenzoesäure versetzt. Anschliessend wird 1 Stunde bei 0° C gerührt, wobei ein weisser Niederschlag entsteht. Das Reaktionsgemisch wird mit 400 ml 17-%iger wässriger Natriumcarbonat-Lösung und 400 ml Wasser extrahiert. Die organische Phase wird über Natriumsulfat getrocknet und im Vakuum vom Lösungsmittel befreit. Der Rückstand wird in wenig Methylenchlorid aufgenommen und an Kieselgel chromatographiert, wobei mit Aethylacetat/n-Hexan/ Methylenchlorid (1:1:1, v/v/v) eluiert wird. Man erhält nach Kristallisation aus Aethylacetat/n-Hexan 2,2-Dimethyl-5-(methylsulfinyl) -1,3-benzodioxol als weisse Kristalle vom Schmelzpunkt 87-88° C.

c) Zu einer auf 0° C gekühlten Lösung von 16,5 g 2,2-Dimethyl-5-(methylsulfinyl) -1,3-benzodioxol und 6,2 g Pyridin in 230 ml Methylenchlorid gibt man nacheinander 5,43 g Brom und 12,1 g N-Bromsuccinimid. Das Reaktionsgemisch wird 3 Stunden bei 0° C und dann noch 12 Stunden bei 20° C gerührt. Die orangefarbene Lösung wird auf 0° C gekühlt, mit 40 ml 1M wässriger Natriumsulfitlösung versetzt, und der pH des Gemisches wird durch Zugabe von 17-%iger wässriger Natriumcarbonatlösung auf 7 gestellt. Nach 10-minütigem Rühren werden die Phasen getrennt. Die organische Phase wird nacheinander mit 50 ml 1N wässriger Salzsäure, 50 ml 5-%iger Natriumhydrogencarbonatlösung und 3 mal mit je 100 ml Wasser gewaschen, über Natriumsulfat getrocknet und im Vakuum eingedampft. Der Rückstand wird an Kieselgel chromatographiert, wobei mit Aethylacetat/Hexan (1:2, v/v) eluiert wird. Man erhält nach Kristallisation aus Aceton/Wasser 5-[(Brommethyl)sulfinyl] -1,3-benzodioxol als weisse Kristalle vom Schmelzpunkt 88-89° C.

d) Ein Gemisch von 8,73 g 5-[(Brommethyl)sulfinyl]-2,2-dimethyl -1,3-benzodioxol, 6,45 g (Z)-2-[Amino-α-(hydroxyimino)] -4-thiazolessigsäure-äthylester und 4,97 g Kaliumcarbonat in 36 ml Dimethylsulfoxid wird 2 Stunden bei 75° C gerührt. Nach dem Abkühlen wird das Reaktionsgemisch mit 100 ml Aethylacetat verdünnt und 4 mal mit je 50 ml 5-%iger wässriger Natriumchloridlösung extrahiert. Die organische Phase wird über Natriumsulfat getrocknet und im Vakuum vom Lösungsmittel befreit. Der Rückstand wird in 60 ml Methylenchlorid aufgenommen. Ungelöstes Material wird durch Filtration abgetrennt, und das Filtrat wird bei 20° C mit 9,4 g 55-%iger m-Chlorperbenzoesäure versetzt. Das Reaktionsgemisch wird 1 Stunde bei 20° C gerührt, dann mit Methylenchlorid verdünnt und nacheinander mit 17-%iger wässriger Natriumcarbonatlösung und Wasser gewaschen. Die organische Phase wird über Natriumsulfat getrocknet und im Vakuum vom Lösungsmittel befreit. Der Rückstand wird am Kieselgel chromatographiert. Ein Gemisch von Aethylacetat/ n-Hexan (1:1, v/v) eluiert das Produkt, welches aus Aethylacetat/n-Hexan kristallisiert wird. Man erhält 2-Amino-4-thiazolglyoxylsäure-äthylester -(Z)-O-[[[(3,4-(isopropylidendioxy)phenyl]sulfonyl] methyl]oxim als weisse Kristalle vom Schmelzpunkt 160-161° C.

e) Eine Lösung von 441 mg 2-Amino-4-thiazolglyoxylsäure-äthylester -(Z)-O-[[[(3,4-isopropylidendioxy)phenyl]sulfonyl] methyl]oxim in 5M äthanolischer Salzsäure wird während 3 Stunden auf 80° C erhitzt. Die Reaktionslösung wird genutscht, und das Lösungsmittel wird im Vakuum entfernt. Der Rückstand wird in 2 ml Aethanol und 2 ml 2N wässriger Natronlauge aufgenommen. Die erhaltene dunkle Lösung wird 30 Minuten bei 20° C gerührt. Dann werden 4 ml Wasser zugegeben, und der pH wird mit 3N wässriger Salzsäure auf 7 gestellt. Die Lösung wird im Vakuum auf ca. 3 ml konzentriert und dann an MCl-Gel CHP20P chromatographiert. Man eluiert zunächst mit 2-%iger wässriger Essigsäure, dann mit Wasser/Methanol-Gemischen mit steigenden Anteilen von Methanol. Mit Wasser/Methanol (9:1, v/v) wird das Produkt eluiert, welches aus Methanol/Diäthläther kristallisiert wird. Man erhält 2-Amino-4-thiazolglyoxylsäure -(Z)-O-[[(3,4-dihydroxyphenyl)sulfonyl]methyl]oxim als weisse Kristalle vom Schmelzpunkt 167° C (Zers.).

$^1$H NMR (DMSO-$d_6$): δ 5,24 (s, 2H); 6.73 (s, 1H); 6,88 (d, J = 8 Hz, 1H); 7,19 (dd, J = 8 und 1 Hz, 1H); 7,23 (d, J = 1Hz, 1H); 7,26 (s, 2H); 9.79 (breites s, 1H); 10,15 (breites s, 1H) ppm.

## Beispiel 2

Wird in dem in Beispiel 1 beschriebenen Verfahren (7R)-7-Aminocephalosporansäure durch die äquimolare Menge von (6R,7R)-7-Amino -3-[[(5-methyl-1,3,4-thiadiazol-2-yl)thio]methyl] -8-oxo-5-thia-1-azabicyclo[4.2.0]oct -2-en-2-carbonsäure ersetzt, so erhält man nach chromatographischer Reinigung an MCl-Gel und Lyophilisieren der Produktfraktionen (6R,7R)-7-[(Z)-2-(Amino-4-thiazolyl) -2-[[[(3,4-dihydroxyphenyl)sulfonyl]methoxy]imino] acetamido]-3-[[(5-methyl-1,3,4-thiadiazol-2-yl)thio]methyl] -8-oxo-5-thia-1-azabicyclo[4.2.0]oct-2-en-2-carbonsäure als weisses Pulver.

$^1H$ NMR (DMSO-$d_6$): δ 2,68 (s, 3H); 3,44 (d, J = 19 Hz, 1H); 3,66 (d, J = 19 Hz, 1H); 4,20 (d, J = 13 Hz, 1H); 4,52 (d, J = 13 Hz, 1H); 5,06 (d, J = 5 Hz, 1H); 5,14 (d, J = 12,5 Hz, 1H); 5,21 (d, J = 12,5 Hz 1H); 5,64 (dd, J = 8 und 5 Hz, 1H); 6,70 (s, 1H); 4,89 (d, J = 8 Hz, 1H); 7,19 (d, J = 1 Hz, 1H); 7,21 (dd, J = 8 und 1 Hz, 1H); 7,30 (s, 2H) ppm.

## Beispiel 3

Wird in dem in Beispiel 1 beschriebenen Verfahren (7R)-7-Aminocephalosporansäure durch die äquimolare Menge (6R,7R)-7-Amino-3-(azidomethyl) -8-oxo-5-thia-1-azabiclyclo[4.2.0] oct -2-en-2-carbonsäure ersetzt, so erhält man nach chromatographischer Reinigung an MCI-Gel und lyophilisieren der Produktfraktionen (6R,7R)-7-[(Z)-2-(Amino -4-thiazolyl)-2-[[[(3,4-dihydroxyphenyl)sulfonyl]methoxy] imino]acetamido]-3-(azidomethyl) -8-oxo-5-thia-1-azabicyclo[4.2.0]oct-2-en-2-carbonsäure als weisses Pulver.
$^1H$ NMR (DMSO-$d_6$): δ 3,45 (d, J = 19 Hz, 1H); 3,61 (d, J = 19 Hz, 1H); 3,91 (d, J = 14 Hz, 1H); 4,36 (d, J = 14 Hz, 1H); 5,10 (d, J = 5 Hz, 1H); 5,16 (d, J = 13 Hz, 1H); 5,24 (d, J = 13 Hz, 1H); 5,76 (dd, J = 8 und 5 Hz, 1H); 6,69 (s, 1H); 6,92 (d, J = 9 Hz, 1H); 7,20 (m, 2H); 7,31 (s, 2H); 9,69 (s, 1H); 9,76 (d, J = 8 Hz, 1H); 10,12 (s, 1H) ppm.

## Beispiel 4

Wird in dem in Beispiel 1 beschriebenen Verfahren (7R)-7-Aminocephalosporansäure durch die äquimolare Menge 1-[[(6R,7R)-7-Amino-2-carboxy-8-oxo -5-thia-1-azabicylco[4.2.0]oct-2-en-3-yl]methyl] pyridinium-betain-hydrochlorid ersetzt, so erhält man nach chromatographischer Reinigung an MCI-Gel und Lyophilisieren der Produktfraktionen 1-[(6R,7R)-7-[(Z)-2-(2-Amino -4-thiazolyl)-2-[[[3,4-dihydroxyphenyl)-sulfonyl]methoxy] imino]acetamido]-2-carboxy -8-oxo-5-thia-1-azabicyclo[4.2.0]oct-2-en-3-yl] pyridiniumbetain als weisses Pulver.
$^1H$ NMR (DMSO-$d_6$): δ u.a. 2,97 (d, J = 18 Hz, 1H); 3,46 (d, J = 18 Hz, 1H); 6,67 (s, 1H); 6,89 (d, J = 8 Hz, 1H); 7,18 (m, 2H); 7,29 (breites s, 2H) ppm.

## Beispiel 5

In einem ersten Kolben wird eine Lösung von 52 mg 2-Amino-4-thiazolglyoxylsäure-(Z) -O-[[(3,4-dihydroxyphenyl) sulfonyl]methyl]oxim in 0,7 ml N,N-Dimethylformamid auf 0°C gekühlt und mit 27 mg 1-Hydroxy-benzotriazol und 36 mg N,N′-Dicyclohexylcarbodiimid versetzt. Das Reaktionsgemisch wird 1 Stunde bei 0°C gerührt, wobei sich ein Niederschlag bildet.

In einem zweiten Kolben werden 74 mg (6R,7R)-7-Amino-3-[[(2-carboxy -5-methyl-s-triazolo[1,5-a]-pyrimidin-7-yl)thio]methyl] -8-oxo-5-thia-1-azabicyclo[4.2.0]oct-2-en-2-carbonsäure und 86 mg N,O-Bis-(trimethylsilyl)acetamid in 0,7 ml N,N-Dimethylformamid während 30 Minuten bei 20°C gerührt. Es entsteht eine klare Lösung, welche bei 0°C zu der Lösung im ersten Kolben zugegeben wird. Das Reaktionsgemisch wird 2 Stunden bei 20°C gerührt und dann zwischen Aethylacetat und 2-%iger wässriger Natriumhydrogencarbonatlösung verteilt. Die Wasserphase wird mit 3N wässriger Salzsäure auf pH 7 gestellt, im Vakuum bis auf ein Volumen von ca. 5 ml eingeengt, und die konzentrierte Lösung wird an MCI-Gel CHP20P chromatographiert. Man eluiert zuerst mit 1-%iger Essigsäure und später mit Gemischen von 1-%iger wässriger Essigsäure/Methanol bei steigenden Anteilen von Methanol. Durch Lyophilisieren der im Vakuum konzentrierten Produktfraktionen erhält man (6R,7R)-7-[(Z)-(2-Amino -4-thiazolyl)-2-[[[(3,4-dihydroxyphenyl)sulfonyl]methoxy] imino]acetamido] -3-[[(2-carboxy-5-methyl-s-triazolo[1,5-a]pyrimidin -7-yl)thio]-methyl]-8-oxo-5-thia -1-azabicyclo[4.2.0]oct-2-en-2-carbonsäure als weisses Pulver.
$^1H$ NMR (DMSO-$d_6$): δ 2,62 (s, 3H); 3,53 (d, J = 19 Hz, 1H); 3,74 (d, J = 19 Hz, 1H); 4,38 (d, J = 13 Hz, 1H); 4,47 (d, J = 13 Hz, 1H); 5,12 (d, J = 5 Hz, 1H); 5,15 (d, J = 12 Hz, 1H); 5,23 (d, J = 12 Hz, 1H); 5,75 (dd, J = 8 und 5 Hz, 1H); 6,69 (s, 1H); 6,92 (d, J = 9 Hz, 1H); 7,20 (m, 2H); 7,30 (breites s, 2H); 7,45 (s, 1H); 9,68 (breites s, 1H); 9,72 (d, J = 8 Hz, 1H); 10,14 (breites s, 1H) ppm.

## Beispiel 6

Nach dem in Beispiel 5 beschriebenen Verfahren werden 37 mg 2-Amino-4-thiazolglyoxylsäure-(Z) -O-[-[(3,4-dihydroxyphenyl)sulfonyl]methyl]oxim mit 47 mg (6R,7R)-7-Amino-3-[[(2,5-dihydro -6-hydroxy-2-methyl-5-oxo-as-triazin -3-yl)thio]methyl]-8-oxo-5-thia-1-azabicyclo[4.2.0]oct -2-en-2-carbonsäure umgesetzt. Die bei der Chromatographie an MCI-Gel nach der im Beispiel 1 beschriebenen Methode erhaltenen Produktfraktionen werden konzentriert, und der pH der erhaltenen Lösung wird mit verdünnter Natronlauge auf 7 gestellt. Durch Lyophilisieren erhält man (6R,7R)-7-[(Z)-2-(2-Amino-4-thiazolyl) -2-[[[(3,4-dihydrophenyl)sulfonyl]methoxy]imino] acetamido]-3-[[(2,5-dihydro-6-hydroxy-2-methyl-5-oxo-as -triazin-3-yl)thio]-methyl]-8-oxo-5-thia -1-azabicyclo[4.2.0]oct -2-en-2-carbonsäure-Dinatriumsalz als blassgelbes Pulver.

$^1$H NMR (DMSO-$d_6$): δ u.a. 4,91 (d, J=5 Hz, 1H); 5,09 (d, J=13 Hz, 1H); 5,19 (d, J=13 Hz, 1H); 5,43 (m, 1H); 6,77 (s, 1H) ppm.

## Beispiel 7

48 mg 4-[[(Aminooxy)methyl]sulfonyl]pyrokatechol werden in 2 ml 1N äthanolischer Salzsäure gelöst, und die Lösung wird in Vakuum vollständig eingeengt. Der Rückstand wird in 3 ml Aethanol gelöst, und das Lösungsmittel wird im Vakuum vollständig entfernt. Das rohe Hydrochlorid wird zusammen mit 90 mg (6R,7R)-7-(2-Amino-4-thiazolglyoxylamido)-3-[[(2-carbamoyl-5-methyl-s-triazolo[1,5-a]pyrimidin-7-yl)thio]-methyl]-8-oxo-5-thia-1-azabicyclo[4.2.0]oct-2-en-2-carbonsäure in 0,5 ml N,N-Dimethylacetamid gelöst, und die Lösung wird 64 Stunden bei Raumtemperatur gerührt. Die Reaktionslösung wird bei 20° C langsam mit 5 ml Wasser versetzt, wobei ein Niederschlag entsteht, der abgenutscht wird. Das so erhaltene Rohprodukt wird zur Reinigung in 3 ml Wasser durch Zugabe von wenig 1N Natronlauge in Lösung gebracht und dann an MCI-Gel CHP20P (Mitsubishi Chemical Industries, Ltd.) chromatographiert. Man eluiert zunächst mit 1-%iger wässriger Essigsäure und dann mit Gemischen von 1-%iger wässriger Essigsäure/Methanol bei steigenden Anteilen von Methanol. Durch Einengen und Lyophilisieren der Produktfraktionen erhält man (6R, 7R)-7-[(Z)-2-(2-Amino-4-thiazolyl)-2-[[[(3,4-dihydroxyphenyl)sulfonyl]methoxy]imino]acetamido] -3-[[(2-carbamoyl-5-methyl-s-triazolo[1,5-a]pyrimidin-7-yl)thio]methyl]-8-oxo-5-thia -1-azabicyclo[4.2.0]oct-2-en-2-carbonsäure als weisses Pulver.

$^1$H NMR (DMSO-$d_6$):δ 2,61 (s, 3H); 3,50 (d, J=18 Hz, 1H); 3,77 (d, J=18 Hz, 1H); 4,38 (d, J=14 Hz, 1H); 4,43 (d, J=14 Hz, 1H); 5,14 (d, J=5 Hz, 1H); 5,16 (d, J=14 Hz, 1H); 5,24 (d, J=14 Hz, 1H); 5,74 (dd, J=8 und 5 Hz, 1H); 6,69 (s, 1H); 6,91 (d, J=8 Hz, 1H); 7,20 (m, 2H); 7,29 (breites s, 2H); 7,41 (s, 3H); 7,88 (s, 1H); 8,17 (s, 1H); 9,67 (s, 1H); 9,72 (d, J=8 Hz, 1H); 10,12 (s, 1H) ppm.

Eine Suspension von 373 mg (6R, 7R)-7-[(Z)-2-(2-Amino-4-thiazolyl)-2-[[[(3,4-dihydroxyphenyl)sulfonyl]-methoxy]imino]acetamido] -3-[[(2-carbamoyl-5-methyl-s-triazolo[1,5-a]pyrimidin-7-yl)thio]methyl]-8-oxo-5-thia -1-azabicyclo[4.2.0]oct-2-en-2-carbonsäure in 7 ml Wasser wird unter Rühren portionenweise mit 91 mg N-Methyl-D-glucamin versetzt. Die resultierende klare Lösung wird lyophilisiert. Man erhält (6R,7R)-7-[(Z)-2-(2-Amino -4-thiazolyl)-2-[[[(3,4-dihydroxyphenyl)sulfonyl]methoxy] imino]acetamido]-3-[[(2-carbamoyl-5-methyl-s-triazolo [1,5-a]pyrimidin-7-yl)thio]methyl]-8-oxo-5-thia -1-azabicyclo[4.2.0]oct-2-en-2-carbonsäure-N-Methyl-D-glucaminsalz als weisse Pulver.

$^1$H NMR (DMSO-$d_6$): δ 2,54 (s,3H); 2,59 (s,3H); 2,7-4,0 (m,ca. 20H); 4,34 (d,J=14Hz,1H); 4,56 (d,J=14Hz,1H); 4,94 (d,J=5Hz,1H); 5,12 (d,J=14Hz,1H); 5,20 (d,J=14Hz,1Hz); 5,45 (dd,J=8 und 5Hz,1H); 6,75 (s,1H); 6,86 (d,J=8Hz,1H); 7,15 (d,J=1Hz,1H); 7,2-7.4 (m,3H); 7,72 (s,1H); 7,82 (s,1H); 8,17 (s,1H); 9,45 (d,J=8Hz,1H) ppm.

Das als Ausgangsmaterial eingesetzte 4-[[(Aminooxy)methyl]sulfonyl]pyrokatechol kann wie folgt hergestellt werden:

a) Ein Gemisch von 29,1 g 5-[(Brommethyl)sulfinyl]-2,2-dimethyl-1,3-benzodioxol, 19,6 g N-Hydroxyphthalimid und 16,7 g Kaliumcarbonat in 120 ml Dimethylsulfoxid wird 2 Stunden bei 75° C gerührt. Nach dem Abkühlen wird das Reaktionsgemisch mit 400 ml Aethylacetat verdünnt und 4 mal mit je 200 ml 5-%iger Natriumchloridlösung extrahiert. Die organische Phase wird über Natriumsulfat getrocknet und im Vakuum von Lösungsmittel befreit. Der Rückstand wird in 200 ml Methylenchlorid gelöst und unter Kühlen im Eisbad mit 32 g 55-%iger m-Chlorperbenzoesäure versetzt. Das Reaktions gemisch wird 1 Stunde bei 20° C gerührt, dann mit Methylenchlorid verdünnt und nacheinander mit 17-%iger wässriger Natriumcarbonatlösung und Wasser gewaschen. Die organische Phase wird über Natriumsulfat getrocknet, das Lösungsmittel wird im Vakuum verdampft und das verbleibende Oel aus Methylenchlorid/Hexan kristallisiert. Man erhält N-[[(3,4-Isopropylidendioxy)phenyl]sulfonyl] methoxy]phthalimid als weisse Kristalle vom Schmelzpunkt 191-193 °C.

b) Eine Suspension von 2,34 g N-[[(3,4-Isopropylidendioxy)phenyl]sulfonyl] methoxy]phthalimid in 10 ml Aethanol wird mit 0,4 ml Hydrazinhydrat versetzt, und das Gemisch wird 45 Minuten bei 20 °C gerührt. Es entsteht zuerst eine klare Lösung, aus der sich später ein weisser Niederschlag abscheidet. Das Reaktionsgemisch wird genutscht, das Filtrat im Vakuum vollständig eingeengt und der Rückstand in 240 ml 0,6N Salzsäure gelöst. Aus dieser Lösung werden bei Normaldruck innert 3 Stunden 200 ml Lösungsmittel abdestilliert. Die konzentrierte Lösung wird gekühlt, durch Zugabe von 2N wässriger Salzsäure auf pH 3,5 gestellt und dann an MCI-Gel CHP20P mit 1-%iger wässriger Essigsäure als Eluens chromatographiert. Die Substanzfraktionen werden im Vakuum vom Lösungsmittel befreit, und der Rückstand wird mit Diäthyläther verrieben. Man erhält 4-[[(Aminooxy)methyl]sulfonyl]pyrokatechol als blassviolette Kristalle vom Schmelzpunkt 151 °C (Zers.).

Die als Ausgangsverbindung verwendete (6R,7R)-7-(2Amino-4-thiazolglyoxylamido)-3-[[(2-carbamoyl -5-methyl-s-triazolo[1,5k-a]pyrimidin-7-yl)thio]methyl] -8-oxo-5-thia-1-azabicyclo[4.2.0]oct-2-en-2-carbonsäure kann wie folgt hergestellt werden:

c) Eine Suspension von 2,50 g 7-Mercapto-5-methyl-s-triazolo[1,5-a]pyrimidin -2-carbonsäure-methylester (Eur. Pat. Publ. 150,507) in 25 ml 25-proz. wässrigem Ammoniak wird 6 Stunden bei Raumtemperatur gerührt. Das Gemisch wird filtriert und der Reststoff im Vakuum bei 50° getrocknet. Man erhält 7-Mercapto-5-methyl-s-triazolo[1,5-a]pyrimidin-2-carbonsäureamid als 1:1:1 Addukt mit Wasser und Ammoniak.
$^1$H NMR (DMSO-$d_6$): $\delta$ 2,26 (s,3H); 6,72 (s,1H); 7,17 (s,4H,$NH_4^+$); 7,60 (breites s, 1H); 7,84 (breites s, 1H) ppm.

d) Eine Mischung von 5,46 g (7R)-7-Aminocephalosporansäure und 4,85 g 7-Mercapto-5-methyl-s-triazolo[1,5-a]pyrimidin-2-carbonsäureamid-Ammoniumsalz wird unter gutem Rühren mit 50 ml einer 20-proz. Lösung von Bortrifluorid in Acetonitril versetzt. Die Temperatur wird mittels Eisbadkühlung auf unter 40° gehalten. Das Reaktionsgemisch wird 1 Stunde bei 20° gerührt und anschliessend mit 200 ml Wasser verdünnt. Es bildet sich ein weisser Niederschlag der durch Filtration gesammelt wird. Das noch feuchte Material wird in 50 ml 3N HCl gelöst und die Lösung filtriert. Aus dem Filtrat kristallisiert nach kurzer Zeit ein weisses Produkt aus. Durch Filtration, Waschen mit $H_2O$ und Aceton und Trocknen im Vakuum erhält man (6R,7R)-7-Amino-3[[(2-carbamoyl-5-methyl-s-triazolo[1,5-a]pyrimidin-7-yl)thio]methyl]-8-oxo-5-thia -1-azabicyclo[4.2.0]oct-2-en-2-carbonsäure als Hydrochlorid.
$^1$H NMR (DMSO-$d_6$): $\delta$ 2,61 (s,3H); 3,74 (d,J = 17,5 Hz,1H); 2,87 (d,J = 17,5 Hz,1H); 4,46 (d,J = 12,5 Hz,1H); 4,54 (d,J = 12,5 Hz,1H); 5,20 (d,J = 5 Hz,1H); 5,25 (d,J = 5 Hz,1H); 7,43 (s,1H); 7,89 (s,1H); 8,19 (s,1H) ppm.
MS: 422 $(M + H)^+$.
IR (KBr): 1770.

e) Eine Suspension von 1,71 g (6R,7R)-7-Amino-3-[[(2-carbamoyl-5-methyl -s-triazolo[1,5-a]pyrimidin-7-yl)thio]methyl]-8-oxo-5-thia -1-azabicyclo[4.2.0]oct-2-en-2-carbonsäure-hydrochlorid in 20 ml Methylenchlorid wird mit 2,74 ml N,O-Bis-(trimethylsily)acetamid versetzt. Nachdem alles in Lösung gegangen ist, werden 1,32 g 2-Amino-4-thiazolthioglyoxylsäure-S-(2-benzothiazolyl)ester zugesetzt und das Gemisch 1,5 Stunden bei 20° gerührt. Ungelöstes Material wird durch Filtration abgetrennt und das Filtrat wird mit 40 ml Methylenchlorid verdünnt. Beim Zutropfen von 2 ml Aethanol entsteht ein gelber Niederschlag, welcher durch Filtration gesammelt und im Vakuum getrocknet wird. Man erhält (6R,7R)-7-(2-Amino-4-thiazolglyoxylamido)-3-[[(2-carbamoyl-5-methyl-s-triazolol[1,5-a]pyrimidin 7-yl)thio]methyl]-8-oxo-5-thia-1-azabicyclo-[4.2.0]oct-2-en-2-carbonsäure als gelbes Pulver.
$^1$H NMR (DMSO-$d_6$): $\delta$ 2,60 (s,3H); 3,54 (d,J = 17,5 Hz,1H); 3,74 (d,J = 17,5 Hz,1H); 4,42 (d,J = 14 Hz,1H); 4,56 (d,J = 14 Hz,1H); 5,14 (d,J = 5 Hz,1H); 5,72 (d,J = 5 Hz,1H); 7,41 (s,2H); 7,55 (s,1H) ppm.

## Beispiel 8

(6R,7R)-7-(2-Amino-4-thiazolglyoxylamido) -3-[[(3-carbamoyl-5-methylpyrazolo[1,5-a]pyrimidin -7-yl)-thio]methyl]-8-oxo-5-thia-1-azabicyclo[4.2.0]oct -2-en-2-carbonsäure wird nach dem in Beispiel 7 beschriebenen Verfahren mit 4-[[(Aminooxy)methyl]sulfonyl]pyrokatechol umgesetzt. Nach Reinigung des Rohproduktes mit dem in Beispiel 7 beschriebenen chromatographischen Verfahren und Lyophilisieren der Produktfraktionen erhält man (6R,7R)-7-[(Z)-2-(2-Amino-4-thiazolyl)-2-[[[(3,4-dihydroxyphenyl)sulfonyl]-methoxy]imino]acetamido] -3-[[(3-carbamoyl-5-methylpyrazolo[1,5-a]pyrimidin -7-yl)thio]methyl]-8-oxo -5-thia-1-azabicyclo[4.2.0]oct-2-en-2-carbonsäure als weisses Pulver.

$^1H$ NMR (DMSO-$d_6$): δ 2,62 (s, 3H); 3,54 (d, J=18 Hz, 1H); 3,75 (d, J=18 Hz, 1H); 4,34 (d, J=13 Hz, 1H); 4,44 (d, J=13 Hz, 1H); 5,15 (d, J=5 Hz, 1H); 5,16 (d, J=14 Hz, 1H); 5,22 (d, J=14 Hz, 1H); 5,74 (dd, J=8 und 5 Hz, 1H); 6,69 (s, 1H); 6,91 (d, J=8 Hz, 1H); 7,18-7,23 (m, 3H); 7,30 (breites s, 2H); 7,49 (breites s, 1H); 7,55 (breites s, 1H); 8,48 (s, 1H); 9,67 (breites s, 1H); 9,73 (d, J=8 Hz, 1H); 10,09 (breites s, 1H) ppm.

Die als Ausgangsverbindung verwendete (6R,7R)-7-(2-Amino-4-thiazolglyoxylamido)-3-[[(3-carbamoyl -5-methylpyrazolo[1,5-a]pyrimidin-7-yl)thio]methyl] -8-oxo-5-thia-1-azabicyclo[4.2.0]oct-2-en-2-carbonsäure kann wie folgt hergestellt werden:

a) 7-Mercapto-5-methylpyrazolo[1,5-a]pyrimidin-3-carbonsäureäthylester (J. Med. Chem. 1981, 24(5), 610-13) wird in 1N wässriger Natronlauge erhitzt, bis alles in Lösung geht. Das Reaktionsgemisch wird abgekühlt und dessen pH mit wässriger Salzsäure auf 1 gestellt. Der entstandene Niederschlag wird abfiltriert, getrocknet und aus Dimethylformamid umkristallisiert. Man erhält 7-Mercapto-5-methyl-pyrazolo-[1,5-a]pyrimidin-3-carbonsäure als gelbes Pulver vom Schmelzpunkt 161-162° (Zers).

b) Eine Suspension von 105 g 7-Mercapto-5-methylpyrazolo[1,5-a]pyrimidin-3-carbonsäure in 400 ml Methylenchlorid wird mit 6,3 ml 1-Chlor-N,N,2-trimethyl-1-propenamin versetzt und solange im Ultraschallbad behandelt, bis der grösste Teil in Lösung geht. Das Gemisch wird über einen Glasfaserfilter genutscht und in das klare Filtrat bei 20° Ammoniak eingeleitet. Es entsteht ein kristalliner Niederschlag, welcher abgenutscht, getrocknet und anschliessend in 100 ml $H_2O$ während 5 Minuten verrührt wird. Das Ungelöste wird abfiltriert und das Filtrat im Vakuum auf das halbe Volumen eingeengt. Beim Stehenlassen bei 0° erhält man 7-Mercapto-5-methylpyrazolo[1,5-a]pyrimidin-3-carbonsäureamid als Ammoniumsalz in Form von weissen Kristallen.
$^1H$ NMR (DMSO-$d_6$): δ 2,28 (s,3H); 6,65 (s,1H); 7,00 (breites s,1H); 7,17 (breites s,4H,$NH_4^+$); 7,94 (breites s,1H) ppm.

c) Eine Mischung von 2,80 g 7-Mercapto-5-methylpyrazolo[1,5-a]pyrimidin -3-carbonsäureamid-Ammoniumsalz und 3,38 g (7R)-7-Aminocephalosporansäure werden mit 25 ml einer 20-proz. Lösung von Bortrifluorid in Acetonitril versetzt und 40 Minuten bei 20° gerührt. Das Reaktionsgemisch wird mit 30 ml Wasser versetzt und der pH mittels 28-proz. wässriger Natronlauge auf 3,5 gestellt. Der dabei gebildete Niederschlag wird genutscht und das so gewonnene Material an OPTI-UP ($C_{12}$) (ANTEC AG, CH-Bennwil) mit Wasser als Eluens chromatographiert. Man erhält (6R,7R)-7-Amino-3[[(3-carbamoyl -5-methylpyrazolo-[1,5-a]pyrimidin-7-yl)thio]methyl]-8-oxo -5-thia-1-azabicyclo[4.2.0]oct-2-en-2-carbonsäure.
$^1H$ NMR (DMSO-$d_6$): δ 2,60 (s,3H); 3,56 (d,J=17,5 Hz,1H); 3,78 (d,J=17,5 Hz,1H); 4,30 (d,J=12,5 Hz,1H); 4,43 (d,J=12,5 Hz),1H); 4,82 (d,J=5 Hz,1H); 5,04 (d,J=5 Hz,1H); 7,20 (s,1H); 7,50 (breites s,1H); 7,56 (breites s,1H); 8,48 (s,1H) ppm.
IR (KBr) 1795.

d) Eine Suspension von 2,05 g (6R,7R)-7-Amino-3-[[(3-carbamoyl -5-methylpyrazolo[1,5-a]pyrimidin-7-yl)thio]methyl]-8-oxo -5-thia-1-azabicyclo[4.2.0]oct-2-en-2-carbonsäure in 15 ml Acetonitril und 15 ml Wasser wird mit 0,69 ml Triäthylamin versetzt, worauf eine klare Lösung entsteht. Zu dieser Lösung werden 2,28 g 2-Amino-4-thiazolthioglyoxylsäure-s-(2-benzothiazolyl)ester zugegeben. Nach 3-stündigem Rühren bei 20° wird das Reaktionsgemisch genutscht, und das Filtrat zwischen Essigester und Wasser verteilt. Die wässrige Phase wird im Vakuum wenig eingeengt und der pH mit 3N Salzsäure auf 2,3 gestellt, worauf sich ein gelber Niederschlag bildet. Nach Filtration, Waschen mit Wasser und Trocknen erhält man (6R,7R)-7-(2-Amino-4-thiazolglyoxylamido)-3-[[(3-carbamoyl -5-methylpyrazolo[1,5-a]pyrimidin-7-yl)thio]methyl]-8-oxo -5-thia-1-azabicyclo[4.2.0]oct-2-en-2-carbonsäure.
$^1H$ NMR (DMSO-$d_6$): 2,61 (s,3H); 3,62 (d,J=17,5 Hz,1H); 3,82 (d,J=17,5 Hz,1H); 4,36 (d,J=12,5 Hz,1H); 4,47 (d,J=12,5 Hz,1H); 5,22 (d,J=5 Hz,1H); 5,78 (dd,J= 8 und 5 Hz,1H); 7,22 (s,1H); 7,49 (breites s,1H); 7,56 (breites s,2H); 7,84 (s,1H); 8,48 (s,1H); 9,82 (d,J=8 Hz,1H) ppm.
IR (KBr) 1779.

## Beispiel 9

(6R,7R)-7-(2-Amino-4-thiazolglyoxylamido) -3-[[(3-carbamoyl -7-(trifluormethyl)pyrazolo[1,5-a]pyrimidin-5-yl)thio]methyl]-8-oxo-5-thia-1-azabicyclo[4.2.0]oct-2-en-2-carbonsäure wird nach dem in Beispiel 7 beschriebenen Verfahren mit 40-[[(Aminoxy)methyl]sulfonyl]pyrokatechol umgesetzt. Nach Reinigung des Rohproduktes mit dem in Beispiel 7 beschriebenen chromatographischen Verfahren und Lyophilisieren des Produktes erhält man (6R,7R)-7-[(Z)-2-(2-Amino-4-thiazolyl)-2-[[[(3,4-dihydroxyphenyl)sulfonyl]methoxy]-imino]acetamido] -3-[[(3-carbamoyl-7-(trifluormethyl)pyrazolo[1,5-a]pyrimidin -5-yl)thio]methyl]-8-oxo-5-thia-1-azabicyclo[4.2.0]oct -2-en-2-carbonsäure als blassgelbes Pulver.

$^1H$ NMR (DMSO-$d_6$): δ 3,56 (d, J=18 Hz, 1H); 3,73 (d, 18 Hz, 1H); 4,27 (d, J=14 Hz, 1H); 4.60 (d, J=14 Hz, 1H); 5,12 (d, J=5 Hz, 1H); 5,14 (d, J=13 Hz, 1H); 5,23 (d, J=13 Hz, 1H); 5,74 (dd, J=8 und 5 Hz, 1H); 6,67 (s, 1H); 6,90 (d, 8 Hz, 1H); 7,1-7,6 (m, ca. 6H); 7.82 (s,1H); 8,58 (s,1H); 9,68 (s, 1H); 9,71 (d, J=8 Hz, 1H); 10,09 (s, 1H) ppm.

Die als Ausgangsverbindung verwendete (6R,7R)-7-(2-Amino-4-thiazolglyoxylamido)-3-[[(3-carbamoyl -7-(trifluormethyl)pyrazolo[1,5-a]pyrimidin-5-yl)thio]methyl] -8-oxo-5-thia-1-azabicyclo[4.2.0]oct-2-en-2-carbonsäure kann wie folgt hergestellt werden:

a) Ein Gemisch von 11,5 g 5-Aminopyrazol-4-carbonsäure-methylester, 16 ml ω,ω,ω-Trifluoracetessigsäure-äthylester und 150 g Polyphosphorsäure wird während 16 Stunden unter Rühren auf 100° erhitzt. Nach dem Abkühlen auf 20° wird kaltes Wasser zugegeben und das Gemisch mit Essigester extrahiert. Die organische Phase wird mit 1N wässriger Salzsäure und wässriger gesättigter Natriumchloridlösung gewaschen und über $Na_2SO_4$ getrocknet. Das Lösungsmittel wird im Vakuum abgedampft und der Rückstand in Aether aufgenommen. Das feste Produkte wird abgenutscht und aus 2-Propanol umkristallisiert. Man erhält ein Isomeres des Produktes, nämlich 7-Hydroxy-5-(trifluormethyl)pyrazolo[1,5-a]pyrimidin-3-carbonsäure-methylester vom Smp. 216-217°. Das in den Mutterlaugen ange- reicherte Produkt wird aus Essigester kristallisiert. Man erhält 5-Hydroxy-7-(trifluormethyl)pyrazolo[1,5-a]pyrimidin -3-carbonsäure-methylester vom Smp. 149-150°.

b) Ein Gemisch aus 2,70 g 5-Hydroxy-7-(trifluormethyl)pyrazolo[1,5-a]pyrimidin -3-carbonsäure-methylester und 1,26 g 4-Dimethylaminopyridin wird in 50 ml Phosphoroxychlorid während 2,5 Stunden auf 100° erhitzt. Die entstandene Lösung wird im Vakuum eingeengt und dann zwischen Essigester und gesättigter Natriumchloridlösung verteilt. Die organische Phase wird nacheinander mit 1N Salzsäure und gesättigter Natriumchloridlösung gewaschen, über Natriumsulfat getrocknet, im Vakuum vom Lösungsmittel befreit und der Rückstand wird aus Essigester/Petroläther kristallisiert. Man erhält 5-Chlor-7-(trifluormethyl)-pyrazolo[1,5-a]pyrimidin -3-carbonsäure-methylester als weisse Kristalle vom Smp. 126-127°.

c) Eine Mischung von 2,30 g 5-Chlor-7-(trifluoromethyl)pyrazolo[1,5-a]pyrimidin -3-carbonsäure-methylester und 2,0 g Natriumhydrogensulfid-Monohydrat in 60 ml Wasser wird 1 Stunde bei 60° gerührt. Das Reaktionsgemisch wird abgekühlt, mit Salzsäure angesäuert und mit Essigester extra hiert. Die organische Phase wird mit gesättigter Natriumchloridlösung gewaschen, über Natriumsulfat getrocknet und im Vakuum bis zur Kristallisation eingeengt. Es wird Petroläther zugegeben und das feste Produkt genutscht. Man erhält 5-Mercapto-7-(trifluormethyl)pyrazolo[1,5-a]pyrimidin-3-carbonsäure-methylester.

$^1H$ NMR (DMSO-$d_6$); δ 3,82 (s,3H); 7,30 (s,1H); 8.40 (s,1H) ppm.

d) Ein Lösung von 1,80 g 5-Mercapto-7-(trifluormethyl)pyrazolo[1,5-a]pyrimidin -3-carbonsäure-methylester in 800 ml 25-proz. wässrigen Ammoniak wird während 24 Stunden bei 20° stehen gelassen. Die Lösung wird im Vakuum auf ein kleines Volumen konzentriert, mit 1N Salzsäure sauer gestellt und mit Essigester extrahiert. Die Oganische Phase wird mit gesättigter Natriumchloridlösung gewaschen, über Natriumsulfat getrocknet und im Vakuum eingedampft. Der zurückbleibende Kristallbrei wird mit Petroläther versetzt und filtriert. Man erhält 5-Mercapto-7-(trifluormethyl)pyrazolo[1,5-a]pyrimidin -3-carbonsäureamid.

$^1H$ NMR (DMSO-$d_6$): δ 7,28 (s,1H); 7,62 (breites s,1H); 8,08 (breites s,1H); 8,45 (s,1H) ppm.

e) Eine Mischung von 1,60 g 5-Mercapto-7-(trifluormethyl)pyrazolo[1,5-a]pyrimidin -3-carbonsäureamid und 1,66 g (7R)-7-Aminocephalosporansäure in 120 ml Acetonitril wird unter Rühren mit 20 ml einer 20-proz. Lösung von Bortrifluorid in Acetonitril versetzt und weitere 5 Stunden bei 20° gerührt. Es werden 100 ml Wasser zugegeben und das Gemisch im Vakuum auf ein Volumen von ca. 80 ml eingeengt. Der pH wird mit 25-proz. wässrigem Ammoniak auf 2,8 gestellt. Nach 1 Stunde Rühren wird der erhaltene Niederschlag filtriert, nacheinander mit Wasser, Aceton und Aether gewaschen und getrocknet. Man erhält (6R,7R)-7-Amino-3-[[(3-carbamoyl -7-(trifluormethyl)pyrazolo[1,5-a]pyrimidin-5-yl)thio]methyl]-8-oxo-5-thia-1-azabicyclo[4.2.0]oct-2-en-2-carbonsäure.

$^1H$ NMR (DMSO-$d_6$): δ 3,53 (d,J=17,5 Hz,1H); 3,72 (d,J=17,5 Hz,1H); 4,24 (d,J=12,5 Hz,1H); 4,58 (d,J=12,5 Hz,1H); 4,80 (d,J=5 Hz,1H); 4,98 (d,J=5 Hz,1H); 7,35 (breites s,1H); 7,52 (breites s,1H); 7,82 (s,1H); 8,57 (s,1H) ppm.

f) Eine Suspension von 1,425 g (6R,7R)-7-Amino-3-[[(3-carbamoyl -7-(trifluormethyl)pyrazolo[1,5-a]-pyrimidin-5-yl)thio]methyl]-8-oxo-5-thia-1-azabicyclo[4.2.0]oct-2-en-2-carbonsäure wird mit 0,475 ml N,O-Bis-(trimethylsilyl)acetamid versetzt. Nachdem eine klare Lösung entstanden ist, werden 1,20 g 2-Amino-4-thiazolthioglyoxylsäure-s-(2-benzothiazolyl)ester zugegeben und das Gemisch 2,5 Stunden bei 20° gerührt. Das Reaktionsgemisch wird durch einen Glasfaserfilter filtriert, das Filtrat im Vakuum konzentriert und zwischen Aethylacetat und 0,25M Kaliumhydrogencarbonatlösung verteilt. Die wässerige Phase wird im Vakuum konzentriert und dann an Opti-Up ($C_{12}$) chromatographiert, wobei zuerst mit Wasser und dann mit Wasser/Acetonitril-Gemischen, bei steigenden Anteilen von Acetonitril, eluiert wird. Die Produktfraktionen werden vereinigt, im Vakuum konzentriert und mit 3N Salzsäure auf pH 2 gestellt, wobei sich ein gelber

Niederschlag bildet. Man erhält (6R,7R)-7-(2-Amino-4-thiazolglyoxylamido) -3-[[(3-carbamoyl-7-(trifluormethyl)pyrazolo[1,5-a]pyrimidin -5-yl)thio]methyl]-8-oxo-5-thia-1-azabicyclo[4.2.0]oct-2-en-carbonsäure.

$^1$H NMR (DMSO-$d_6$): δ 3,60 (d,J=18 Hz,1H); 3,77 (d,J=18 Hz,1H); 4,30 (d,J=12 Hz,1H); 4,62 (d,J=12 Hz,1H); 5,20 (d,J=5 Hz,1H); 5,76 (dd,J=8 Hz und J=5 Hz,1H); 7,33 (s,1H); 7,40 (s,2H); 7,50 (s,1H); 7,80 (s,1H); 7,83 (s,1H); 8,57 (s,1H); 9,29 (d,J=8 Hz,1H) ppm.
IR (KBr): 1775
MS: 629 $(M+H)^+$.

## Beispiel 10

34 mg (6R,7R)-7-(2-Amino-4-thiazolglyoxylamido) -3-[[(2-carbamoyl-5-methyl-3-triazolo[1,5-a]pyrimidin -7-yl)thio]methyl] -8-oxo-5-thia-1-azabicyclo[4.2.0]oct-2-en-2-carbonsäure werden nach dem in Beispiel 7 beschriebenen Verfahren mit 28 mg 2-[2-(Aminooxy)-2-methylpropionyl] -1-[(3,4-dihydroxyphenyl)sulfonyl]-hydrazin umgesetzt. Nach Reinigung des Rohproduktes mit dem in Beispiel 1 beschriebenen chromatographischen Verfahren und Lyophilisieren des Produktes erhält man (6R,7R)-7-[(Z) -2-(2-Amino-4-thiazolyl)-2-[[1-[3-[(3,4-dihydroxyphenyl) sulfonyl]carbazolyl] -1-methyläthoxy]imino]acetamido]-3-[[(2-carbamoyl) -5-methyl-s-triazolo[1,5-a]pyrimidin-7-yl(thio]methyl] -8-oxo-5-thia-1-azabicyclo[4.2.0]oct -2-en-2-carbonsäure als weisses Pulver.

$^1$H NMR (DMSO-$d_6$): δ 1,25 (s, 3H); 1,27 (s, 3H); 2,60 (s, 3H); 3,66 (d, J=18Hz, 1H); 3,86 (d, J=18 Hz, 1H); 4,42 (d, J=13 Hz, 1H); 4,56 (d, J=13 Hz, 1H); 5,24 (d, J=5 Hz, 1H); 5,86 (dd, J=8 und 5 Hz, 1H); 6,79 (d, J=9 Hz, 1H); 6,82 (s, 1H); 7,09 (dd, J=9 und 1,5 Hz, 1H); 7,19 (d, J=1,5 Hz, 1H); 7,31 (breites s, 1H); 7,41 (breites s, 1H); 7,86 (s, 1H); 8,23 (s, 1H); 9,37 (d, J=4 Hz, 1H); 9,45 (d, J=4 Hz, 1H); 9,6-10,0 (m, ca 3 H) ppm.

Das als Ausgangsmaterial eingesetzte 2-[2-(Aminooxy-2-methylpropionyl] -1-[(3,4-dihydroxyphenyl)-sulfonyl]-hydrazin kann wie folgt hergestellt werden:

a) Zu einem Gemisch von 5,15 g Hydrazinhydrat und 10 ml Wasser gibt man 2,9 g 3,4-Diacetoxy-benzolsulfochlorid und erhitzt das Gemisch 1 Stunde auf 70°C. Die klare Lösung wird gekühlt, und das Lösungsmittel wird im Vakuum abgedampft. Der Rückstand wird in Wasser aufgenommen und an MCl-Gel CHP20P chromatographiert. Man eluiert zunächst mit Wasser und anschliessend mit Wasser/Methanol Gemischen bei steigenden Anteilen von Methanol. Die Produktfraktionen werden im Vakuum vollständig eingeengt, und der Rückstand wird aus Methanol/Diäthyläther kristallisiert. Man erhält 3,4-Dihydroxybenzolsulfonhydrazid als weisse Kristalle vom Schmelzpunkt 172°C (Zers.).

b) Ein Gemisch von 204 mg 3,4-Dihydroxybenzolsulfonhydrazid und 397 mg 2-Methyl-2-(phthalimidooxy)thiopropionsäure-S-(2-benzothiazolyl)ester werden in 5 ml Acetonitril 3 Stunden auf 60°C erhitzt. Nach dem Abkühlen wird ungelöstes Material abgenutscht. Das Filtrat wird im Vakuum vom Lösungsmittel befreit, und der Rückstand wird in 3 ml Aethanol aufgenommen. Die Suspension wird mit 100 mg Hydrazinhydrat versetzt und 1 Stunde bei 20° gerührt. Es bildet sich zunächst eine klare Lösung, aus der ein weisser Niederschlag ausfällt. Ungelöstes Material wird abfiltriert, und das Filtrat wird im Vakuum eingedampft. Der kristalline Rückstand wird 10 Minuten mit 10 ml 0,1N wässriger Salzsäure verrührt und das unlösliche Material wird durch Filtration abgetrennt. Das Filtrat wird durch Zugabe von 2N wässriger Natronlauge auf pH 7 gestellt und an MCl-Gel CHP20P chromatographiert, wobei zuerst mit 2-%iger wässriger Essigsäure und dann mit Wasser/Methanol (9:1, v/v) eluiert wird. Durch Eindampfen der Produktfraktionen im Vakuum erhält man 2-[2-(Aminooxy)-2-methylpropionyl]-1 -[(3,4-dihydroxyphenyl)-sulfonyl]hydrazin als weisse Kristalle vom Schmelzpunkt 220°C (Zers.).

$^1$H NMR (DMSO-$d_6$): δ 1,10 (s, 6H); 5,89 (breites s, 2H); 6,78 (d, J=8 Hz, 1H); 7,09 (dd, J=8 und 2 Hz, 1H); 7,16 (d, J=2 Hz, 1H) ppm.

## Beispiel 11

122 mg 4-[[(Aminooxy)methyl]sulfonyl]pyrokatechol werden nach dem in Beispiel 7 beschriebenen Verfahren mit 236 mg (6R,7R)-7-(2-Amino-4-thiazolglyoxylamido)-3-[[[2 -(methoxycarbonyl)-5-methyl-s-triazolo[1,5-a]pyrimidin -7-yl]thio]methyl]-8-oxo-5-thia-1-azabicyclo[4.2.0]oct -2-en-2-carbonsäure umgesetzt. Das erhaltene Rohprodukt wird in 10 ml Wasser aufgeschlämmt und durch langsame Zugabe von 0,1N

Natronlauge in Lösung gebracht, wobei darauf zu achten ist, dass der pH der Lösung 7 nicht übersteigt. Durch Chromatographie an MCI-Gel CHP 20P unter Verwendung von Wasser sowie Wasser/Methanol-Gemischen mit steigenden Anteilen von Methanol als Elutionsmittel und Lyophilisieren der eingeengten Produktfraktionen erhält man (6R,7R)-7-[(Z)-2-(2-Amino-4-thiazolyl)-2-[[[(3,4 -dihydroxyphenyl)sulfonyl]-methoxy]imino]acetamido]-3-[[(2 -(methoxycarbonyl)-5-methyl-s-triazolo[1,5-a]pyrimidin -7-yl]thio]methyl]-8-oxo-5-thia-1-azabicyclo[4.2.0]oct -2-en-2-carbonsäure-Mononatriumsalz als weisses Pulver.
$^1$H NMR (DMSO-$d_6$): δ 2,61 (s, 3H); 3,11 (d, J=18 Hz, 1H); 3,47 (d, J=18 Hz, 1H); 3,93 (s, 3H); 4,35 (d, J=15 Hz, 1H); 4,56 (d, J=15 Hz, 1H); 4,91 (d, J=5Hz, 1H); 5,11 (d, J=15 Hz, 1H); 5,20 (d, J=15 Hz, 1H); 5,42 (dd, J=7 Hz und 5 Hz, 1H); 6,78 (s, 1H); 6,86 (d, J=8 Hz, 1H); 7,16 (d, J=1 Hz, 1H); 7,22-7,36 (m, ca. 4H); 7,84 (s, 1H); 9,43 (d, J=7 Hz, 1H) ppm.

Das Ausgangsmaterial kann wie folgt hergestellt werden:

a) 1,10 g Methyl 7-mercapto-5-methyl-3-triazolo[1,5-a]pyrimidin-2-carboxylat und 1,34 g (7R)-7-Amino-cephalosporansäure werden in 5 ml Acetonitril suspendiert und mit 10 ml einer 20-proz. Lösung von Bortrifluorid in Acetonitril versetzt und 90 Minuten bei 20° gerührt. Das Reaktionsge misch wird mit 40 ml Wasser versetzt und der pH mittels 28-proz. Natronlauge auf 2,5 gestellt. Nach 1 Stunde Rühren bei 0° wird der Niederschlag abgenutscht und getrocknet. Man erhält (6R,7R)-7-Amino-3-[[2-(methoxycarbonyl)-[5-methyl-s-triazolo[1,5-a]pyrimidin-7-yl]thio]methyl]-8-oxo-5-thia-1-azabicyclo[4.2.0]oct-2-en-carbonsäure als weisses Pulver.
$^1$H NMR (DMSO-$d_6$): δ 2,62 (s, 1H); 3,57 (d, J=18 Hz, 1H); 3,77 (d, J=18 Hz,1H); 3,94 (s, 3H); 4,37 (d, J=12 Hz, 1H); 4,45 (d, J=12 Hz, 1H); 4,86 (d, J=5 Hz, 1H); 5,04 (d, J=5 Hz, 1H); 7,44 (s, 1H) ppm.
IR (KBr): 1784 $cm^{-1}$.

b) 1,50 g (6R,7R)-7-Amino-3-[[2-(methoxycarbonyl)-[5-methyl-s-triazolo[1,5-a]pyrimidin-7-yl]thio]-methyl]-8-oxo-5-thia-1-azabicyclo[4.2.0]oct-2-en-carbonsäure werden in 7,5 ml Acetonitril und 7,5 ml Wasser mit 0,49 ml Triäthylamin in Lösung gebracht. Die Lösung wird mit 1,50 g 2-Amino-4-thiazolthioglyoxylsäure-S-(2-benzothiazolyl)ester versetzt und 1 Stunde bei 20° gerührt. Das Reaktionsgemisch wird zwischen Wasser und Essigester verteilt und die wässrige Phase an Optiup ($C_{12}$) (Antec AG, CH-Bennwil) mit einem Gradienten von 0% bis 10% Acetonitril in Wasser chromatographiert. Die Produktfraktionen werden auf ca. 25 ml eingeengt und der pH auf 2,5 gestellt. Der Niederschlag wird abgenutscht und getrocknet. Man erhält (6R,7R)-7-(2-Amino-4-thiazolglyoxylamido)-3-[[[2 --(methoxycarbonyl)-5-methyl-s-triazolo[1,5-a]pyrimidin -7-yl]thio]methyl]-8-oxo-5-thia-1-azabicyclo[4.2.0]oct -2-en-2-carbonsäure als gelbes Pulver.
$^1$H NMR (DMSO-$d_6$): δ 2,62 (s, 3H), 3,62 (d, J=18 Hz, 1H); 3,80 (d, J=18 Hz, 1H); 3,94 (s, 3H); 4,40 (d, J=12 Hz, 1H); 4,50 (d, J=12 Hz, 1H); 5,20 (d, J=5 Hz, 1H); 5,78 (dd, J=8 Hz und J=5 Hz, 1H); 7,41 (s, 2H); 7,46 (s, 1H); 7,82 (s, 1H); 9,81 (d, J=8 Hz, 1H) ppm.
IR (KBr): 1776 $cm^{-1}$.

## Beispiel 12

In analoger Weise zu den in Beispiel 11 bzw. Beispiel 7 beschriebenen Verfahren werden 307 mg 4-[[-(Aminooxy)methyl]sulfonyl]pyrokatechol mit 531 mg (6R,7R)-7-(2-Amino-4-thiazolglyoxylamido)-3 -[[(7-methylpyrazolo[1,5-a]pyrimidin-5yl)thio]methyl] -8-oxo-5-thia-1-azabicyclo[4.2.0]oct-2-en-2-carbonsäure umgesetzt. Man erhält (6R,7R)-7-[(Z)-2-(2-Amino-4-thiazolyl-2-[[[(3,4 -dihydroxyphenyl)sulfonyl]methoxy]imino]-acetamido]-3-[[(7 -methylpyrazolo[1,5-a]pyrimidin-5-yl)thio]methyl-8-oxo-5 -thia-1-azabicyclo[4.2.0]oct-2-en-2-carbonsäure-Mononatriumsalz als weisses Pulver.
$^1$H NMR (DMSO-$d_6$): δ 2,63 (s, 3H); 3,11 (d, J=18 Hz, 1H); 3,49 (d, J=18 Hz, 1H); 4,21 (d, J=13 Hz, 1H); 4,49 (d, J=13 Hz, 1H); 4,94 (d, J=5 Hz, 1H); 5,20 (d, J=14 Hz, 1H); 5,27 (d, J=14 Hz, 1H); 5,46 (m, 1H); 6,54 (d, J=1 Hz, 1h); 6,76 (s, 1H); 6,79 (d, J=10 Hz, 1H); 6,93 (s, 1H); 7,10 (d, J=1 Hz, 1H); 7,19 (dd, J=10 und 1 Hz, 1H); 8,09 (d, J=1 Hz, 1H); 9,44 (breites s, 1H) ppm.

Das Ausgangsmaterial kann wie folgt hergestellt werden:

a) 7,20 g Methyl 5-mercapto-7-methylpyrazolo[1,5-a]pyrimidin-3-carboxylat werden in 2N Natronlauge auf 100° erhitzt, bis fast alles gelöst ist. Die Lösung wird abgekühlt, filtriert und das Filtrat auf pH 2 gestellt. Das Produkt wird abgenutscht, mit Wasser und Aceton gewaschen und getrocknet. Man erhält 5-Mercapto-7-methylpyrazolo[1,5-a]pyrimidin-3-carbonsäure vom Smp. 225° (Zers.).
$^1$H NMR (DMSO-$d_6$): δ 2,50 (s, 3H); 6,80 (s, 1H); 8,25 (s, 1H) ppm.
MS (70 eV): 209 ($M^+$).

b) 2,00 g 5-Mercapto-7-methylpyrazolo[1,5-a]pyrimidin-3-carbonsäure werden unter Argon 1 Stunde auf 220° erhitzt. Das Reaktionsgemisch wird nach dem Abkühlen in 10-proz. Kaliumhydrogencarbonatlösung aufgenommen. Der unlösliche Teil wird abgenutscht und in Wasser aufgenommen. Die Lösung wird auf pH 2 angesäuert und der Niederschlag genutscht. Nach Trocknen erhält man 7-Methylpyrazolo[1,5-a]-pyrimidin-5-thiol.
$^1$H NMR (DMSO-$d_6$): δ 2,50 (s, 3H); 6,04 (d, J=2 Hz, 1H); 6,71 (s, 1H); 7,93 (d, J=2 Hz, 1H); 13,8 (s, breit, 1H) ppm.
MS (70 eV): 165 ($M^+$).

c) 0,70 g 7-Methylpyrazolo[1,5-a]pyrimidin-5-thiol und 1,15 g (7R)-7-Amino-cephalosporansäure werden in 10 ml einer 20-proz. Lösung von Bortrifluorid in Acetonitril gelöst und 1 Stunde bei Raumtemperatur gerührt. Das Reaktionsgemisch wird eingeengt und in 10 ml Wasser aufgenommen. Der Feststoff wird abgenutscht. Man erhält (6R,7R)-7-Amino-3-[[(7-methylpyrazolo[1,5-a]pyrimidin -5-yl]thio]methyl]-8-oxo-5-thia-1-azabicyclo[4.2.0]oct-2 -en-2-carbonsäure als rötliches Pulver.
$^1$H NMR (DMSO-$d_6$): δ 2,64 (s, 3H); 3,48 (d, J=18 Hz, 1H); 3,74 (d, J=18 Hz, 1H); 3,97 (d, J=12 Hz, 1H); 4,72 (d, J=12 Hz, 1H); 4,78 (d, J=5 Hz, 1H); 4,98 (d, J=5 Hz, 1H); 6,56 (d, J=2 Hz, 1H), 6,88 (s, 1H); 8,12 (d, J=2 Hz, 1H) ppm.
IR (KBr): 1799 $cm^{-1}$.

d) 1,00 g (6R,7R)-7-Amino-3-[[(7-methylpyrazolo[1,5-a]pyrimidin -5-yl]thio]methyl]-8-oxo-5-thia-1-azabicyclo[4.2.0]oct-2 -en-2-carbonsäure werden in 5 ml Acetonitril und 5 ml wasser mit 0,37 ml Triäthylamin in Lösung gebracht. Die Lösung wird mit 1,0 g 2-Amino-4-thiazolthioglyoxylsäure-S-(2-benzothiazolyl)-ester versetzt und 1 Stunde bei 20° gerührt. Das Reaktionsgemisch wird zwischen Wasser und Essigester verteilt und die wässrige Phase an Optiup ($C_{12}$) (Antec AG, CH-Bennwil) mit einem Gradienten von 0% bis 10% Acetonitril in Wasser chromatographiert. Die Produktfraktionen werden auf 10 ml eingeengt und mit HCl auf pH 2,2 gestellt. Der Niederschlag wird abgenutscht. Man erhält (6R,7R)-7-(2-Amino-4-thiazolglyoxylamido)-3 -[[(7-methylpyrazolo[1,5-a]pyrimidin-5-yl)thio]methyl] -8-oxo-5-thia-1azabicyclo[4.2.0]oct-2-en-2-carbonsäure als gelbes Pulver.
$^1$H NMR (DMSO-$d_6$): δ 2,64 (s, 3H); 3,54 (d, J=18 Hz, 1H); 3,28 (d, J=18 Hz, 1H), 4,00 (d, J=12 Hz, 1H); 4,78 (d, J=12 Hz, 1H); 5,14 (d, J=5 Hz, 1H); 5,72 (dd, J=8 Hz u. J=5 Hz, 1H); 6,56 (d, J=2 Hz, 1H); 6,89 (s, 1H); 7,43 (s, 2H); 7,80 (s, 1H); 8,12 (s, 1H); 9,79 (d, J=8 Hz, 1H) ppm.
IR (KBr): 1776 $cm^{-1}$.

## Beispiel 13

In analoger Weise zu dem in Beispiel 11 bzw. Beispiel 7 beschriebenen Verfahren werden 307 mg 4-[[(Aminooxy)methyl]sulfonyl]pyrokatechol mit 575 mg (6R,7R)-7-(2-Amino-4-thiazolglyoxylamido)-3-[[(3-carboxy -7-methylpyrazolo[1,5-a]pyrimidin-5-yl)thio]methyl]-8-oxo -5-thia-1-azabicyclo[4.2.0]oct-2-en-2-carbonsäure umgesetzt. Man erhält (6R,7R)-7-[(Z)-2-(2-Amino-4-thiazolyl)-2-[[[(3,4 -dihydroxyphenyl)sulfonyl]-methoxy]imino]acetamido]-3-[[(3 -carboxy-7-methylpyrazolo[1,5-a]pyrimidin-5-yl)thio]methyl] -8-oxo-5-thia-1-azabicyclo[4.2.0]oct-2-en-2-carbonsäure als weisses Pulver.
$^1$H NMR (DMSO-$d_6$): δ 2,67 (s, 3H); 3,68 (d, J=18 Hz, 1H); 3,81 (d, J=18 Hz, 1H); 4,20 (d, J=14 Hz, 1H); 4,55 (d, J=14 Hz, 1H); 5,12 (d, J=5 Hz, 1H); 5,15 (d, J=13 Hz, 1H); 5,23 (d, J=13 Hz, 1H); 5,70 (dd, J=8 und 5 Hz, 1H); 6,67 (s, 1H); 6,90 (d, J=9 Hz, 1H); 7,09-7,23 (m, 3H); 7,28 (breites s, 2H); 8,48 (s, 1H); 9,69 (d, J=8 Hz, 1H); 10,10 (breites s, 1H) ppm.

Das Ausgangsmaterial kann wie folgt hergestellt werden:

a) Eine Suspension von 4,00 g (7R)-7-Aminocephalosporansäure und 3,34 g 5-Mercapto-7-methylpyrazolo[1,5-a]pyrimidin-3-carbonsäure in 20 ml Acetonitril werden mit 25 ml einer 20-proz. Lösung von Bortrifluorid in Acetonitril versetzt und das Gemisch 1,5 Stunden bei 20° gerührt. Es werden 150 ml kaltes Wasser zugegeben und das Gemisch 1,5 Stunden bei 0° gerührt. Der Niederschlag wird abgenutscht und getrocknet. Man erhält (6R,7R)-7-Amino-3-[[(3-carboxy-7-methylpyrazolo[1,5-a]pyrimidin-5-yl)thio]-methyl]-8-oxo-5-thia-1-azabicyclo[4.2.0]oct-2-en-2-carbonsäure als beinahe farbloses Pulver
$^1$H NMR (DMSO-$d_6$): δ 2,67 (s, 3H), 3,76 (m, 2H); 4,18 (d, J=12 Hz, 1H); 4,52 (d, J=12 Hz, 1H); 4,80 (d, J=5 Hz, 1H); 5,00 (d, J=5 Hz, 1H); 7,14 (s, 1H); 8,47 (s, 1H) ppm.
MS (70 eV) 422 $(M+H)^+$.

b) 3,00 g (6R,7R)-7-Amino-3-[[(3-carboxy -7-methylpyrazolo[1,5-a]pyrimidin-5-yl)thio]methyl]-8-oxo -5-thia-1-azabicyclo[4.2.0]oct-2-en-2-carbonsäure werden in 15 ml Acetonitril und 15 ml Wasser mit 1,02 ml Triäthylamin in Lösung gebracht. Die Lösung wird mit 3,15 g 2-Amino-4-thiazolthio glyoxylsäure-S-(2-benzothiazolyl)ester versetzt und 1 Stunde gerührt. Das Reaktionsgemisch wird zwischen Wasser und Essigester verteilt und die wässrige Phase wird an Optiup ($C_{12}$) (Antec AG, CH-Bennwil) mit einem Gradienten von 0% bis 20% Acetonitril in Wasser chromatographiert. Die Produktfraktionen werden auf ca. 50 ml eingeengt und der pH auf 2,5 gestellt. Der Niederschlag wird abgenutscht. Nach Trocknen erhält man (6R,7R)-7-(2-Amino-4-thiazolglyoxylamido)-3-[[(3-carboxy -7-methylpyrazolo[1,5-a]pyrimidin-5-yl)thio]-methyl]-8-oxo -5-thia-1-azabicyclo[4.2.0]oct-2-en-2-carbonsäure als gelbes Pulver.
$^1H$ NMR (DMSO-$d_6$): δ 2,67 (s, 3H); 3,83 (m, 2H); 4,22 (d, J = 12 Hz, 1H); 4,54 (d, J = 12 Hz, 1H); 5,17 (d, J = 5 Hz, 1H); 5,71 (dd, J = 8 Hz und J = 5 Hz, 1H); 7,15 (s, 1H); 7,41 (s, 2H); 7,80 (s, 1H); 8,47 (s, 1H); 9,76 (d, J = 8 Hz, 1H) ppm.
IR (KBr): 1778 $cm^{-1}$.

## Beispiel 14

In analoger Weise zu dem in Beispiel 11 bzw. Beispiel 7 beschriebenen Verfahren werden 613 mg 4-[[(Aminooxy)methyl]sulfonyl]pyrokatechol mit 1,06 g (6R,7R)-7-(2-Amino-4-thiazolglyoxylamido)-3-[[(5 -methylpyrazolo[1,5-a]pyrimidin-7-yl)thio]methyl]-8-oxo -5-thia-1-azabicyclo[4.2.0]oct-2-en-2-carbonsäure umgesetzt. Man erhält (6R,7R)-7-[(Z)-2-(2-Amino-4-thiazolyl)-2-[[[(3,4 -dihydroxyphenyl)sulfonyl]methoxy]-imino]acetamido]-3-[[(5 -methylpyrazolo[1,5-a]pyrimidin-7-yl)thio]methyl-8-oxo-5 -thia-1-azabicyclo[4.2.0]oct-2-en-2-carbonsäure-Mononatriumsalz als weisses Pulver.
$^1H$ NMR (DMSO-$d_6$): δ 2,50 (s, 3H); 3,17 (d, J = 18 Hz, 1H); 3,50 (d, J = 18 Hz, 1H); 4,27 (d, J = 14 Hz, 1H). 4,53 (d, J = 14 Hz, 1H); 4,96 (d, J = 5 Hz, 1H); 5,09 (d, J = 13 Hz, 1H); 5,15 (d, J = 13 Hz, 1H); 5,46 (d, J = 5 Hz, 1H); 6,51 (d, J = 0,2 Hz, 1H); 6,72 (d, J = 8 Hz, 1H); 6,77 (s, 1H); 7,06 (d, J = 0,2 Hz, 1H); 7,17 (dd, J = 8 und 0,2 Hz, 1H); 7,31 (s, 1H); 8,11 (d, J = 0,2 Hz, 1H); 9,48 (breites s, 1H) ppm.

Das Ausgangsmaterial kann wie folgt hergestellt werden:

a) 110 g 7-Mercapto-5-methylpyrazolo[1,5-a]pyrimindin-3-carbonsäure werden in 500 ml 20-proz. Salzsäure 2,5 Stunden auf Rückflusstemperatur erhitzt. Das Reaktionsgemisch wird abgekühlt, das Produkt abgenutscht und aus Wasser/Aethanol bei einem pH von 6 umkristallisiert. Man erhält 5-Methylpyrazolo[1,5-a]pyrimidin-7-thiol als gelbe Kristalle von einem Schmelzpunkt über 250°.
$^1H$ NMR (DMSO-$d_6$): δ 2,33 (s, 3H); 6,31 (D, J = 2 Hz, 1H); 6,67 (s, 1H); 8,06 (d, J = 2 Hz, 1H); 13,3 (s, breit, 1H) ppm.
MS (70 eV): 165 ($M^+$).

b) 1,1 g 5-Methylpyrazolo[1,5-a]pyrimidin-7-thiol und 1,80 g (7R)-7-Aminocephalosporansäure werden in 18 ml einer 20-proz. Lösung von Bortrifluorid in Acetonitril 1 Stunde gerührt. Das Rektionsgemisch wird eingeengt, der Rückstand in 15 ml Wasser aufgenommen und der pH mit 2N Natronlauge auf 2,5 gestellt. Das ausgefallene Produkt wird abgenutscht und getrocknet. Man erhält (6R,7R)-7-Amino-3-[[(5-methylpyrazolo[1,5-a]pyrimidin -7-yl)thio]methyl]-8-oxo-5-thia-1-azabicyclo[4.2.0]oct-2 -en-2-carbonsäure.
$^1H$ NMR (DMSO-$d_6$): δ 2,50 (s, 1H); 3,54 (d, J = 18 Hz, 1H); 3,77 (d, J = 18 Hz, 1H); 4,24 (d, J = 12 Hz, 1H); 4,40 (d, J = 12 Hz, 1H); 4,80 (d, J = 5 Hz, 1H); 5,03 (d, J = 5 Hz, 1H); 6,57 (d, J = 2 Hz, 1H); 6,98 (s, 1H); 8,15 (d, J = 2 Hz, 1H) ppm.
IR (KBr): 1794.

c) 2,30 g (6R,7R)-7-Amino-3-[[(5-methylpyrazolo[1,5-2]pyrimidin -7-yl)thio]methyl]-8-oxo-5-thia-1-azabicyclo[4.2.0]oct-2 -en-2-carbonsäure werden in 12,5 ml Wasser und 12,5 ml Acetonitril mit 0,80 ml Triäthylamin in Lösung gebracht. Die Lösung wird mit 2,30 g 2-Amino-4-thiazolthioglyoxylsäure-S-(2-benzothiazolyl)ester versetzt und 45 Minuten bei 20° gerührt. Das Reaktionsgemisch wird zwischen Wasser und Essigester verteilt und die wässrige Phase wird an Optiup ($C_{12}$) (Antec AG, Bennwil) mit einem Gradienten von 0% bis 10% Acetonitril in Wasser chromatographiert. Die Produktfraktionen werden eingeengt und der pH auf 2,2 gestellt. Der Niederschlag wird abgenutscht und getrocknet. Man erhält (6R,7R)-7-(2-Amino-4-thizolglyoxylamido)-3-[[(5 -methylpyrazolo[1,5-a]pyrimidin-7-yl)thio]methyl]-8-oxo -5-thia-1-azabicyclo[4.2.0]oct-2-en-2-carbonsäure.
$^1H$ NMR (DMSO-$d_6$): δ 2,50 (s, 3H); 3,62 (d, J = 18 Hz, 1H); 3,81 (d, J = 18 Hz, 1H); 4,31 (d, J = 12 Hz, 1H); 4,45 (d, J = 12 Hz, 1H); 5,22 (d, J = 5 Hz, 1H); 5,78 (dd, J = 8 Hz und J = 5 Hz, 1H); 5,67 (d, J = 2 Hz, 1H); 6,99 (s, 1H); 7,42 (s, 2H); 7,82 (s, 1H); 8,16 (s, J = 2 Hz, 1H); 9,84 (d, J = 8 Hz, 1H) ppm.
IR (KBr): 1776 $cm^{-1}$.

## Beispiel 15

In analoger Weise zu dem in Beispiel 11 bzw. Beispiel 7 beschriebenen Verfahren werden 120 mg 4-[[-(Aminooxy)methyl]sulfonyl]pyrokatechol mit 220 mg (6R,7R)-7-(2-Amino-4-thiazolglyoxylamido) -3-[[2-(hydroxymethyl)-5-methyl-s-triazolo[1,5-a]pyrimidin -7-yl]thio]methyl]-8-oxo-5-thia-1-azabicyclo[4.2.0] oct -2-en-2-carbonsäure umgesetzt. Das erhaltene Rohprodukt wird in ca. 4 ml Wasser aufgeschlämmt und durch langsame Zugabe von 1N Natronlauge in Lösung gebracht, wobei darauf zu achten ist, dass der pH der Lösung 7 nicht übersteigt. Durch Chromatographie an MCl-Gel CHP 20P unter Verwendung von 0,005M Natriumphosphatpuffer mit steigenden Anteilen von Acetonitril als Eluens erhält man gereinigtes Produkt, welches durch Konzentrieren und Ansäuern der Reinfraktionen mit 1N HCl isoliert werden kann. Durch Auflösen dieses Materials in Wasser unter Zugabe von 1 Aequivalent Natronlauge und anschliessender Lyophilisation erhält man (6R,7R)-7-[(Z)-2-(2-Amino-4-thiazolyl)-2-[[[(3,4-dihydroxyphenyl)sulfonyl]methoxy]-imino]acetamido]-3-[[[2-(hydroxymethyl)-5-methyl-5-thiazolo[1,5-2] pyrimidin-7-yl]thio]methyl] -8-oxo-5-thia-1-azabicyclo[4.2.0]oct-2-en -2-carbonsäure-Mononatriumsalz als weisses Pulver.

$^1$H NMR (DMSO-$d_6$): δ u.a. 2,56 (s, 3H); 3,09 (d,J = 18 Hz, 1H); 3,48 (d,J = 18 Hz, 1H); 4,29 (d,J = 14 Hz, 1H); 4,55 (d,J = 14 Hz, 1H); 4,61 (s, 2H); 4,92 (d,J = 5 Hz, 1H); 5,14 (d,J = 13 Hz, 1H); 5,22 (d,J = 13 Hz, 1H); 5,44 (m, 1H); 6,78 (s, 1H); 6,66 (d,J = 8 Hz, 1H); 7,1-7,4 (m, 4H); 7,60 (s, 1H); 9,42 (d,J = 8 Hz, 1H) ppm.

Das Ausgangsmaterial kann wie folgt hergestellt werden:

a) 2,24 g Methyl-7-mercapto-5-methyl-s-triazolo[1,5-a]-pyrimi- din-2-carboxylat werden in 350 ml Tetrahydrofuran suspen- diert und bei 2° innert 15 Minuten mit 40 ml einer 1M-Di-isobutylaluminiumhydrid/Hexan-Lösung versetzt. Das Reak- tionsgemisch wird 10 Minuten bei 2° gerührt und anschlies- send mit Eis versetzt. Das Reaktionsgemisch wird weitgehend eingeengt, der Rückstand in 30 ml Wasser aufgenommen und die Lösung mit konzentrierter Salzsäure auf pH 1,2 angesäuert. Der Feststoff wird durch Filtration gesammelt und am Hoch- vakuum getrocknet. Man erhält 7-Mercapto-5-methyl-3-triazolo[1,5-a]pyrimidin-2-methanol vom Schmelzpunkt 233° (Zers.).

$^1$H NMR (DMSO-$d_6$): δ 2,33 (s, 3H); 4,59 (s, 2H); 6,87 (s, 1H) ppm.

b) 0,400 g 7-Mercapto-5-methyl-s-triazolo[1,5-a]pyrimidin-2-methanol und 0,520 g (7R)-7-Aminocephalosporansäure werden in 5,0 ml einer 20-proz. Lösung von Bortrifluorid in Acetonitril gelöst und die Lösung 30 Minuten bei 20° gerührt. Das Reaktionsgemisch wird mit 10 ml Wasser verdünnt und mit 2N Natronlauge auf pH 3,0 gestellt. Der Niederschlag wird abfiltriert und getrocknet. Man erhält (6R,7R)-7-Amino-3-[[[(2-hydroxymethyl) -5-methyl-s-triazolo[1,5-a]pyrimidin-7-yl]thio]methyl] -8-oxo-5-thia-1-azabicyclo[4.2.0]oct -2-en-2-carbonsäure als beiges Pulver.

$^1$H NMR (DMSO-$d_6$): δ 2,57 (s, 3H); 3,54 (d,J = 18Hz, 1H); 3,75 (d,J = 18 Hz, 1H); 4,31 (d,J = 12Hz, 1H); 4,44 (d,J = 12Hz, 1H); 4,62 (s, 2H); 4,81 (d,J = 5Hz, 1H); 5,02 (d,J = 5Hz, 1H); 7,25 (s, 1H).

c) 0,70 g (6R,7R)-7-Amino-3-[[[(2-hydroxymethyl)-5-methyl-s-triazolo[1,5-a]pyrimidin-7-yl]thio]methyl]-8-oxo-5-thia-1-azabicyclo]4.2.0]oct-2-en-2-carbonsäure werden in 10 ml N,N-Dimethylformamid und 10 ml Methylenchlorid suspendiert und durch Zugabe von 1,62 ml N,O-Bis-(trimethylsilyl)acetamid in Lösung gebracht. Das Gemisch wird auf 0° abgekühlt und mit 0,95 g 2-Amino-4-thiazolthio-glyoxylsäure-S-(2-benzothiazolyl)ester versetzt und 1,5 Stunden bei dieser Temperatur gerührt. Das Reaktionsgemisch wird zwischen Aethylacetat und gesättigter Natriumhydrogencarbonatlösung verteilt. Die wässrige Phase wird an Opti-Up $C_{12}$ mit Wasser bis 5% Acetonitril in Wasser chromatographiert. Einheitliche Fraktionen werden konzentriert und mit 1N Salzsäure auf pH 2,5 gestellt. Das ausgefallene Produkt wird abfiltriert und am Hochvakuum getrocknet. Man erhält (6R,7R)-7-(2-Amino-4-thiazolglyoxylamido) -3-[[2-(hydroxymethyl)-5-methyl-s-triazolo[1,5-a]pyrimidin -7-yl]thio]methyl]-8-oxo-5-thia-1-azabicyclo[4.2.0]oct-2-en-2-carbonsäure als gelbes Pulver.

$^1$H NMR (DMSO-$d_6$): δ 2,58 (s, 3H); 3,60 (δ, J = 18Hz, 1H); 3,82 (d,J = 18 Hz, 1H); 4,37 (δ,J = 12Hz, 1H); 4,50 (d,J = 12Hz, 1H); 4,62 (s, 2H); 5,22 (d,J = 5Hz, 1H); 5,77 (dd,J = 5 und 8Hz, 1H); 7,26 (s, 1H); 7,44 (s, breit 2H); 7,84 (s, 1H); 9,83 (d,J = 8Hz, 1H) ppm.

## Beispiel 16

Nach dem im Beispiel 1 beschriebenen Verfahren werden 161 mg 2-Amino-4-thiazolglyoxylsäure-(Z)-0-[2-[(3,4-dihydroxyphenyl)sulfonamido]äthyl]oxim mit 206 mg (6R,7R)-7-Amino-3-[[2-carbamoyl-5-methyltriazolo[1,5-a]pyrimidin-7-yl)thio]methyl]-8-oxo-5-thia-1-azabicyclo[4.2.0]oct-2-en-2-carbonsäure hydrochlorid umgesetzt. Das im Beispiel 1 beschriebene Aufarbeitungsprozedere liefert eine auf pH 7

eingestellte Wasserphase, welche auf eine mit 1%-iger wässrigen Essigsäure konditionierte MCl-Gel-Säule (CHP20P) aufgetragen wird. Man eluiert zunächst mit 1%-iger wässriger Essigsäure, dann mit Wasser und schliesslich mit Wasser/Acetonitrilgemischen bei steigenden Anteilen von Acetonitril. Das Produkt wird mit einem 4:1 (v/v) Wasser/Acetonitril-Gemisch eluiert. Durch Lyophilisieren der im Vakuum eingeengten Produktfraktionen erhält man (6R,7R)-7-[(Z)-2-(2-Amino-4-thiazolyl)-2-[[2-[3,4-dihydroxyphenyl)sulfonamido]-äthoxy]imino]acetamido]-3-[[(2-carbamoyl-5-methyl-s-triazolo[1,5,-a]pyrimidin-7-yl)thio]methyl]-8-oxo-5-thia-1-azabicyclo[4.2.0]oct-2-en-2-carbonsäure als weisses Pulver.

$^1$H NMR (DMSO-$d_6$): δ 2,61 (s ,3H); 2,98 (dt, J=7 und 7Hz, 1H); 3,53 (d, J=17Hz, 1H); 3,78 (d, J=17Hz, 1H); 3,98 (t, J=7Hz, 1H); 4,37 (d, J=13Hz, 1H); 4,50 (d, J=13Hz, 1H); 5,18 (d, J=5Hz, 1H); 5,81 (dd, J=8 und 5 Hz, 1H; 6,74 (s, 1H); 6,86 (d, J=8Hz, 1H); 7,09 (dd, J=8 und 2Hz, 1H); 7,15 (d, J=2Hz, 1H); 7,28 (breites s, 2H); 7,34 (t, J=7Hz, 1H); 7,39 (s, 1H); 7,88 (s,1H); 8,17 (s, 1H); 9,56 (d, J=8Hz, 1H); 9,67 (breites s, 1H); 9,91 (breites s, 1H) ppm

Das als Ausgangsmaterial eingesetzte 2-Amino-4-thiazolylglyoxylsäure-(Z)-0-[2-3,4-dihydroxyphenyl)-sulfonamido]-äthyl]oxim kann wie folgt hergestellt werden:

a) Eine Lösung von 10 g 2-Amino-4-thiazolglyoxylsäure-äthylester-(Z)-0-(2-bromäthyl)oxim in 100 ml Methylenchlorid wird nacheinander mit 3,14 ml Triäthylamin und einer Lösung von 8,8 g Tritylchlorid in 50 ml Methylenchlorid versetzt. Die Reaktionslösung wird während 18 Stunden bei 20°C gerührt, dann mit Wasser gewaschen und über Magnesiumsulfat getrocknet. Das Lösungsmittel wird im Vakuum entfernt, wobei rohes 2-(Tritylamino)-4-thiazolglyoxylsäure-äthylester-(Z)-0-(2-bromäthyl)oxim als Oel erhalten wird.

b) Eine Lösung von 17,5 g 2-(Tritylamino)-4-thiazolglyoxylsäure-äthylester-(Z)-0-(2-bromäthyl)oxim und 5 g Natriumazid in 200 ml N,N-Dimethylformamid wird während 15 Stunden bei 50°C gerührt. Die Reaktionslösung wird auf 20°C gekühlt und zwischen Ethylacetat und Wasser verteilt. Die organische Phase wird über Natriumsulfat getrocknet und das Lösungsmittel im Vakuum entfernt. Man erhält rohes 2-(Tritylamino)-4-thiazolglyoxylsäure-äthylester-(Z)-0-(2-azidoäthyl)oxim als Oel.

c) 16,4 g 2-(Tritylamino)-4-thiazolglyoxylsäure-äthylester-(Z)-0-(2-azidoäthyl)oxim werden in 300 ml Methanol in Gegenwart von 2,5 g Palladiumkohle (5%-ig) während 3,5 Stunden bei Normaldruck hydriert. Der Katalysator wid abfiltriert und das Lösungsmittel im Vakuum entfernt. Der Rückstand wird aus Tetrachlorkohlenstoff/Petroläther (tiefsiedend) kristallisiert. Man erhält 2-(Tritylamino)-4-thiazolglyoxylsäure-äthylester-(Z)-0-(2-aminoäthyl)oxim als weisse Kristalle vom Schmelzpunkt 126-144°C.

d) Zu einer Lösung von 6,0 g 2-(Tritylamino)-4-thiazolglyoxylsäure-äthylester-(Z)-0-(2-aminoäthyl)-oxim und 0,49 g 4-Dimethylamino-pyridin in 50 ml N,N-Dimethylformamid gibt man 1,17 g 3,4-Diacetoxy-benzolsulfochlorid. Die Reaktionslösung wird während 3 Stunden bei 20°C gerührt, dann werden 10 ml 2N Natronlauge zugegeben, und die Lösung wird noch eine Stunde bei 20°C gerührt. Nun werden noch weitere 20 ml Wasser zugegeben, und dann wird der pH des Reaktionsgemisches durch Zugabe von 3N Salzsäure auf 3 abgesenkt. Der Niederschlag wird genutscht, mit wenig Wasser gewaschen und dann in 60 ml 80%-iger wässrigen Essigsäure während 40 Minuten auf 50°C erhitzt. Das Gemisch wird auf 20°C gekühlt und im Vakuum vollständig eingedampft. Der Rückstand wird in 30 ml Methanol gelöst und im Eisbad mit insgesamt 180 ml Diäthyläther versetzt. Der Niederschlag wird genutscht, in wenig Wasser aufgeschlämmt und durch tropfenweise Zugabe von 2N Natronlauge in Lösung gebracht. Die Lösung von pH 7,5 wird auf eine mit 1%-iger wässriger Essigsäure konditionierte MCl-Gel-Säule (CHP20P) aufgetragen und nach dem im Beispiel 16 beschriebenen Prozedere chromatographiert. Das Produkt wird mit einem 9:1-(v/v) Wasser/Acetonitril-Gemisch eluiert. Die Reinfraktionen werden im Vakuum vollständig eingeengt, und der feste Rückstand wird aus Methanol/Diäthyläther kristallisiert. Man erhält 2-Amino-4-thiazolglyoxylsäure-(Z)-0-[2-[(3,4-dihydroxyphenyl)sulfonamido]äthyl]oxim als weisse Kristalle vom Schmelzpunkt 92-94°C (Zersetzung).

$^1$H NMR (DMSO-$d_6$): δ 2,94 (dd, J=7 und 5 Hz); 4,01 (t, J=7Hz, 1H); 6,83 (s, 1H); 6,87 (d, J=9Hz, 1H; 7,09 (dd, J=9 und 1,5Hz, 1 H); 7,18 (d, J=1,5Hz, 1H); 7,25 (breites s, 2H); 7,44 (t, J=5Hz, 1H); 9,89 (breites s, 1H) ppm.

## Beispiel 17

Ein Gemisch von 146 mg (6R,7R)-7-[(Z)-2-(2-Amino-4-thiazolyl)-2-[(2-jodäthoxy)imino]acetamido]-3-[[[2-(hydroxymethyl)-5-methyl-s-triazolo[1,5-a]pyrimidin-7-yl]thio]methyl]-8-oxo-5-thia-1-azabicyclo[4,2,0]oct-2-en-2-carbonsäure, 75 mg 2,2-Diphenyl-1,3-benzodioxol-5-sulfinsäure-lithiumsalz und 24 mg Natriumhydrogencarbonat in 0,5 ml N,N-Dimethylformamid wird während 24 Stunden bei 20°C gerührt. Das Reaktionsgemisch wird mit 5 ml Wasser versetzt und der pH des Gemisches durch Zugabe von 3N

Salzsäure auf 3 gesenkt. Der Niederschlag wird abgenutscht, mit wenig Wasser gewaschen und dann unter Zugabe von wenig 2N Natronlauge in 4 ml Wasser gelöst. Diese Lösung von pH 7,5 wird auf eine mit 1%-iger wässriger Essigsäure konditionierte MCl-Gel-Säule (CHP20P) aufgetragen und gemäss dem im Beispiel 16 beschriebenen Prozedere chromatographiert. Die mit einem 2:1 (v/v) Wasser/ Acetonitrilgemisch eluierte Fraktion wird im Vakuum konzentiert und lyophilisiert. Das Lyophilisat wird in 90%-iger wässriger Trifluoressigsäure gelöst und die Lösung während 20 Minuten bei 20 °C gerührt. Die Lösungsmittel werden im Vakuum entfernt und der Rückstand zwischen Aethylacetat und 2%-iger Natriumhydrogencarbonatlösung verteilt. Die Wasserphase wird nach dem in Beispiel 16 beschriebenen Prozedere chromatographiert. Man erhält nach Einengen und Lyophilisieren der Reinfraktionen (6R,7R)-7-[(Z)-2-(2-Amino-4-thiazolyl)-2-[[2-[(3,4-dihydroxyphenyl)sulfonyl]äthoxy]imino]acetamido]-3-[[[2-(hydroxymethyl)-5-methyl-s-triazolo[1,5-a]-pyrimidin-7-yl]thio]methyl]-8-oxo-5-thia-1-azabicyclo[4.2.0]oct-2-en-2-carbonsäure als weisses Pulver.
$^1$H NMR (DMSO-$d_6$): δ 2,58 (s, 3H); 3,53 (m, 3H); 3,78 (d, J = 18Hz, 1H); 4,21 (m, 2H); 4,33 (d, J = 13Hz, 1H); 4,50 (d, J = 13Hz, 1H); 4,62 (s, 2H); 5,17 (d, J = Hz, 1H); 5,55 (breites s, 1H), 5,78 (dd, J = 8 und 5Hz, 1H); 6,78 (s, 1H); 6,94 (d, J = 8Hz, 1H); 7,18-7,28 (m, 6H); 9,50 (d, J = 8Hz, 1H); 9,85 (breites s, 1H); 10,20 (breites s, 1H) ppm

Die Als Ausgangsmaterial eingesetzt (6R,7R)-7-[(Z)-2-(2-Amino-4-thiazolyl)-2-[(2-jodäthoxy)-imino]-acetamido]-3-[[[2-(hydroxymethyl)-5-methyl-s-triazolo[1,5-a]pyrimidin-7-yl]thio]methyl]-8-oxo-5-thia-1-azabicyclo-[4.2.0]oct-2-en-2-carbonsäure kann wie folgt hergestellt werden:

a) Eine Lösung von 3,41 g 2-Amino-4-thiazolglyoxylsäure-(Z)-0-(2-jodäthyl)oxim in 50 ml N,N-Dimethylformamid wird auf 0 ° C gekühlt und nacheinander mit 1,36 1-Hydroxybenzotriazol und 2,04 g N,N′-Dicyclohexylcarbodiimid versetzt. Das Reaktionsgemisch wird 2 Stunden bei 0 ° C gerührt, wobei sich ein Niederschlag bildet, und dann mit einer auf 0 ° C vorgekühlten Lösung von 4,08 g (6R,7R)-7-Amino-3-[[-[(2-hydroxymethyl)-5-methyl-s-triazolo[1,5-a]pyrimidin-7-yl]thio]methyl]-8-oxo-5-thia-1-azabicyclo[4.2.0]oct-2-en-2-carbonsäure und 1,1 g Triäthylamin in 40 ml N,N-Dimethylformamid versetzt. Das Gemisch wird während 3 Stunden bei 0 ° C gerührt. Der Niederschlag wird abgenutscht und das Filtrat auf 40 ml Wasser gegossen. Durch Zugabe von 3N HCl wird der pH der Lösung auf 3,0 gesenkt. Nach 10-minütigem Rühren im Eisbad wird der Niederschlag genutscht und mit Wasser gewaschen. Das Nutschgut wird in wenig Wasser aufgeschlämmt und durch langsame Zugabe von ca. 2 ml 2N Natronlauge gelöst. Diese Lösung von pH 7,5 wird auf eine mit 1%-er wässriger Essigsäure konditionierte MCl-Gel-Säule (CHP20P) aufgetragen und nach dem in Beispiel 16 beschriebenen Prozedere chromatographiert. Das Produkt wird mit einem 4:1 (v/v) Wasser/Acetonitril-Gemisch eluiert. Die Produktfraktionen werden im Vakuum eingesetzt und das Produkt durch Ansäuren der Lösung mit 3N HCl auf pH 2,8 ausgefällt. Man isoliert (6R,7R)-7-[(Z)-2-(2-Amino-4-thiazolyl)-2-[(2-jodäthoxy)imino]-acetamido]-3-[[[2-(hydroxymethyl)-5-methyl-s-triazolo[1,5-a]-pyrimidin-7-yl]thio]methyl]-8-oxo-5-thia-1-azabicyclo[4.2.0]oct-2-en-2-carbonsäure als beiges Pulver.
$^1$H NMR (DMSO-$d_6$) : δ 2,58 (s, 3H); 3,35 (t, J = 7Hz, 2H); 3,58 (d, J = 14Hz, 1H); 3,76 (d, J = 14Hz, 1H); 4,27 (t, J = 7Hz, 2H); 4,35 (d, J = 11Hz, 1H); 4,44 (d, J = 11Hz, 1H); 4,62 (s, 2H); 5,19 (d, J = 4Hz, 1H); 5,54 (breites s, 1H) 5,81 (dd, J = 8 und 4Hz, 1H); 6,79 (s, 1H); 7,27 (breites s, 3H); 9,64 (d, J = 8Hz, 1H) ppm

Das als Ausgangsmaterial eingesetzte 2,2-Diphenyl-1,3-benzodioxol-5-sulfinsäure-lithiumsalz kann wie folgt hergestellt werden:

a) Zu einer Lösung von 27,4 g 2,2-Diphenyl-1,3-bezodioxol in 80 ml Chloroform werden 18,75 g N-Bromsuccinimid gegeben, und das Gemisch wird 20 Stunden am Rückfluss gekocht. Das Reaktionsgemisch wird gekühlt und das ausgefallene Succinimid abfiltriert. Das Filtrat wird mit Wasser gewaschen, über Natriumsulfat getrocknet und das Lösungsmittel im Vakuum entfernt. Durch Kristallisation des Rückstandes aus Aethanol erhält man 5-Brom-2,2-diphenyl-1,3-benzodioxol als weisse Kristalle vom Schmelzpunkt 88-89 ° C.

b) Zu einem Gemisch von 24 ml 1,7 M Butyllithium/n-Hexan-Lösung und 40 ml Diäthyläther tropft man innert 5 Minuten bei -50 ° C eine Lösung von 14,13 g 5-Brom-2,2-diphenyl-1,3-benzodioxol in 60 ml Diäthyläther. Die Reaktionslösung wird 40 Minuten gerührt, wobei die Temperatur auf -15 ° C ansteigt. Die Lösung wird erneut auf -50 ° C gekühlt und dann wird während 15 Minuten Schwefeldioxid durch die Lösung geleitet, wobei sich ein weisser Niederschlag bildet. Das Reaktionsgemisch wird auf 20 ° C aufgewärmt und überschüssiges Schwefeldioxid durch einen schwachen Strom von Argon ausgetrieben.

Das Gemisch wird genutsch und das Nutschgut mit Diäthyläther gewaschen und im Vakuum getrocknet. Man erhält 2,2-Diphenyl-1,3-benzodioxol-5-sulfinsäure-lithiumsalz als weisses, amorphes Material vom Schmelzpunkt 120-130 ° C (Zersetzung).
$^1$H NMR (DMSO-$d_6$): δ 6,92 (d, J = 8Hz, 1H); 6,97 (dd, J = 8 und 1Hz, 1H); 7,08 (d, J = 1Hz, 1H); 7,35-7,6 (m, 15H)ppm

Beispiel A

Herstellung von Trockenampullen für die intramuskuläre Verabreichung:

Es wird in üblicher Weise ein Lyophilisat von 1 g des Natriumsalzes der (6R,7R)-7-[(Z)-2-(2-Amino-4-thiazolyl)-2-[[[(3,4-dihydroxyphenyl)sulfonyl]methoxy] imino]acetamido]-3-[[(2-carbamoyl-5-methyl -s-triazolo[1,5-a]pyrimidin-7-yl)thio]methyl] -8-oxo-5-thia-1-azabicyclo[4.2.0]oct-2-en-2-carbonsäure hergestellt und in eine Ampulle abgefüllt. Vor der Verabreichung wird das Lyophilisat mit 2,5 ml einer 2%-igen wässrigen Lidokainhydrochloridlösung versetzt.

Das gleiche Verfahren ist auf (6R,7R)-7-[(Z)-2-(2-Amino-4-thiazolyl)-2-[[[(3,4-dihydroxyphenyl)sulfonyl]-methoxy]imino]acetamido]-3-[[[2-(hydroxymethyl)-5-methyl-5-thiazolo[1,5-a]pyrimidin-7-yl]thio]methyl]-8-oxo-5-thia-1-azabicyclo[4.2.0]oct-2-en-2-carbonsäure-Mononatriumsalz anwendbar.

**Ansprüche**

1. Acylderivate der allgemeinen Formel

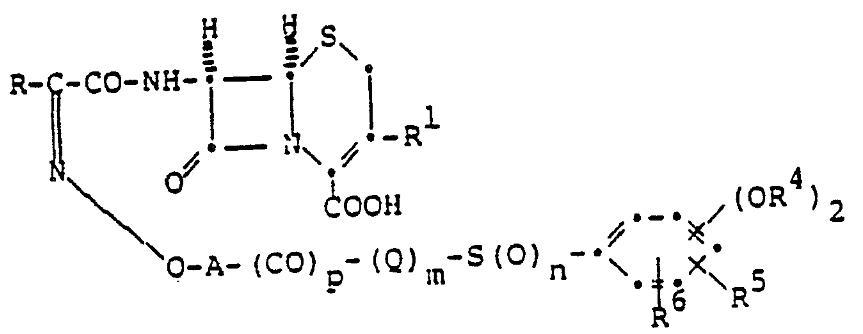

I

worin R eine einkernige, carbocyclische aromatische Gruppe, eine 5-gliedrige, aromatische, heterocylische Gruppe, welche als Heteroringglieder ein Sauerstoff-oder Schwefelatom oder eine Imino- der niedere Alkyliminogruppe und gegebenenfalls noch ein oder zwei Stickstoffatome enthält, oder eine 6-gliedrige, aromatische, heterocyclische Gruppe, welche ein bis drei Stickstoffatome als Heteroringglieder enthält, $R^1$ Wasserstoff oder ein in der Cephalosporinchemie verwendbarer 3-Substituent, A gegebenenfalls durch Carboxy, Carbamoyl, niederes Alkylcarbamoyl oder Di(niederes Alkyl)carbamoyl substituiertes niederes Alkylen oder $C_{3-7}$-Cycloalkylen, Q gegebenenfalls durch Carboxy, Carbamoyl, niederes Alkylcarbamoyl oder Di(niederes Alkyl)carbamoyl substituiertes niederes Alkylen oder $C_{3-7}$-Cycloalkylen oder die Gruppe $-NR^2$- oder $-NR^2NR^3$-, $R^2$ und $R^3$ je Wasserstoff oder niederes Alkyl, p und m die Zahl 0 oder 1, n die Zahl 0, 1 oder 2, $R^4$ Wasserstoff, niederes Alkanoyl oder Tri(niederes Alkyl)-silyl, 2 Reste $R^4$ zusammen Diphenylmethylen, $R^5$ Wasserstoff, niederes Alkyl, Hydroxy, niederes Alkoxy, Halogen, Nitro oder die Gruppe $-OCOR^7$, $-OCOOR^{71}$, $-N(R^7)_2$, $-NHCOR^7$, $-NCOOR^{71}$, $-COR^7$, $-SR^7$, $-SOR^7$, $-SO_2R^7$, $-SO_3H$, $-COOR^7$ oder $-CON(R^7)_2$, $R^6$ Wasserstoff, niederes Alkyl oder Halogen, $R^7$ Wasserstoff oder niederes Alkyl und $R^{71}$ niederes Alkyl bedeuten, und die beiden Gruppen $-OR^4$ über benachbarte Kohlenstoffatome des Phenylringes gebunden sind,

sowie leicht hydrolysierbare Ester und pharmazeutisch annehmbare Salze dieser Verbindungen und Hydrate von Verbindungen der Formel I bzw. von deren Estern und Salzen.

2. Verbindungen nach Anspruch 1, dadurch gekennzeichnet, dass $R^4$ Wasserstoff, niederes Alkanoyl oder Tri(niederes Alkyl)-silyl) darstellt.

3. Verbindungen nach Anspruch 2, dadurch gekennzeichnet, dass A niederes Alkyliden und n die Zahl 2 und entweder m und p die Zahl 0 oder m und p die Zahl 1, Q die Gruppe $-NR^2NR^3$- und $R^2$ und $R^3$ je Wasserstoff bedeuten.

4. Verbindungen nach Anspruch 2 oder 3, dadurch gekennzeichnet, dass $R^4$, $R^5$ und $R^6$ je Wasserstoff bedeuten.

5. Verbindungen nach Anspruch 4, dadurch gekennzeichnet, dass sich die beiden Gruppen $-OR^4$ in den Stellungen 3 und 4 des Phenylringes befinden.

6. Verbindungen nach einem der Ansprüche 3-5, dadurch gekennzeichnet, dass A Methylen oder Isopropyliden bedeutet.

7. Verbindungen nach einem der Ansprüche 3-5, dadurch gekennzeichnet, dass der Substituent in 7-Stellung eine der Gruppen

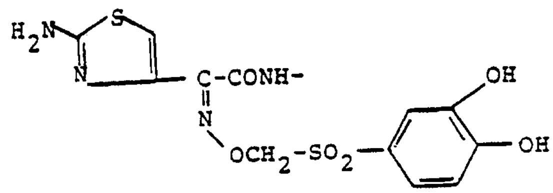

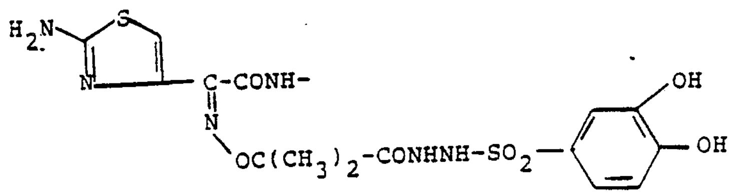

darstellt.

8. Verbindungen nach Anspruch 2, dadurch gekennzeichnet, dass der Substituent in 7-Stellung die Gruppe

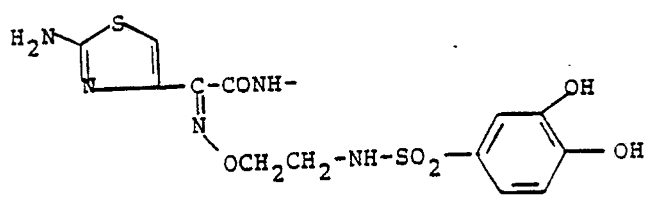

darstellt.

9. Verbindungen nach einem der Ansprüche 2-8, dadurch gekennzeichnet, dass $R^1$ Wasserstoff, niederes Alkyl, niederes Alkoxy, Halogen oder die Gruppe $-CH_2-R'$ oder $-CH_2-S-R''$, $R'$ Azido, niederes Alkanoyloxy, Carbamoyloxy, N-(niederes Alkyl)carbamoyloxy, N,N-Di(niederes Alkyl)carbamoyloxy oder eine über ein Stickstoffatom gebundene, N-haltige heterocyclische Gruppe und $R''$ eine über ein Kohlenstoffatom gebundene heterocyclische Gruppe bedeuten.

10. Verbindungen nach Anspruch 9, dadurch gekennzeichnet, dass $R^1$ die Gruppe $-CH_2-S-R''$ bedeutet.

11. Verbindungen nach Anspruch 10, dadurch gekennzeichnet, dass $R''$ eine bicyclische, 8- bis 10-gliedrige, partiell ungesättigte oder aromatische heterocyclische Gruppe bedeutet, welche ein Sauerstoff- oder Schwefelatom und/oder 1 bis 5 Stickstoffatome als Ringglieder enthält.

12. Verbindungen nach Anspruch 11, dadurch gekennzeichnet, dass die heterocyclische Gruppe $R''$ unsubstituiert oder durch niederes Alkyl, niederes Alkoxy, niederes Alkandiyl, Halo- gen, Trifluormethyl, Hydroxy, Oxo, Carboxy, niederes Alkoxy- carbonyl, Carbamoyl, N-(niederes Alkyl)carbamoyl, N,N-Di-(niederes Alkyl)carbamoyl, Amino, niederes Alkylamino, Di(niederes Alkyl)amino, Mercapto, niederes Alkylthio, niederes Hydroxyalkyl, niederes Aminoalkyl, niederes Alkyl- amino-niederes Alkyl, Di(niederes Alkyl)-amino-niederes Alkyl, niederes Carboxyalkyl, Carbamoyl-niederes Alkyl, N-(niederes Alkyl)carbamoyl-niederes Alkyl, N,N-Di(niederes Alkyl)-carbamoyl-niederes Alkyl und/oder Sulfo-niederes Alkyl mono-, di- oder trisubstituiert ist.

13. Verbindungen nach Anspruch 12, dadurch gekennzeichnet, dass die heterocyclische Gruppe $R''$ der allgemeinen Formel

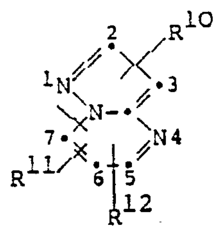 (a), 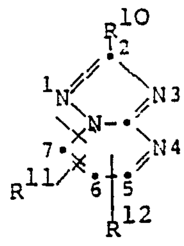 (b), 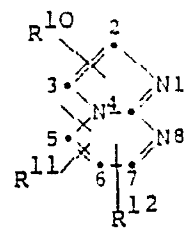 (c) oder 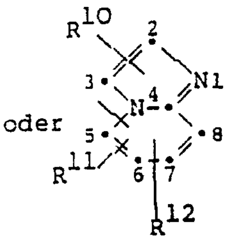 (d

worin $R^{10}$ Wasserstoff, Amino, Carboxy, niederes Alkoxycarbonyl, Carbamoyl, Hydroxymethyl, N-(niederes Alkyl)carbamoyl oder N,N-Di(niederes Alkyl)carbamoyl und $R^{11}$ und $R^{12}$ je Wasserstoff, niederes Alkyl oder Trifluormethyl oder zusammen eine $C_{3-4}$-Alkandiylgruppe bedeuten, entspricht.

14. Verbindungen nach Anspruch 13, dadurch gekennzeichnet, dass $R^{10}$ Wasserstoff, Amino, Carboxy, niederes Alkoxycarbonyl, Carbamoyl, N-(niederes Alkyl)carbamoyl oder N,N-Di(niederes Alkyl)carbamoyl darstellt.

15. Verbindungen nach Anspruch 14 oder 15, dadurch gekennzeichnet, dass die heterocyclischen Gruppen der Formeln (a) und (b) über die 5- oder 7-Stellung, insbesondere über die 7-Stellung, diejenige der Formel (c) über die 5- oder 7-Stellung, insbesondere über die 5-Stellung, und diejenige der Formel (d) über die 5- oder 8-Stellung, insbesondere über die 5-Stellung verknüpft sind.

16. Verbindungen nach einem der Ansprüche 13-15, dadurch gekennzeichnet, dass sich der Substituent $R^{10}$ in der 3-Stellung befindet, wenn die heterocyclische Gruppe der Formel (a), (c) oder (d) entspricht.

17. Verbindungen nach einem der Ansprüche 13-16, dadurch gekennzeichnet, dass $R^{10}$ Carbamoyl bedeutet.

18. Verbindungen nach einem der Ansprüche 13, 15 und 16, dadurch gekennzeichnet dass $R^{10}$ Hydroxymethyl bedeutet.

19. Verbindungen nach einem der Ansprüche 13-18, dadurch gekennzeichnet, dass $R^{11}$ Wasserstoff und $R^{12}$ niederes Alkyl oder Trifluormethyl, insbesondere Methyl, bedeuten.

20. Verbindungen nach Anspruch 10, dadurch gekennzeichnet, dass $R''$ 2-Carbamoyl-5-methyl-s-triazolo[1,5-a]pyrimidin-7-yl bedeutet.

21. Verbindungen nach Anspruch 10, dadurch gekennzeichnet, dass $R''$ 2-(Hydroxymethyl)-5-methyl-s-triazolo[1,5-a]-pyrimidin-7-yl bedeutet.

22. Verbindungen nach einem der Ansprüche 2-21, dadurch gekennzeichnet, dass R 2-Amino-4-thiazolyl bedeutet.

23. (6R,7R)-7-[(Z)-2-(2-Amino-4-thiazolyl)-2-[[[(3,4-dihydroxyphenyl)sulfonyl]methoxy]imino]acetamido]-3-[[(2-carbamoyl-5-methyl-s-triazolo[1,5-a]pyrimidin-7-yl)thio]methyl]-8-oxo-5-thia-1-azabicyclo[4.2.0]oct-2-en-2-carbonsäure sowie ihre pharmazeutisch annehmbaren Salze und Hydrate dieser Säure und Salze.

24. (6R,7R)-7-[(Z)-2-(2-Amino-4-thiazolyl)-2-[[[(3,4-dihydroxyphenyl)sulfonyl]methoxy]imino]acetamido]-3-[[(2,5-dihydro-6-hydroxy-2-methyl-5-oxo-as-triazin-3-yl)thio]methyl]-8-oxo-5-thia-1-azabicyclo[4.2.0]oct-2-en-2-carbonsäure,
(6R,7R)-3-(Acetoxymethyl)-7-[(Z)-2-(2-amino-4-thiazolyl)-2-[[[(3,4-dihydroxyphenyl)sulfonyl]methoxy]imino]-acetamido]-8-oxo-5-thia-1-azabicyclo[4.2.0]oct-2-en-2-carbonsäure,
(6R,7R)-7-[(Z)-2-(2-Amino-4-thiazolyl)-2-[[[(3,4-dihydroxyphenyl)sulfonyl]methoxy]imino]acetamido]-3-[[(2-carboxy-5-methyl-s-triazolo[1,5-a]pyrimidin-7-yl)thio]methyl]-8-oxo-5-thia-1-azabicyclo[4.2.0]oct-2-en-2-carbonsäure,
(6R,7R)-7-[(Z)-2-(2-Amino-4-thiazolyl)-2-[[[(3,4-dihydroxyphenyl)sulfonyl]methoxy]imino]acetamido]-3-(azidomethyl)-8-oxo-5-thia-1-azabicyclo[4.2.0]oct-2-en-2-carbonsäure,
1-[(6R,7R)-7-[(Z)-2-(2-Amino-4-thiazolyl)-2-[[[(3,4-dihydroxyphenyl)sulfonyl]methoxy]imino]acetamido]-2-carboxy-8-oxo-5-thia-1-azabicyclo[4.2.0]oct-2-en-3-yl]pyridiniumbetain,
(6R,7R)-7-[(Z)-2-(2-Amino-4-thiazolyl)-2-[[[(3,4-dihydroxyphenyl)sulfonyl]methoxy]imino]acetamido]-3-[[(5-methyl-1,3,4-thiadiazol-2-yl)thio]methyl]-8-oxo-5-thia-1-azabicyclo[4.2.0]oct-2-en-2-carbonsäure,
(6R,7R)-7-[(Z)-2-(2-Amino-4-thiazolyl)-2-[[[(3,4-dihydroxyphenyl)sulfonyl]methoxy]imino]acetamido]-3-[[(3-carbamoyl-5-methylpyrazolo[1,5-a]pyrimidin-7-yl)thio]methyl]-8-oxo-5-thia-1-azabicyclo[4.2.0]oct-2-en-2-carbonsäure,
(6R,7R)-7-[(Z)-2-(2-Amino-4-thiazolyl)-2-[[[(3,4-dihydroxyphenyl)sulfonyl]methoxy]imino]acetamido]-3-[[(3-carbamoyl-7-(trifluormethyl)pyrazolo[1,5-a]pyrimidin-5-yl)thio]methyl]-8-oxo-5-thia-1-azabicyclo[4.2.0]oct-2-en-2-carbonsäure,
(6R,7R)-7-[(Z)-2-(2-Amino-4-thiazolyl)-2-[[1-[3-[(3,4-dihydroxyphenyl) sulfonyl]carbazolyl]-1-methyläthoxy]-imino]acetamido]-3-[[(2-carbamoyl)-5-methyl-s-triazolo[1,5-a]pyrimidin-7-yl)thio]methyl]-8-oxo-5-thia-1-azabicyclo[4.2.0]oct-2-en-2-carbonsäure,
6R,7R)-7-[(Z)-2-(2-Amino-4-thiazolyl)-2-[[[(3,4-dihydroxyphenyl)sulfonyl]methoxy]imino]acetamido]-3-[[(2-(methoxycarbonyl)-5-methyl-s-triazolo[1,5-a]pyrimidin-7-yl]thio]methyl]-8-oxo-5-thia-1-azabicyclo[4.2.0]oct-2-en-2-carbonsäure,
(6R,7R)-7-[(Z)-2-(2-Amino-4-thiazolyl)-2-[[[(3,4-dihydroxyphenyl)sulfonyl]methoxy]imino]acetamido]-3-[[(7-methylpyrazolo[1,5-a]pyrimidin-5-yl)thio]methyl-8-oxo-5-thia-1-azabicyclo[4.2.0]oct-2-en-2-carbonsäure,
(6R,7R)-7-[(Z)-2-(2-Amino-4-thiazolyl)-2-[[[(3,4-dihydroxyphenyl)sulfonyl]methoxy]imino]acetamido]-3-[[(5-methylpyrazolo[1,5-a]pyrimidin-7-yl)thio]methyl]-8-oxo-5-thia-1-azabicyclo[4.2.0]oct-2-en-2-carbonsäure,
(6R,7R)-7-[(Z)-2-(2-Amino-4-thiazolyl)-2-[[[(3,4-dihydroxyphenyl)sulfonyl]methoxy]imino]acetamido]-3-[[(3-carboxy-7-methylpyrazolo[1,5-a]pyrimidin-5-yl)thio]methyl]-8-oxo-5-thia-1-azabicyclo[4.2.0]oct-2-en-2-carbonsäure,
(6R,7R)-7-[(Z)-2-(2-Amino-4-thiazolyl)-2-[[[(3,4-dihydroxyphenyl)sulfonyl]methoxy]imino]acetamido]-3-[[(2,5-dimethylpyrazolo[1,5-a]pyrimidin-7-yl)thio]methyl]-8-oxo-5-thia-1-azabicyclo[4.2.0]oct-2-en-2-carbonsäure,
(6R,7R)-7-[(Z)-2-(2-Amino-4-thiazolyl)-2-[[[(3,4-dihydroxyphenyl)sulfonyl]methoxy]imino]acetamido]-3-[[(2,8-bis(trifluormethyl)-4-chinolinyl]thio]methyl-8-oxo-5-thia-1-azabicyclo[4.2.0]oct-2-en-2-carbonsäure und
3-[[[(6R,7R)-7-[(Z)-2-(2-Amino-4-thiazolyl)-2-[[[(3,4-dihydroxyphenyl)sulfonyl]methoxy]imino]acetamido]-2-carboxy-8-oxo-5-thia-1-azabicyclo[4.2.0]oct-2-en-3-yl]methyl]thio]-1-(carbamoylmethyl)pyridiniumbetain
sowie ihre pharmazeutisch annehmbaren Salze und Hydrate dieser Säuren und Salze.

25. (6R,7R)-7-[(Z)-2-(2-Amino-4-thiazolyl)-2-[[[(3,4-dihydroxyphenyl)sulfonyl]methoxy]imino]acetamido]-3-[[[2-(hydroxymethyl)-5-methyl-s-triazolo[1,5-a]pyrimidin-7-yl]-thio]methyl]-8-oxo-5-thia-1-azabicyclo[4.2.0]-oct-2-en-2-carbonsäure
sowie ihre pharmazeutisch annehmbaren Salze und Hydrate dieser Säure und Salze.

26. (6R,7R)-7-[(Z)-2-(2-Amino-4-thiazolyl)-2-[[2-[3,4-di-hydroxyphenyl)sulfonamido]äthoxy]imino]-acetamido]-3-[[(2-carbamoyl-5-methyl-s-triazolo[1,5-a]pyrimidin-7-yl)thio]methyl]-8-oxo-5-thia-1-azabicyclo-[4.2.0]oct-2-en-2-carbonsäure und
(6R,7R)-7-[(Z)-2-(2-Amino-4-thiazolyl)-2-[[(3,4-dihydroxyphenyl)sulfonyl]äthoxy]imino]acetamido]-3-[[[2-(hydroxymethyl)-5-methyl-s-triazolo[1,5-a]pyrimidin-7-yl]thio]methyl]-8-oxo-5-thia-1-azabicyclo[4.2.0]oct-2-en-2-carbonsäure
sowie ihre pharmazeutisch annehmbaren Salze und Hydrate dieser Säuren und Salze.

27. Verbindungen der allgemeinen Formel

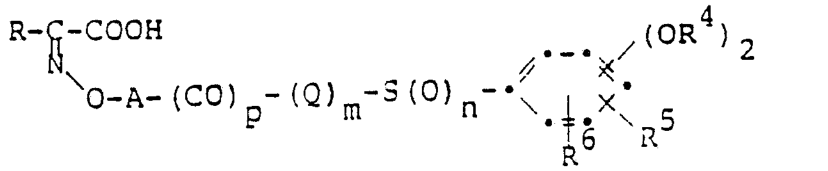

III

worin R, $R^4$, $R^5$, $R^6$, A, Q, m, n und p obige Bedeutung besitzen.

28. Verbindungen der allgemeinen Formel

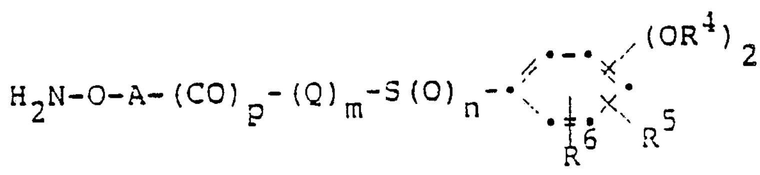

V

worin $R^4$, $R^5$, $R^6$, A, Q, m, n und p obige Bedeutung besitzen.

29. Verbindungen der allgemeinen Formel

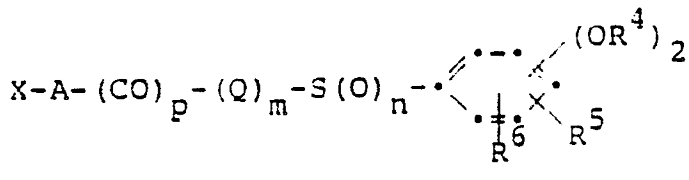

VII

worin X eine Abgangsgruppe bedeutet, und $R^4$, $R^5$, $R^6$, A, Q, m, n und p obige Bedeutung besitzen.

30. Verbindungen der allgemeinen Formel

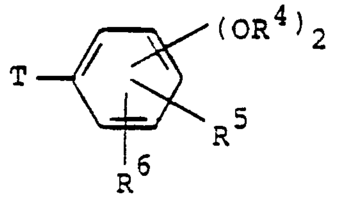

IX + XIII + XVI

worin $R^4$-$R^6$ obige Bedeutung besitzen, T eine der Gruppen H-Q'-S(O)$_n$-, $HO_2S$- und HS- darstellt und Q' und n die in Anspruch 34 gegebene Bedeutung haben.

31. Verbindungen der Formel

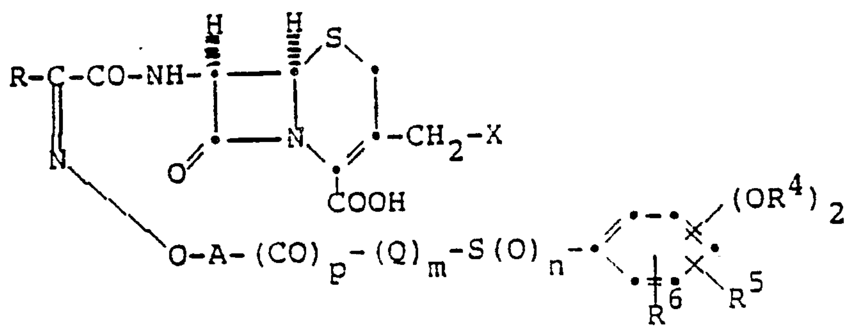

XV

worin R, $R^4$, $R^5$, $R^6$, A, Q, X, m, n und p obige Bedeutung besitzen.

32. Verbindungen nach einem der Ansprüche 1-26 zur Anwendung als therapeutische Wirkstoffe.

33. Verbindungen nach einem der Ansprüche 1-26 zur Anwendung als antibiotisch wirksame Stoffe.

34. Verfahren zur Herstellung der Verbindungen gemäss einem der Ansprüche 1-26, dadurch gekennzeichnet, dass man

a) eine Verbindung der allgemeinen Formel

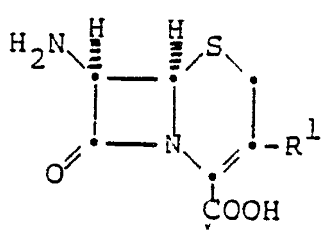

II

worin $R^1$ obige Bedeutung besitzt,
oder einen leicht hydrolysierbaren Ester davon oder ein Säureadditionssalz einer dieser Verbindungen mit einer Verbindung der allgemeinen Formel

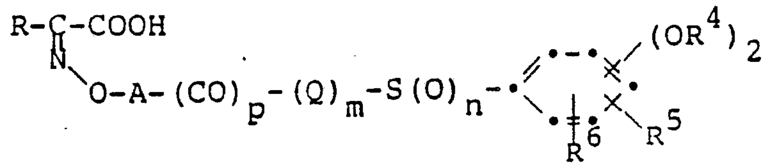

III

worin R, $R^4$, $R^5$, $R^6$, A, Q, m, n und p obige Bedeutung besitzen, acyliert, oder

b) eine Verbindung der allgemeinen Formel

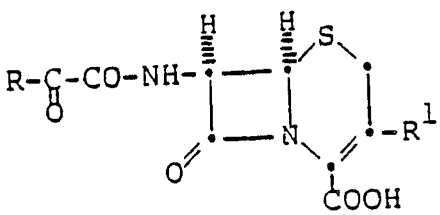

IV

worin R und $R^1$ obige Bedeutung besitzen, oder einen leicht hydrolysierbaren Ester davon gegebenenfalls in Gegenwart eines Kupfersalzes mit einer Verbindung der allgemeinen Formel

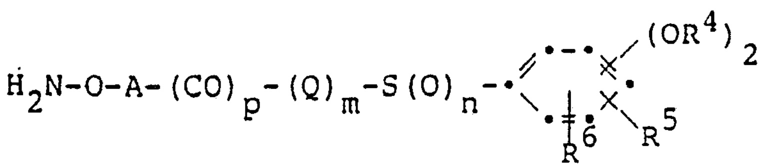

V

worin $R^4$, $R^5$, $R^6$, A, Q, m, n und p obige Bedeutung besitzen, oder einem Säureadditionssalz davon umsetzt, oder

c) eine Verbindung der allgemeinen Formel

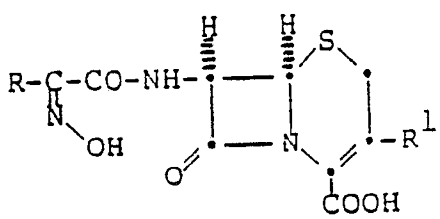

VI

worin R und $R^1$ obige Bedeutung besitzen, oder einen leicht hydrolysierbaren Ester davon mit einer Verbindung der allgemeinen Formel

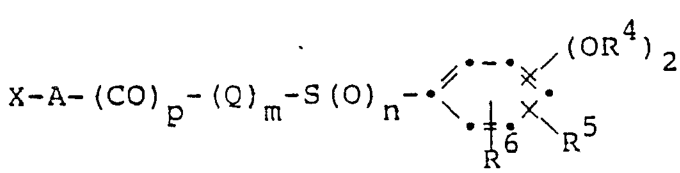

VII

worin X eine Abgangsgruppe bedeutet, und $R^4$, $R^5$, $R^6$, A, Q, m, n und p obige Bedeutung besitzen, alkyliert, oder

d) eine Verbindung der allgemeinen Formel

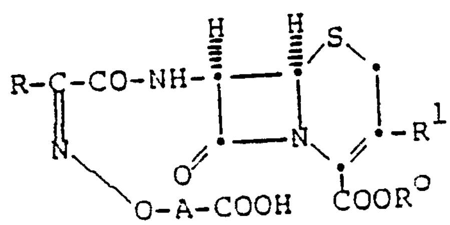

VIII

worin $R^0$ eine durch Hydrolyse entfernbare Gruppe bedeutet, und R, $R^1$ und A obige Bedeutung besitzen, mit einer Verbindung der allgemeinen Formel

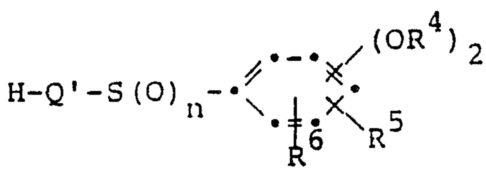

IX

worin Q′ die Gruppe $-NR^2-$ oder $-NR^2NR^3-$ bedeutet, und $R^2$, $R^3$, $R^4$, $R^5$, $R^6$ und n obige Bedeutung besitzen,
umsetzt, oder

e) eine Verbindung der allgemeinen Formel

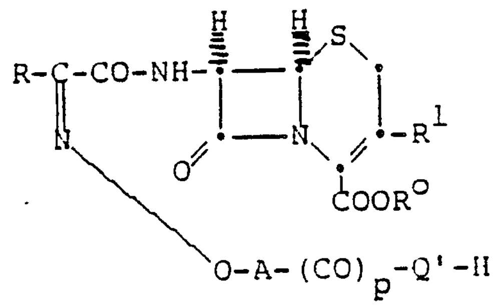

X

worin $R^0$, R, $R^1$, A, Q′ und p obige Bedeutung besitzen
mit einem reaktiven Derivat einer Sulfonsäure der allgemeinen Formel

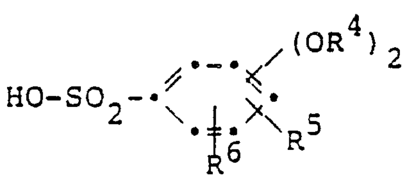

XI

worin $R^4$, $R^5$ und $R^6$ obige Bedeutung besitzen,
umsetzt, oder

f) eine Verbindung der allgemeinen Formel

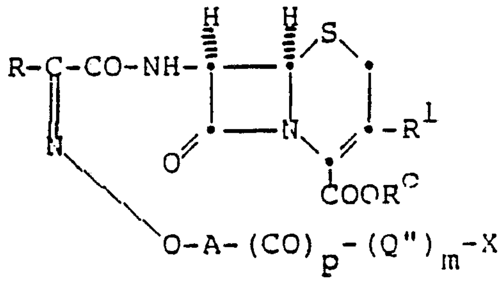

XII

worin Q" gegebenenfalls durch Carboxy, Carbamoyl, niederes Alkylcarbamoyl oder Di(niederes Alkyl)-carbamoyl substituiertes niederes Alkylen oder $C_{3-7}$-Cycloalkylen bedeutet, und $R^0$, R, $R^1$, A, p, m und X obige Bedeutung besitzen,
entweder in Gegenwart einer Base mit einer Sulfinsäure oder einem Mercaptan der allgemeinen Formel

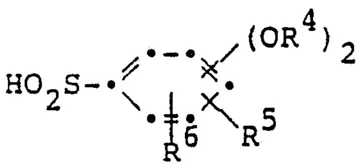

XIII bzw.

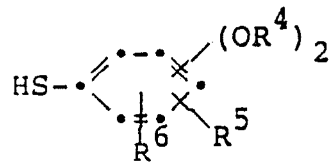

XIV

worin $R^4$, $R^5$ und $R^6$ obige Bedeutung besitzen,
oder mit einem Salz davon umsetzt, oder

g) eine Verbindung der allgemeinen Formel

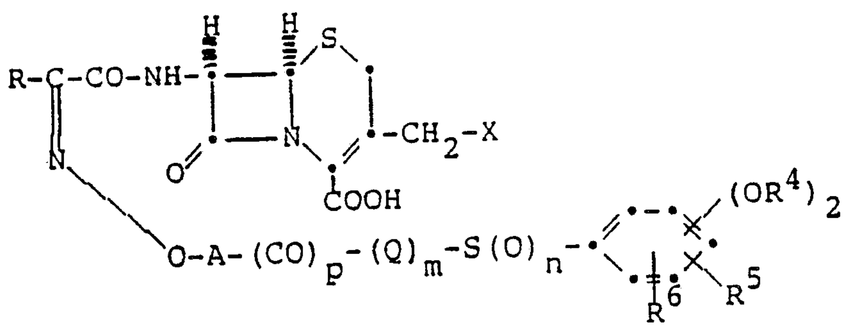

XV

worin R, $R^4$, $R^5$, $R^6$, A, Q, X, m, n und p obige Bedeutung besitzen,
oder einen leicht hydrolysierbaren Ester davon mit einer Verbindung der allgemeinen Formel

HS-R" XVI

worin R" eine über ein Kohlenstoffatom gebundene heterocyclische Gruppe bedeutet,
umsetzt,

h) erwünschtenfalls einen erhaltenen, leicht hydrolysierbaren Ester einer Verbindung der Formel I hydrolysiert und

i) erwünschtenfalls ein erhaltenes Produkt in ein pharmazeutisch annehmbares Salz und/oder ein Hydrat überführt.

35. Arzneimittel enthaltend eine Verbindung nach einem der Ansprüche 1-26 und einen therapeutisch inerten Träger.

36. Antibiotisch wirksame Mittel enthaltend eine Verbindung nach einem der Ansprüche 1-26 und einen therapeutisch inerten Träger.

37. Verwendung von Verbindungen nach einem der Ansprüche 1-26 bei der Bekämpfung oder Verhütung von Krankheiten.

38. Verwendung von Verbindungen nach einem der Ansprüche 1-26 bei der Bekämpfung oder Verhütung von Infektionskrankheiten.

39. Verwendung von Verbindungen nach einem der Ansprüche 1-26 bei der Herstellung von antibiotisch wirksamen Arzneimitteln.

Patentansprüche für den folgenden Vertragsstaaten: GR, ES

1. Verfahren zur Herstellung von Acylderivaten der allgemeinen Formel

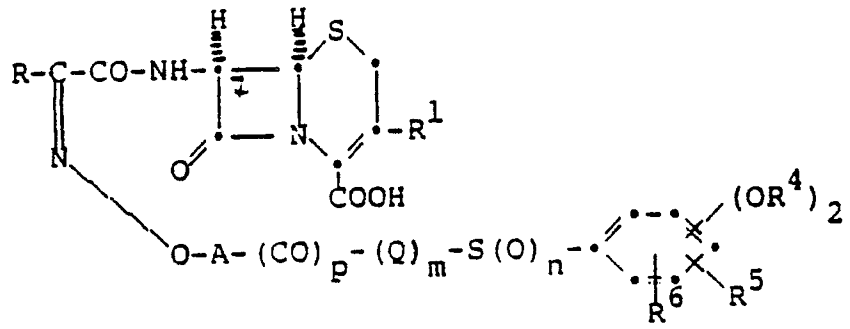

I

worin R eine einkernige, carbocyclische aromatische Gruppe, eine 5-gliedrige, aromatische, heterocylische Gruppe, welche als Heteroringglieder ein Sauerstoff-oder Schwefelatom oder eine Imino- der niedere Alkyliminogruppe und gegebenenfalls noch ein oder zwei Stickstoffatome enthält, oder eine 6-gliedrige, aromatische, heterocyclische Gruppe, welche ein bis drei Stickstoffatome als Heteroringglieder enthält, $R^1$ Wasserstoff oder ein in der Cephalosporinchemie verwendbarer 3-Substituent, A gegebenenfalls durch Carboxy, Carbamoyl, niederes Alkylcarbamoyl oder Di(niederes Alkyl)carbamoyl substituiertes niederes Alkylen oder $C_{3-7}$-Cycloalkylen, Q gegebenenfalls durch Carboxy, Carbamoyl, niederes Alkylcarbamoyl oder Di(niederes Alkyl)carbamoyl substituiertes niederes Alkylen oder $C_{3-7}$-Cycloalkylen oder die Gruppe $-NR^2-$ oder $-NR^2NR^3-$, $R^2$ und $R^3$ je Wasserstoff oder niederes Alkyl, p und m die Zahl 0 oder 1, n die Zahl 0, 1 oder 2, $R^4$ Wasserstoff, niederes Alkanoyl oder Tri(niederes Alkyl)-silyl, 2 Reste $R^4$ zusammen Diphenylmethylen, $R^5$ Wasserstoff, niederes Alkyl, Hydroxy, niederes Alkoxy, Halogen, Nitro oder die Gruppe $-OCOR^7$, $-OCOOR^{71}$, $-N(R^7)_2$, $-NHCOR^7$, $-NCOOR^{71}$, $-COR^7$, $-SR^7$, $-SOR^7$, $-SO_2R^7$, $-SO_3H$, $-COOR^7$ oder $-CON(R^7)_2$, $R^6$ Wasserstoff, niederes Alkyl oder Halogen, $R^7$ Wasserstoff oder niederes Alkyl und $R^{71}$ niederes Alkyl bedeuten, und die beiden Gruppen $-OR^4$ über benachbarte Kohlenstoffatome des Phenylringes gebunden sind,
sowie von leicht hydrolysierbaren Estern und pharmazeutisch annehmbaren Salzen dieser Verbindungen und von Hydraten von Verbindungen der Formel I bzw. von deren Estern und Salzen, dadurch gekennzeichnet, dass man

a) eine Verbindung der allgemeinen Formel

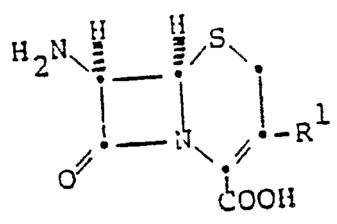

II

worin $R^1$ obige Bedeutung besitzt,
oder einen leicht hydrolysierbaren Ester davon oder ein Säureadditionssalz einer dieser Verbindungen mit einer Verbindung der allgemeinen Formel

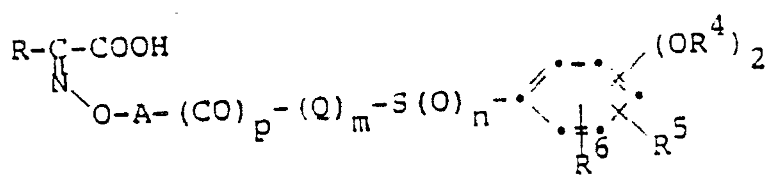

III

worin R, $R^4$, $R^5$, $R^6$, A, Q, m, n und p obige Bedeutung besitzen, acyliert, oder

b) eine Verbindung der allgemeinen Formel

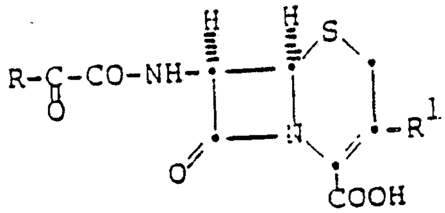

IV

worin R und $R^1$ obige Bedeutung besitzen, oder einen leicht hydrolysierbaren Ester davon gegebenenfalls in Gegenwart eines Kupfersalzes mit einer Verbindung der allgemeinen Formel

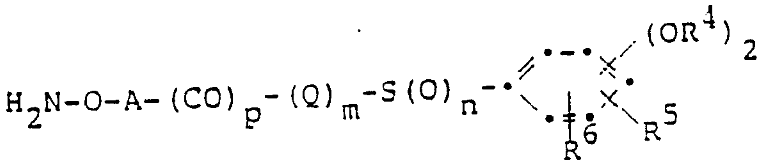

V

worin $R^4$, $R^5$, $R^6$, A, Q, m, n und p obige Bedeutung besitzen, oder einem Säureadditionssalz davon umsetzt, oder

c) eine Verbindung der allgemeinen Formel

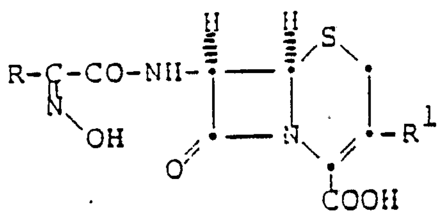

VI

worin R und $R^1$ obige Bedeutung besitzen, oder einen leicht hydrolysierbaren Ester davon mit einer Verbindung der allgemeinen Formel

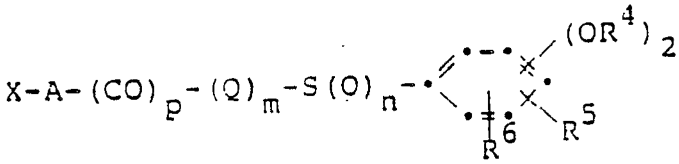

VII

worin X eine Abgangsgruppe bedeutet, und $R^4$, $R^5$, $R^6$, A, Q, m, n und p obige Bedeutung besitzen, alkyliert, oder

d) eine Verbindung der allgemeinen Formel

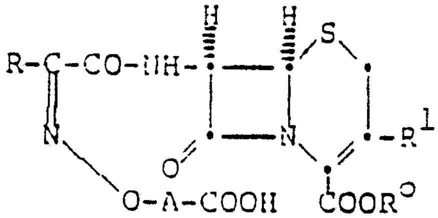

VIII

worin $R^0$ eine durch Hydrolyse entfernbare Gruppe bedeutet, und R, $R^1$ und A obige Bedeutung besitzen, mit einer Verbindung der allgemeinen Formel

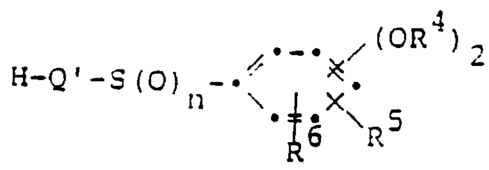

IX

worin Q′ die Gruppe $-NR^2-$ oder $-NR^2NR^3-$ bedeutet, und $R^2$, $R^3$, $R^4$, $R^5$, $R^6$ und n obige Bedeutung besitzen,
umsetzt, oder

e) eine Verbindung der allgemeinen Formel

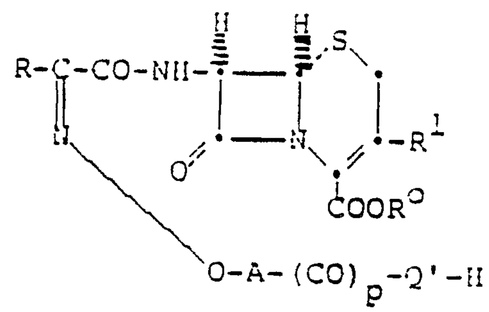

X

worin $R^0$, R, $R^1$, A, Q′ und p obige Bedeutung besitzen mit einem reaktiven Derivat einer Sulfonsäure der allgemeinen Formel

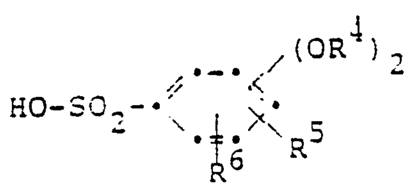

XI

worin $R^4$, $R^5$ und $R^6$ obige Bedeutung besitzen,
umsetzt, oder

f) eine Verbindung der allgemeinen Formel

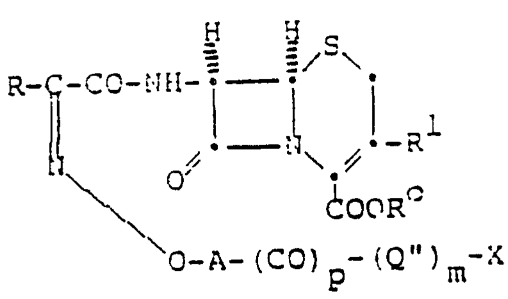

XII

worin Q″ gegebenenfalls durch Carboxy, Carbamoyl, niederes Alkylcarbamoyl oder Di(niederes Alkyl)-carbamoyl substituiertes niederes Alkylen oder $C_{3-7}$-Cycloalkylen bedeutet, und $R^0$, R, $R^1$, A, p, m und X obige Bedeutung besitzen,
entweder in Gegenwart einer Base mit einer Sulfinsäure oder einem Mercaptan der allgemeinen Formel

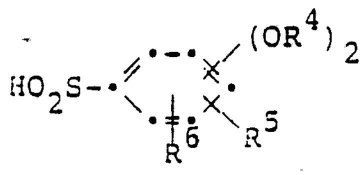 XIII bzw. 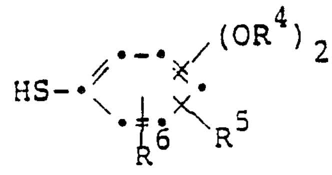 XIV

worin $R^4$, $R^5$ und $R^6$ obige Bedeutung besitzen, oder mit einem Salz davon umsetzt, oder

g) eine Verbindung der allgemeinen Formel

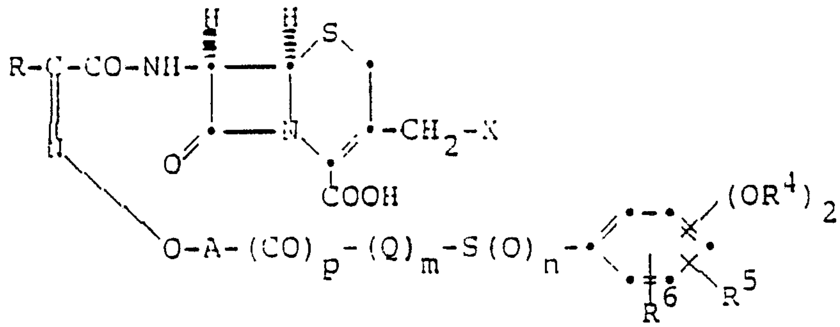 XV

worin R, $R^4$, $R^5$, $R^6$, A, Q, X, m, n und p obige Bedeutung besitzen, oder einen leicht hydrolysierbaren Ester davon mit einer Verbindung der allgemeinen Formel

$HS-R''$ XVI

worin $R''$ eine über ein Kohlenstoffatom gebundene heterocyclische Gruppe bedeutet, umsetzt,

h) erwünschtenfalls einen erhaltenen, leicht hydrolysierbaren Ester einer Verbindung der Formel I hydrolysiert und

i) erwünschtenfalls ein erhaltenes Produkt in ein pharmazeutisch annehmbares Salz und/oder ein Hydrat überführt.

2. Verfahren zur Herstellung von Verbindungen nach Anspruch 1, worin $R^4$ Wasserstoff, niederes Alkanoyl oder Tri(niederes Alkyl)silyl darstellt, dadurch gekennzeichnet, dass man entsprechend substituierte Ausgangsverbindungen einsetzt.

3. Verfahren zur Herstellung von Verbindungen nach Anspruch 2, worin A niederes Alkyliden und n die Zahl 2 und entweder m und p die Zahl 0 oder m und p die Zahl 1, Q die Gruppe $-NR^2NR^3-$ und $R^2$ und $R^3$ je Wasserstoff bedeuten, dadurch gekennzeichnet, dass man entsprechend substituierte Ausgangsverbindungen einsetzt.

4. Verfahren zur Herstellung von Verbindungen nach Anspruch 2 oder 3, worin $R^4$, $R^5$ und $R^6$ je Wasserstoff bedeuten, dadurch gekennzeichnet, dass man entsprechend substituierte Ausgangsverbindungen einsetzt.

5. Verfahren zur Herstellung von Verbindungen nach Anspruch 4, worin sich die beiden Gruppen $-OR^4$ in den Stellungen 3 und 4 des Phenylringes befinden, dadurch gekennzeichnet, dass man entsprechend substituierte Ausgangsverbindungen einsetzt.

6. Verfahren zur Herstellung von Verbindungen nach einem der Ansprüche 3-5, worin A Methylen oder Isopropyliden bedeutet, dadurch gekennzeichnet, dass man entsprechend substituierte Ausgangsverbindungen einsetzt.

7. Verfahren zur Herstellung von Verbindungen nach einem der Ansprüche 3-5, worin der Substituent in 7-Stellung eine der Gruppen

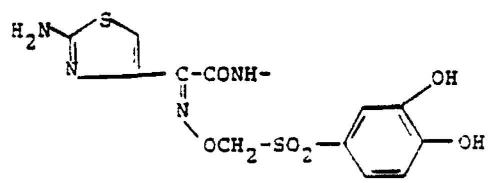

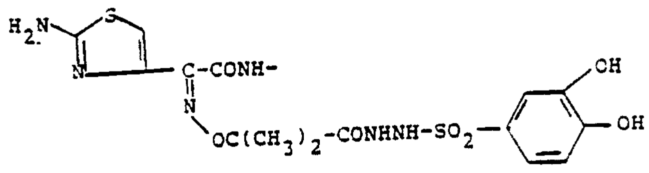

darstellt, dadurch gekennzeichnet, dass man entsprechend substituierte Ausgangsverbindungen einsetzt.

8. Verfahren zur Herstellung von Verbindungen nach Anspruch 2, worin der Substituent in 7-Stellung die Gruppe

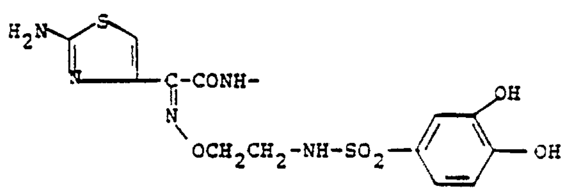

darstellt, dadurch gekennzeichnet, dass man entsprechend substituierte Ausgangsverbindungen einsetzt.

9. Verfahren zur Herstellung von Verbindungen nach einem der Ansprüche 2-8, worin $R^1$ Wasserstoff, niederes Alkyl, niederes Alkoxy, Halogen oder die Gruppe $-CH_2-R'$ oder $-CH_2-S-R''$, $R'$ Azido, niederes Alkanoyloxy, Carbamoyloxy, N-(niederes Alkyl)carbamoyloxy, N,N-Di(niederes Alkyl)carbamoyloxy oder eine über ein Stickstoffatom gebundene, N-haltige heterocyclische Gruppe und $R''$ eine über ein Kohlenstoffatom gebundene heterocyclische Gruppe bedeuten, dadurch gekennzeichnet, dass man entsprechend substituierte Ausgangsverbindungen einsetzt.

10. Verfahren zur Herstellung von Verbindungen nach Anspruch 9, worin $R^1$ die Gruppe $-CH_2-S-R''$ bedeutet, dadurch gekennzeichnet, dass man entsprechend substituierte Ausgangsverbindungen einsetzt.

11. Verfahren zur Herstellung von Verbindungen nach Anspruch 10, worin $R''$ eine bicyclische, 8- bis 10-gliedrige, partiell ungesättigte oder aromatische heterocyclische Gruppe bedeutet, welche ein Sauerstoff- oder Schwefelatom und/oder 1 bis 5 Stickstoffatome als Ringglieder enthält, dadurch gekennzeichnet, dass man entsprechend substituierte Ausgangsverbindungen einsetzt.

12. Verfahren zur Herstellung von Verbindungen nach Anspruch 11, worin die heterocyclische Gruppe $R''$ unsubstituiert oder durch niederes Alkyl, niederes Alkoxy, niederes Alkandiyl, Halo- gen, Trifluormethyl, Hydroxy, Oxo, Carboxy, niederes Alkoxy- carbonyl, Carbamoyl, N-(niederes Alkyl)carbamoyl, N,N-Di-(niederes Alkyl)carbamoyl, Amino, niederes Alkylamino, Di(niederes Alkyl)amino, Mercapto, niederes Alkylthio, niederes Hydroxyalkyl, niederes Aminoalkyl, niederes Alkyl- amino-niederes Alkyl, Di(niederes Alkyl)-amino-niederes Alkyl, niederes Carboxyalkyl Carbamoyl-niederes Alkyl, N-(niederes Alkyl)carbamoyl-niederes Alkyl, N,N-Di(niederes Alkyl)-carbamoyl-niederes Alkyl und/oder Sulfo-niederes Alkyl mono-, di- oder trisubstituiert ist, dadurch gekennzeichnet, dass man entsprechend substituierte Ausgangsverbindungen einsetzt.

13. Verfahren zur Herstellung von Verbindungen nach Anspruch 12, worin die heterocyclische Gruppe $R''$ der allgemeinen Formel

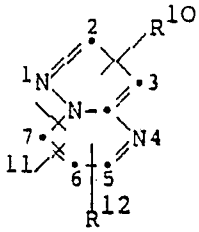
30

(a),

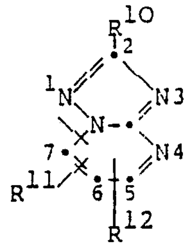
(b),

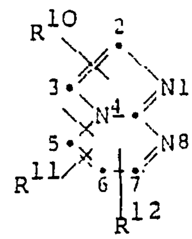
(c) oder

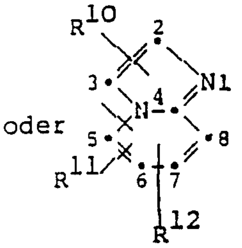
(d

worin $R^{10}$ Wasserstoff, Amino, Carboxy, niederes Alkoxycarbonyl, Carbamoyl, Hydroxymethyl, N-(niederes Alkyl)carbamoyl oder N,N-Di(niederes Alkyl)carbamoyl und $R^{11}$ und $R^{12}$ je Wasserstoff, niederes Alkyl oder Trifluormethyl oder zusammen eine $C_{3-4}$-Alkandiylgruppe bedeuten,
entspricht, dadurch gekennzeichnet, dass man entsprechend substituierte Ausgangsverbindungen einsetzt.

14. Verfahren zur Herstellung von Verbindungen nach Anspruch 13, worin $R^{10}$ Wasserstoff, Amino, Carboxy, niederes Alkoxycarbonyl, Carbamoyl, N-(niederes Alkyl)carbamoyl oder N,N-Di(niederes Alkyl)-carbamoyl darstellt, dadurch gekennzeichnet, dass man entsprechend substitueierte Ausgangsverbindungen einsetzt.

15. Verfahren zur Herstellung von Verbindungen nach Anspruch 14 oder 15, worin die heterocyclischen Gruppen der Formeln (a) und (b) über die 5- oder 7-Stellung, insbesondere über die 7-Stellung, diejenige der Formel (c) über die 5- oder 7-Stellung, insbesondere über die 5-Stellung, und diejenige der Formel (d) über die 5- oder 8-Stellung, insbesondere über die 5-Stellung verknüpft sind, dadurch gekennzeichnet, dass man entsprechend substituierte Ausgangsverbindungen einsetzt.

16. Verfahren zur Herstellung von Verbindungen nach einem der Ansprüche 13-15, worin sich der Substituent $R^{10}$ in der 3-Stellung befindet, wenn die heterocyclische Gruppe der Formel (a), (c) oder (d) entspricht, dadurch gekennzeichnet, dass man entsprechend substituierte Ausgangsverbindungen einsetzt.

17. Verfahren zur Herstellung von Verbindungen nach einem der Ansprüche 13-16, worin $R^{10}$ Carbamoyl bedeutet, dadurch gekennzeichnet, dass man entsprechend substituierte Ausgangsverbindungen einsetzt.

18. Verfahren zur Herstellung von Verbindungen nach einem der Ansprüche 13, 15 und 16, worin $R^{10}$ Hydroxymethyl bedeutet, dadurch gekennzeichnet, dass man entsprechend substituierte Ausgangsverbindungen einsetzt.

19. Verfahren zur Herstellung von Verbindungen nach einem der Ansprüche 13-18, worin $R^{11}$ Wasserstoff und $R^{12}$ niederes Alkyl oder Trifluormethyl, insbesondere Methyl, bedeuten, dadurch gekennzeichnet, dass man entsprechend substituierte Ausgangsverbindungen einsetzt.

20. Verfahren zur Herstellung von Verbindungen nach Anspruch 10, worin $R''$ 2-Carbamoyl-5-methyl-s-triazolo[1,5-a]pyrimi- din-7-yl bedeutet, dadurch gekennzeichnet, dass man entsprechend substituierte Ausgangsverbindungen einsetzt.

21. Verfahren zur Herstellung von Verbindungen nach Anspruch 10, worin $R''$ 2-(Hydroxymethyl)-5-methyl-s-triazolo[1,5-a]- pyrimidin-7-yl bedeutet, dadurch gekennzeichnet, dass man entsprechend substituierte Ausgangsverbindungen einsetzt.

22. Verfahren zur Herstellung von Verbindungen nach einem der Ansprüche 2-21, worin R 2-Amino-4-thiazolyl bedeutet, dadurch gekennzeichnet, dass man entsprechend substituierte Ausgangsverbindungen einsetzt.

23. Verfahren zur Herstellung von Verbindungen nach Anspruch 2, nämlich von (6R,7R)-7-[(Z)-2-(2-Amino-4-thiazolyl)-2-[[[(3,4-dihydroxyphenyl)sulfonyl]methoxy]imino]acetamido]-3-[[(2-carbamoyl-5-methyl-s-triazolo[1,5-a]pyrimidin-7-yl)thio]-methyl]-8-oxo-5-thia-1-azabicyclo[4.2.0]oct-2-en-2-carbon-säure sowie von ihren pharmazeutisch annehmbaren Salzen und von Hydraten dieser Säure und Salze, dadurch gekennzeichnet, dass man entsprechend substituierte Ausgangsverbindungen einsetzt.

24. Verfahren zur Herstellung von Verbindungen nach Anspruch 2, nämlich von (6R,7R)-7-[(Z)-2-(2-Amino-4-thiazolyl)-2-[[[(3,4-dihydroxyphenyl)sulfonyl]methoxy]imino]acetamido]-3-[[(2,5-dihydro-6-hydroxy-2-methyl-5-oxo-as-triazin-3-yl)thio]-methyl]-8-oxo-5-thia-1-azabicyclo [4.2.0]oct-2-en-2-carbonsäure,
(6R,7R)-3-(Acetoxymethyl)-7-[(Z)-2-(2-amino-4-thiazolyl)-2-[[[(3,4-dihydroxyphenyl)sulfonyl]methoxy]imino]-acetamido]-8-oxo-5-thia-1-azabicyclo[4.2.0]oct-2-en-2-carbonsäure,
(6R,7R)-7-[(Z)-2-(2-Amino-4-thiazolyl)-2-[[[(3,4-dihydroxyphenyl)sulfonyl]methoxy]imino]acetamido]-3-[[(2-carboxy-5-methyl-s-triazolo[1,5-a]pyrimidin-7-yl)thio]methyl]-8-oxo-5-thia-1-azabicyclo[4.2.0]oct-2-en-2-carbonsäure,
(6R,7R)-7-[(Z)-2-(2-Amino-4-thiazolyl)-2-[[[(3,4-dihydroxyphenyl)sulfonyl]methoxy]imino]acetamido]-3-(azidomethyl)-8-oxo-5-thia-1-azabicyclo[4.2.0]oct-2-en-2-carbonsäure,
1-[(6R,7R)-7-[(Z)-2-(2-Amino-4-thiazolyl)-2-[[[(3,4-dihydroxyphenyl)sulfonyl]methoxy]imino]acetamido]-2-carboxy-8-oxo-5-thia-1-azabicyclo[4.2.0]oct-2-en-3-yl]pyridiniumbetain,
(6R,7R)-7-[(Z)-2-(2-Amino-4-thiazolyl)-2-[[[(3,4-dihydroxyphenyl)sulfonyl]methoxy]imino]acetamido]-3-[[(5-methyl-1,3,4-thiadiazol-2-yl)thio]methyl]-8-oxo-5-thia-1-azabicyclo[4.2.0]oct-2-en-2-carbonsäure,
(6R,7R)-7-[(Z)-2-(2-Amino-4-thiazolyl)-2-[[[(3,4-dihydroxyphenyl)sulfonyl]methoxy] imino]acetamido]-3-[[(3-carbamoyl-5-methylpyrazolo[1,5-a]pyrimidin-7-yl)thio]methyl]-8-oxo-5-thia-1-azabicyclo[4.2.0]oct-2-en-2-carbonsäure,
(6R,7R)-7-[(Z)-2-(2-Amino-4-thiazolyl)-2-[[[(3,4-dihydroxyphenyl)sulfonyl]methoxy]imino]acetamido]-3-[[(3-carbamoyl-7-(trifluormethyl)pyrazolo[1,5-a]pyrimidin-5-yl)thio]methyl]-8-oxo-5-thia-1-azabicyclo[4.2.0]oct-2-en-2-carbonsäure,
(6R,7R)-7-[(Z)-2-(2-Amino-4-thiazolyl)-2-[[1-[3-[(3,4-dihydroxyphenyl) sulfonyl]carbazolyl]-1-methyläthoxy]-imino]acetamido]-3-[[(2-carbamoyl)-5-methyl-s-triazolo[1,5-a]pyrimidin-7-yl)thio]methyl]-8-oxo-5-thia-1-azabicyclo[4.2.0]oct-2-en-2-carbonsäure,
6R,7R)-7-[(Z)-2-(2-Amino-4-thiazolyl)-2-[[[(3,4-dihydroxyphenyl)sulfonyl]methoxy]imino]acetamido]-3-[[(2-(methoxycarbonyl)-5-methyl-s-triazolo[1,5-a]pyrimidin-7-yl]thio]methyl]-8-oxo-5-thia-1-azabicyclo[4.2.0]oct-2-en-2-carbonsäure,
(6R,7R)-7-[(Z)-2-(2-Amino-4-thiazolyl)-2-[[[(3,4-dihydroxyphenyl)sulfonyl]methoxy]imino]acetamido]-3-[[(7-methylpyrazolo[1,5-a]pyrimidin-5-yl)thio]methyl-8-oxo-5-thia-1-azabicyclo[4.2.0]oct-2-en-2-carbonsäure,
(6R,7R)-7-[(Z)-2-(2-Amino-4-thiazolyl)-2-[[[(3,4-dihydroxyphenyl)sulfonyl]methoxy]imino]acetamido]-3-[[(5-methylpyrazolo[1,5-a]pyrimidin-7-yl)thio]methyl]-8-oxo-5-thia-1-azabicyclo[4.2.0]oct-2-en-2-carbonsäure,
(6R,7R)-7-[(Z)-2-(2-Amino-4-thiazolyl)-2-[[[(3,4-dihydroxyphenyl)sulfonyl]methoxy]imino]acetamido]-3-[[(3-carboxy-7-methylpyrazolo[1,5-a]pyrimidin-5-yl)thio]methyl]-8-oxo-5-thia-1-azabicyclo[4.2.0]oct-2-en-2-carbonsäure,
(6R,7R)-7-[(Z)-2-(2-Amino-4-thiazolyl)-2-[[[(3,4-dihydroxyphenyl)sulfonyl]methoxy]imino]acetamido]-3-[[(2,5-dimethylpyrazolo[1,5-a]pyrimidin-7-yl)thio]methyl]-8-oxo-5-thia-1-azabicyclo[4.2.0]oct-2-en-2-carbonsäure,
(6R,7R)-7-[(Z)-2-(2-Amino-4-thiazolyl)-2-[[[(3,4-dihydroxyphenyl)sulfonyl]methoxy]imino]acetamido]-3-[[(2,8-bis(trifluormethyl)-4-chinolinyl)thio]methyl-8-oxo-5-thia-1-azabicyclo[4.2.0]oct-2-en-2-carbonsäure und
3-[[[(6R,7R)-7-[(Z)-2-(2-Amino-4-thiazolyl)-2-[[[(3,4-dihydroxyphenyl)sulfonyl]methoxy]imino]acetamido]-2-carboxy-8-oxo-5-thia-1-azabicyclo[4.2.0]oct-2-en-3-yl]methyl]thio]-1-(carbamoylmethyl)pyridiniumbetain
sowie von ihren pharmazeutisch annehmbaren Salzen und von Hydraten dieser Säuren und Salze, dadurch gekennzeichnet, dass man entsprechend substituierte Ausgangsverbindungen einsetzt.

25. Verfahren zur Herstellung von Verbindungen nach Anspruch 2, nämlich von (6R,7R)-7-[(Z)-2-(2-Amino-4-thiazolyl)-2-[[[(3,4-dihydroxyphenyl)sulfonyl]methoxy]imino]acetamido]-3-[[[2-(hydroxymethyl)-5-methyl-s-triazolo[1,5-a]pyrimidin-7-yl]thio]methyl]-8-oxo-5-thia-1-azabicyclo[4.2.0]oct-2-en-2-carbonsäure sowie von ihren pharmazeutisch annehmbaren Salzen und von Hydraten dieser Säure und Salze, dadurch gekennzeichnet, dass man entsprechend substituierte Ausgangsverbindungen einsetzt.

26. Verfahren zur Herstellung von Verbindungen nach Anspruch 1, nämlich von (6R,7R)-7-[(Z)-2-(2-Amino-4-thiazolyl)-2-[[2-[3,4-dihydroxyphenyl)sulfonamido]äthoxy]imino]acetamido]-3-[[(2-carbamoyl-5-methyl-s-triazolo[1,5-a]pyrimidin-7-yl)thio]methyl]-8-oxo-5-thia-1-azabicyclo[4.2.0]oct-2-en-2-carbonsäure und
(6R,7R)-7-[(Z)-2-(2-Amino-4-thiazolyl)-2-[[2-[(3,4-dihydroxyphenyl)sulfonyl]äthoxy]imino]acetamido]-3-[[[2-(hydroxymethyl)-5-methyl-s-triazolo[1,5-a]pyrimidin-7-yl]thio]methyl]-8-oxo-5-thia-1-azabicyclo[4.2.0]oct-2-en-2-carbonsäure
sowie von ihren pharmazeutisch annehmbaren Salzen und von Hydraten dieser Säuren und Salzen, dadurch gekennzeichnet, dass man entsprechend substituierte Ausgangsverbindungen einsetzt.

27. Verfahren zur Herstellung von pharmazeutischen Präparaten, dadurch gekennzeichnet, dass man ein in Anspruch 1 definiertes Acylderivat der Formel I oder einen leicht hydrolysierbaren Ester oder Salz einer dieser Acylderivate oder ein Hydrat einer Verbindung der Formel I bzw. eines Esters oder Salzes davon als wirksamen Bestandteil mit zur therapeutischen Verabreichung geeigneten, nicht-toxischen, inerten, an sich in solchen Präparaten üblichen festen und flüssigen Trägern und/oder Excipientien vermischt.

28. Verwendung von Endprodukten gemäss einem der Ansprüche 1-26 bei der Behandlung bzw. Prophylaxe von Krankheiten.

29. Verwendung von Endprodukten gemäss einem der Ansprüche 1-26 bei der Behandlung bzw. Prophylaxe von Infektionskrankheiten.

30. Verwendung von Endprodukten gemäss einem der Ansprüche 1-26 bei der Herstellung von Arzneimitteln für die Behandlung bzw. Prophylaxe von Infektionskrankheiten.

31. Verbindungen der allgemeinen Formel

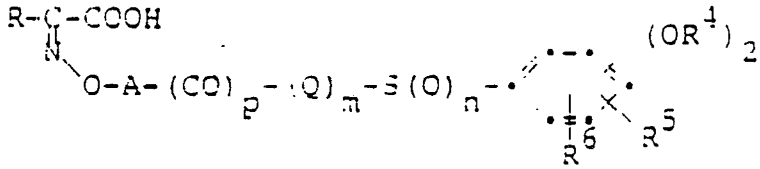

III

worin R, $R^4$, $R^5$, $R^6$, A, Q, m, n und p obige Bedeutung besitzen.

32. Verbindungen der allgemeinen Formel

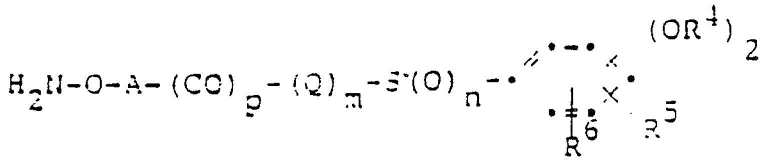

V

worin $R^4$, $R^5$, $R^6$, A, Q, m, n und p obige Bedeutung besitzen,

33. Verbindungen der allgemeinen Formel

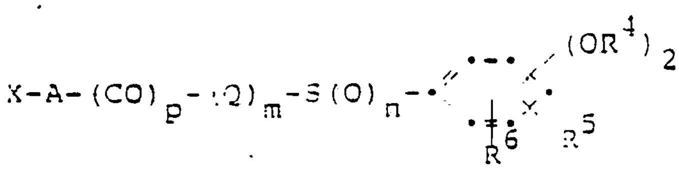

VII

worin X eine Abgangsgruppe bedeutet, und $R^4$, $R^5$, $R^6$, A, Q, m, n und p obige Bedeutung besitzen.

34. Verbindungen der allgemeinen Formel

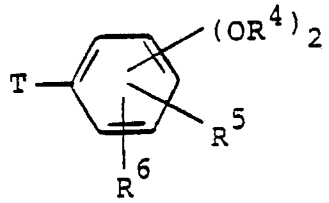

IX + XIII → XVI

I

worin $R^4$-$R^6$ obige Bedeutung besitzen, T eine der Gruppen H-Q′-S(O)$_n$-, $HO_2S$- und HS- darstellt und Q′ und n die in Anspruch 1 gegebene Bedeutung haben.

35. Verbindungen der Formel

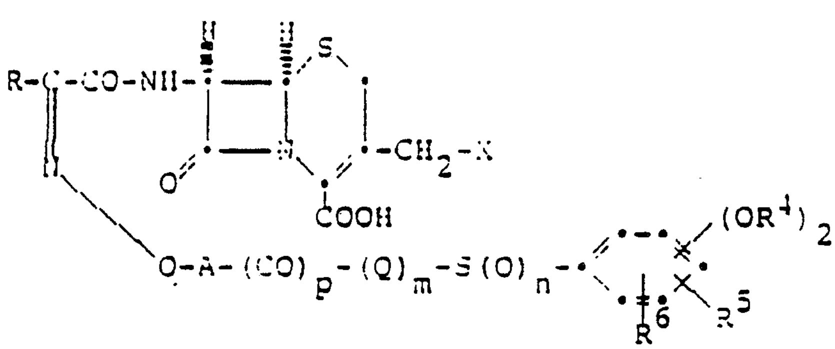

XV

worin R, $R^4$, $R^5$, $R^6$, A, Q, X, m, n und p obige Bedeutung besitzen.